# EUROPEAN PATENT SPECIFICATION

(11) **EP 3 711 743 B1**
(45) Date of publication and mention of the grant of the patent: **23.07.2025**
(21) Application number: 18878137.1
(22) Date of filing: 16.11.2018
(51) Int. Cl.: A61K 8/73, A61Q 5/12, A61Q 5/00, A61Q 5/02, A61Q 19/10, C08L 1/28, A61K 8/44, C11D 3/00, A61K 47/61, A61K 47/38, C08B 11/14, A61K 8/92, A61K 8/41, C11D 3/30, C11D 3/40

(54) **CATIONIC CELLULOSE AND COMPOSITION FOR TREATING HAIR, SKIN, OR FIBER INCLUDING SAME**
KATIONISCHE CELLULOSE UND ZUSAMMENSETZUNG ZUR BEHANDLUNG VON HAAR, HAUT ODER FASER DAMIT
CELLULOSE CATIONIQUE ET COMPOSITION POUR LE TRAITEMENT DES CHEVEUX, DE LA PEAU OU DE FIBRES, COMPRENANT CELLE-CI

(30) Priority: 16.11.2017 KR 20170153068; 30.05.2018 KR 20180061929; 30.05.2018 KR 20180062054; 08.06.2018 KR 20180065925
(43) Date of publication of application: 23.09.2020
(73) Proprietor: LG Household & Health Care Ltd., Seoul 03184 (KR)
(72) Inventor: SONG, Sang-Hun, Daejeon 34114 (KR); SON, Seong Kil, Daejeon 34114 (KR); KANG, Nae Gyu, Daejeon 34114 (KR); YOO, Dohyuk, Daejeon 34114 (KR); PARK, Hyun-Sub, Daejeon 34114 (KR); CHOI, Soo Gyu, Daejeon 34114 (KR); LEE, Jeongrae, Daejeon 34114 (KR); LEE, Sangmin, Daejeon 34114 (KR)
(74) Representative: Goddar, Heinz J.
(86) International application number: PCT/KR2018/014144
(87) International publication number: WO 2019/098774

(56) References cited:
- WO-A1-2011/131412
- WO-A1-97/35549
- WO-A2-2017/034367
- KR-A- 20170 054 062
- US-A- 5 138 043
- BARBETTA, A. ET AL.: "Porous alginate hydrogels: Synthetic methods for tailoring the porous texture", BIOMACROMOLECULES, vol. 10, no. 8, 10 July 2009 (2009-07-10), pages 2328 - 2337, XP055614222

## Description

### [Technical Field]

The present invention relates to a composition for treating hair, skin, or fiber including cationic cellulose.

### [Background Art]

Hair cosmetics generally serve the function of removing contamination from scalp and hair, and have the function of washing the hair to keep it clean and beautiful. In addition, the hair cosmetics are also used for additional purposes, such as allowing ease of combing, imparting softness, smoothness or glossiness to the hair, reducing friction on the surface of hair, preventing static shocks or protecting the hair, *etc.,* in addition to the functions above.

In order to impart a conditioning effect to hair, hair cosmetic compositions generally include a cationic polymer, a cationic surfactant, silicone or oil, *etc.* Repeated use of these chemical components may cause troubles in the hair, pores, or scalp due to cumulative adsorption, which are attributed to skin allergies and inflammation that have reacted with chemical components. In particular, when the cationic polymer among the components used is cumulatively adsorbed in an excessive amount, the hair cosmetic compositions have a disadvantage that the hair may become dry, and also, it may lead to a decrease in elasticity, a decrease in smoothness, increasing the stiffness of hair, which are the problems. In the case of a fabric softener used for the purpose of softening the fiber, a cationic polymer, a cationic surfactant, silicone or oil, *etc.* is used. As with hair cosmetic products, when the cumulative adsorption of a cationic polymer occurs in excess, the fibers become stiff, unlike the intended purposes. In particular, when the cationic polymer contained in shampoo used for a smooth conditioning effect is adsorbed on the hair having anions and thereby forms a cumulative adsorption layer, there is a shortcoming in that the sensation of a polymer is generated and the hair may become stiff. Thus, the use of cationic polymers in high content is avoided for shampoos that are intended for adsorption of hair, which is commonly anionic.

Cationic cellulose is adsorbed on the anionic hair during shampoo to impart a conditioning function, but when repeatedly used, cumulative adsorption occurs, thereby causing the problems mentioned above. In this regard, attempts have been made to improve the changes of physical state through treatments containing oils, or to develop polymers that provide a conditioning effect to hair. When cationic cellulose is used in the common sense, it is thought that the cumulative adsorption phenomenon on the hair or fibers having anions may be improved by way of reducing the nitrogen content in the cationic cellulose, which may decrease the degree of cationization. Thus, nitrogen-containing materials with a nitrogen content of 1.8% by weight or less are present and commonly used. Additionally, the production a nitrogen content of 1.8% by weight or more is difficult to achieve due to the limited production yield according to the repeated production process and the increase in the steric hindrance of cellulose caused by cationization reaction, and thus the production of cellulose having a high nitrogen content is restricted, and as a result, no related material products are available on the market.

To date, there have been no attempts to utilize cationic cellulose with an increased degree of cationization for various purposes through its molecular weight control. Under these circumstances, the present inventors have found that various characteristics are exhibited by controlling the molecular weight of the cationic cellulose, and that each can be used for specific purposes depending on these properties, thereby completing the present invention.

WO 97/35549 A1 discloses shampoo compositions for cleansing and conditioning hair comprising anionic and amphoteric surfactants and cationic cellulose. The cationic cellulose may have a molecular weight of from 400.000 to about 1.500.000.

WO 2011/131412 A1 discloses a cationic cellulose wherein at least half of the glucose units are quaternary substituted. The molecular weight of the polymer may be from 1.000 to 1.000.000 kDA.

WO 2017/034367 A2 discloses carbodiimides for skin or hair modification. Shampoo compositions are prepared comprising carbodiimide-based polymers and polyquaternium-10.

### [Disclosure]

### [Technical Problem]

An object of the present invention is to provide a composition for treating hair, skin, or fiber, including a cationic polymer.

Another object of the present invention is to provide a composition for providing the use for imparting flexibility to hair, skin, or fiber, including the cationic polymer.

Still another object of the present invention is to provide a composition for providing the use for cumulatively adsorbing active ingredients onto hair, skin, or fiber, including a substance to which the cationic polymer and the active ingredients are bound.

Still another object of the present invention is to provide a composition for providing the use for transferring active ingredients to hair, skin, or fiber, including a substance to which the cationic polymer and the active ingredients are bound.

Still another object of the present invention is to provide a composition for providing the use for preventing the loss of hair, skin, or fiber components, including the cationic polymer, a crosslinking-mediating component having a carboxyl group or an amine group, and a carbodiimide-based compound.

### [Technical Solution]

The present invention provides a composition for treating hair, skin, or fiber according to claim 1, and the use of a composition for treating hair, skin, or fiber according to claim 15. Further embodiments are disclosed in the dependent claims.

Hereinafter, the present invention will be described in more detail.

### 1. The Cationic Polymer of the Present Invention

In one aspect of the present invention to achieve the objects above, there is provided a composition for treating hair, skin, or fiber, including the polymer represented by Chemical Formula 1 below: 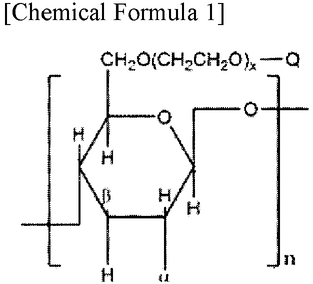
wherein n number of Q is each independently H or 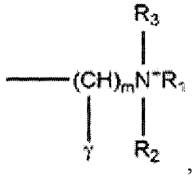 but not all H,
the R₁, R₂, and R₃ are each independently C₁₋₆ alkyl, alkenyl, or alkynyl, m is an integer from 1 to 10, γ is H or OH;
x is an integer from 1 to 100;
n is an integer from 10 to 1000; and
α and β are each independently O(CH₂CH₂O)ₛH, wherein s is an integer from 0 to 100, wherein the molecular weight of the polymer is 300,000 to 2,900,000, and
wherein the polymer represented by Chemical Formula 1 has a nitrogen content of 2.3 wt.% to 3.2 wt.%, based on the total weight of the polymer.

In the polymer, the ratio of n: the number of Q, when Q is 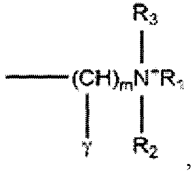 may be 1:0.3 to 1:0.7, which has a nitrogen content of 2.3% to 3.2% by weight based on the total weight of the polymer. The polymer having a nitrogen content in the above range can stably form coacervates when diluted in water together with an anionic surfactant. When the ratio of the number of Q is more than 0.7, the degree of cationization may increase, which increases viscosity, thereby decreasing the amount of polymer to be bound to form coacervates, and there is a problem that it may be difficult to make a shampoo formulation. When the ratio is less than 0.3, the degree of cationization may decrease, and thus decreasing the binding force with the anionic surfactant, which may cause a problem that the function of coacervates may be deteriorated in the washing process.

In the present invention, the polymer is also referred to as cationic cellulose or cationic cellulose polymer, *etc.*

In the present invention, the molecular weight of the polymer is 300,000 to 2,900,000. These polymers can be utilized for various purposes through a molecular weight control herein.

Specifically, when it has a molecular weight of 600,000 to 2,500,000, the cationic polymer exhibits an effect of softening the hair and making the fiber flexible as the cumulative adsorption phenomenon of hair or fiber is prevented.

Meanwhile, when it has a molecular weight of 300,000 to 400,000, the cationic polymer exhibits the property of being cumulatively adsorbed onto the hair, fiber, or skin, *etc.,* and thus exhibits a hair dye-enhancing effect by binding to an active ingredient such as a hair dye.

In addition, when it has a molecular weight of 1,300,000 to 2,900,000, the cationic polymer exhibits the property of being substituted with the cationic polymer already adsorbed on the hair, fiber, or skin and adsorbed thereto, and thus can effectively transfer a functional component for skin or a functional component for hair by binding thereto.

Meanwhile, the cationic polymer having a molecular weight of 600,000 to 2,500,000 exhibits the property of preventing the loss of amphipathic internal components or internal components having a relatively low hydrophobicity during the washing process, and thus may provide the use for preventing the loss of hair, skin, or fiber components by being included in a washing composition together with a crosslinking-mediating component having a carboxyl group or an amine group and a carbodiimide-based compound.

### 2. The Composition for Providing the Use for Imparting Flexibility to Hair, Skin, or Fiber, including the o Cationic Polymer f the Present Invention

In another aspect of the present invention to achieve the objects above, there is provided a composition for providing the use for imparting flexibility to hair, skin, or fiber, including the cationic polymer of the present invention.

The cationic polymer used herein has a molecular weight of 600,000 to 2,500,000, and exhibits the property of softening the hair and making the fibers flexible as the cumulative adsorption phenomenon of hair or fiber is prevented.

The cationic cellulose polymer of the present invention, which exhibits the property of imparting flexibility to hair, skin, or fiber, may be a cationic cellulose polymer into which a cationic quaternized ammonium, in which ethylene oxide (EO) or OH bonded to carbons 2 and 3 of the glucose monomer in the cellulose skeleton is capable of hydrogen bonding, and the OH site bonded to carbon 6 is not capable of hydrogen bonding through a linker, is introduced. The cationic polymer is a cationic cellulose polymer having a nitrogen content of 2.3% to 3.2% by weight and having a molecular weight of 300,000 to 2,900,000, based on the total weight of the polymer.

The cationic cellulose polymer may be a quaternized ammonium salt including hydroxyethyl cellulose and trimethylamine.

The preparation reaction of the cationic cellulose polymer (this preparation method is represented by Reaction 1) may prepare a quaternized ammonium salt obtained by quaternization reaction of hydroxyethyl cellulose with chlorohydroxypropyl trimethylamine.

Specifically, it may be prepared by adding sodium hydroxide and urea in a weight ratio of 7 to 9: 10 to 12: 75 to 85 (sodium hydroxide: urea: water) relative to water as a solvent, and then dissolving hydroxyethyl cellulose in a concentration of 1% to 3% by weight and further adding a quaternized ammonium-based compound. More specifically, it may be prepared by adding sodium hydroxide and urea in a weight ratio of 7.5: 11: 81.5 (sodium hydroxide: urea: water) relative to water as a solvent and then dissolving hydroxyethyl cellulose in a concentration of 2% by weight. The solvent of the above composition helps to improve the solubility and stability of hydroxyethyl cellulose, thereby helping to improve the uniformity of modification in the reaction process [Bi Xiong, Dissolution of cellulose in aqueous NaOH/urea solution: role of urea, Cellulose (2014) 21: 1183-1192]. Subsequently, the quaternized ammonium-based compound may be further added at an appropriate concentration (1 mole or higher moles compared to the glucose monomer of cellulose), and the reaction may be proceeded according to the room temperature or heated temperature conditions to carry out modification.

There are three positions in the glucose ring, *i.e.,* C2, C3, and C6, that have hydroxyl groups which can be modified. Among these, the primary alcohol at C6 has minimum steric hindrance, and thus generally has excellent positional selectivity for modification reactions. In contrast, the difference in the preference for the modification reaction between C2 and C3 is argued differently depending on the conditions such as a solvent system, *etc.* [Mitsuru Abe, Regioselectivity in Acetylation of Cellulose in Ionic Liquids, Materials Science Inc. Nanomaterials & Polymers (2016) 1: 2474-2478].

The cationic cellulose polymer may be a polymer in which ethylene oxide (EO) is substituted at α and β positions in the glucose ring. In addition, as a Comparative Example, a polymer in which an alkyl group is substituted at α and β positions in the glucose ring was used as a cellulose polymer, and this was used to evaluate for the height of the film according to repeated adsorption. A structure including an alkyl group can be prepared by the addition of ethylene oxide (EO) or by the alkylation with reference to the Reference Document "cation-modified cellulose-based fabric and a method for manufacturing same (WO 2016085099 A1)".

In the case of adding EO to the α and β positions in the glucose ring, the addition of EO may be carried out by proceeding gas-phase polymerization using epoxide, spraying the reactants in the chamber thereto and then proceeding a heated and pressurized reaction. In the case of alkylation, a reaction may be performed so that a desired alkyl group can be added using a reactant having a halohydrin, an aldehyde, or an epoxide functional group at the end.

In one embodiment, EO was added to the α or β position in the glucose of the cationic cellulose polymer substituted with trimethyl amine, and as a Comparative Example, a functional group was added by repeated alkylation at the α position in the glucose. As a result, when EO was added at the α or β position in the glucose, the adsorption force was deteriorated due to the cationic repulsive force with the polymer already adsorbed onto the hair or the fiber surface when the polymer was repeatedly used, and at the same time, as the polymer adsorbed onto the hair could form hydrogen bonds at the α and β positions, it was washed away due to hydrophilicity with water, ultimately exhibiting the effect of preventing cumulative adsorption of the cationic polymer. In contrast, in the case of the alkylated cationic cellulose polymer, it was confirmed that the affinity for water molecules was reduced by an increase in hydrophobicity due to the alkyl group, thereby reducing the phenomenon of rinsing the polymer with water.

Therefore, the cationic cellulose polymer may specifically be a polymer in which EO is added to the α or β position in the glucose ring.

In addition, in order to increase the nitrogen content of the quaternized ammonium salt in the cationic polymer, the content of the degree of cationization can be controlled by repeatedly controlling the addition reaction process of the quaternized ammonium compound of Reaction 1 described above, ultimately controlling the nitrogen content, while changing the amount of trimethylamine used and controlling the temperature conditions, and this was confirmed through an elemental content analysis.

The cationic cellulose polymer, which exhibits the property of imparting flexibility to hair, skin, or fiber, has a molecular weight of 300,000 to 2,900,000, and a nitrogen content of 2.3% to 3.2% by weight relative to the total weight of the polymer. The cationic cellulose polymer may have a molecular weight of preferably 600,000 to 2,500,000, more preferably 700,000 to 1,500,000. The cationic cellulose polymer may have a nitrogen content of preferably 2.5% to 3.0% by weight, more preferably 2.7% to 2.8% by weight relative to the total weight of the polymer.

When the molecular weight and nitrogen content of the cationic cellulose polymer are within the range above, the cumulative adsorption phenomenon of hair may be prevented and a conditioning effect may be exhibited on the hair. In one embodiment (Examples 2, and 4 to 5), when the molecular weight of cellulose was 300,000 to 2,900,000, and the nitrogen content was 2.3% to 3.2% by weight, the thickness of the cumulative adsorption layer was reduced and became constant, even when the number of adsorptions of the cationic polymer on the surface of the hair was increased. This is because the adsorption is prevented by the mutual electric repulsive force between the cationic polymer layer adsorbed onto the hair and the cationic polymer to be adsorbed, while the portion, in which a fixed amount of the polymer is adsorbed by molecular weight, is easily dissolved in water and washed down as the polymer adsorption layer having a high degree of cationization shows hydrophilicity in the rinsing process. In addition, it was confirmed that the adsorption thickness was reduced and fluctuated as the rinsing power was increased when the polymer has a degree of cationization and molecular weight within the range above. In contrast, when the molecular weight and the nitrogen content of the cationic cellulose polymer were out of the range (Comparative Examples 8 to 14), it was observed that the adsorption thickness increased as the number of polymer adsorption was increased, that is, cumulative adsorption was observed, and it was confirmed that even when the rinsing power was increased, the cumulative adsorption occurred continually without changing the adsorption thickness.

In the present invention, there is provided a composition for preventing cumulative adsorption, including the cationic cellulose polymer as an active ingredient.

The composition may be treated to an anionic object, and the anionic object may be hair, skin, or fiber.

When the composition is treated to hair, skin, or fiber, an anionic object, the phenomenon in which the cationic cellulose polymer is accumulated and adsorbed onto the anionic object may be prevented even when repeatedly used, and accordingly, the anionic object may become soft and smooth.

In addition, in the present invention, there is provided a composition for treating hair or treating fiber, including the cationic cellulose polymer.

The cationic cellulose polymer is adsorbed onto the hair surface, thereby increasing the smoothness of the hair after rinsing and drying. Through this, when the composition including the cationic cellulose polymer is used for hair treatment, a remarkable hair conditioning effect can be exhibited, thereby providing high satisfaction to the user, in addition to the washing or styling function, which is an original function of the hair cosmetic. Further, the composition can improve the physical state of hair by supplying smoothness of the hair even after repeated use, and thus can maintain the feeling of use.

When the composition is used for fiber treatment, the skin irritation caused by a conventional cationic surfactant used for imparting a fiber softening effect can be avoided, and the feeling of stiffness of fibers resulting from the cumulative adsorption phenomenon caused by the reuse of the polymer used for fiber treatment can be eliminated, thereby providing high satisfaction to the user.

Here, the "prevention of accumulated adsorption" means that a phenomenon in which a polymer is accumulated on a surface of an object to be used when the polymer is repeatedly used or a phenomenon of polymer lamination can be prevented. When the cumulative adsorption of the polymer is prevented, the degree of elasticity and smoothness of the object to be used, which can occur during the cumulative adsorption of the polymer, is reduced, and the problem of stiffness can be solved, thereby allowing the hair or fiber to become soft and flexible.

Here, the composition may contain the cationic polymer in an amount of 0.01% to 10% by weight, preferably 0.05% to 5% by weight, and more preferably 0.1% to 3% by weight relative to the total weight of the composition. When the content of the polymer is less than 0.01% by weight, the hair conditioning effect or the fiber softening effect may be insignificant, and when the content is more than 10% by weight, there is a risk that the formulation stability of the composition may be impaired. The same can be applied to cosmetic compositions for strengthening hair, reinforcing elasticity, or providing protection.

The composition may further include a cationic polymer, a surfactant, or both, which can be conventionally used for hair treatment or fiber treatment compositions, in addition to the cationic cellulose polymer.

The cationic polymer may be any one or more selected from the group consisting of a cellulose-based polymer, a guar-based polymer, and a synthetic material-based polymer. The cellulose-based polymer may be JR125, JR400, JR30M, POLYQUAT-3000KC, LR-400R-LO, LR30M, Ucare LK, Catinal HC100, Catinal HC200, *etc.,* and the guar-based polymer may be guarhydroxypropyltrimonium chloride or hydroxypropyl guarhydroxypropyltrimonium chloride, *etc.* The synthetic material-based polymer may be one or more selected from the group consisting of polyquaternium-22, polyquaternium-47, polyquaternium-53, a dimethyldiallylammonium chloride polymer, an acrylamide-dimethyldiallylammonium chloride copolymer, a polyvinylpyrrolidone (PVP)-dimethylaminoethylmethacrylate copolymer, an acrylic acid-dimethyldiallylammonium chloride copolymer, an acrylamide-dimethylamino ethylmethacrylate methyl chloride copolymer, a trimethylaminoethylmethacrylate polymer, *etc.*

For the purpose of increasing the washing power, the composition may include a surfactant within a range that does not impair the object of the present invention.

As the surfactant, any one or more selected from the group consisting of an anionic surfactant, an amphoteric surfactant, and a nonionic surfactant may be used.

The anionic surfactant may be used without limitation as long as it is a component commonly used in hair compositions, and may include sodium lauryl sulfate, sodium laureth sulfate, ammonium lauryl sulfosuccinate, ammonium myreth sulfate, disodium laureth sulfosuccinate, disodium C₁₂-C₁₄ pareth-2 sulfosuccinate, or mixtures thereof, *etc.*

In addition, the amphoteric surfactant may be any one or more selected from the group consisting of betaine, cocamidopropyl betaine, amido propyl betaine, sulfobetaine, and coco amphocarboxy glycinate.

Additionally, the nonionic surfactant may be any one or more selected from the group consisting of caprylyl/capryl glucoside, coco glucoside, lauramide DEA, cocamide DEA, cocamide methyl MEA, and glyceryl monostearate.

The cationic polymer included in the composition may be contained in an amount of 0.01% to 3% by weight, preferably 0.05% to 1% by weight relative to the total weight of the composition. The anionic surfactant may be included in an amount of 1% to 30% by weight, preferably 3% to 20% by weight relative to the total weight of the composition. The amphoteric surfactant may be included in an amount of 0.1% to 20% by weight, preferably 2% to 15% by weight relative to the total weight of the composition. When each of the components is included in the composition within the range above, the hair conditioning effect of the composition, *etc.,* can be most excellently exhibited for the purpose of the present invention.

Additionally, the composition may further include additional adjuvants used in the art within a range that does not impair the object of the present invention so as to further enhance the conditioning effect. For example, components commonly referred to as silicones such as water-insoluble non-volatile dimethicone, cyclomethicone, aminated silicone, trimethylsilyl amodimethicone, or vinyl silicone, and derivatives thereof, vegetable oils, vegetable fats and oils, animal oils, animal fats and oils, hydrocarbon oils, or synthetic ester oils, *etc.* may be used. The hydrocarbon oil may include liquid paraffin, isoparaffin, or hydrogenated polydecene, *etc.,* and the ester oil may include isopropyl myristate, isopropyl palmitate, isostearyl isostearate, or C₁₂₋₁₅ alkyl benzoate, *etc.,* but these are not limited thereto.

Further, the composition may include any one or more selected from the group consisting of fatty substances, organic solvents, solubilizing agents, thickening agents, gelling agents, softening agents, antioxidants, suspending agents, stabilizing agents, foaming agents, flavoring agents, surfactants, water, ionic or non-ionic emulsifiers, fillers, sequestering agents, preservatives, vitamins, blockers, wetting agents, essential oils, dyes, pigments, hydrophilic or lipophilic active agents, *etc.* commonly used in the compositions for softening hair and fiber.

Additionally, the composition may further include additives that provide beneficial properties to the human body. For example, it may further include additives for imparting beneficial properties to the human body *i.e.,* the properties such as washing, volumizing, trimming, protection, blocking, moisturizing, dyeing, coloring, discoloration, restriction, deodorization, antiseptic, cooling, hair removal, hair growth, anti-dandruff, hair loss prevention, hair tonic, anti-inflammatory, fragrance, aroma, whitening, anti-aging, wrinkle improvement, astringency, relaxation, shrinkage, sebum control, keratin exfoliation, sterilization, antiphlogistic, antipruritic, deodorization, antihistamine, anti-seborrhea, blood circulation promotion, UV protection, or skin metabolism promotion, *etc.*

In addition, the composition may further contain preservatives, thickeners, viscosity modifiers, pH modifiers, flavoring agents, dyes, or conditioning agents, *etc.,* which can be commonly used as components of hair and fiber compositions, and these can be commercially and easily purchased and used. Examples of preservatives include benzoic acid and salts, methyl paraoxybenzoate, a mixture of methylchloroisothiazolinone or methylisothiazolinone (trade name: Kathon CG, manufacturer: The Dow Chemical Company), *etc.* As a thickener and viscosity modifier, hydroxypropylmethylcellulose, hydroxymethylcellulose, sodium chloride, ammonium chloride, propylene glycol, hexylene glycol, sodium xylene sulfonate, or ammonium xylene sulfonate, *etc.* may be used. As a pH modifier, citric acid, sodium hydroxide, or triethanolamine, *etc.* may be used. As a dye, water-soluble tar color, *etc.* may be used. Further, as a conditioning agent, animal and vegetable extracts, proteins and protein derivatives, higher fatty acids, *etc.* may be used.

The composition may be prepared in the form of a general emulsifying formulation or solubilizing formulation.

In addition, the composition may be prepared in any formulation that can be applied to the scalp and fibers, such as liquid, cream, paste, or solid.

Further, the composition may be formulated into any one selected from the group including shampoo, hair conditioner, hair lotion, hair essence, hair gel, hair pack, patch and spray, laundry detergent, fabric softener, *etc.*

### 3. Composition for Providing the Use for Cumulatively Adsorbing Active Ingredients onto Hair, Skin, or Fiber including Substance to which Cationic Polymer of the Present Invention and Active Ingredients are Bound

In still another aspect of the present invention to achieve the objects above, there is provided a composition for providing the use for cumulatively adsorbing active ingredients onto hair, skin, or fiber, including a substance to which the cationic polymer and active ingredients are bound.

The cationic polymer used herein has a molecular weight of 300,000 to 400,000, and exhibits the property of being cumulatively adsorbed onto hair, fiber, or skin.

The polymer of the present invention, which exhibits the property of being cumulatively adsorbed onto hair, fiber, or skin, *etc.* has a function of efficiently transferring active ingredients to OH attached to carbon 2 and/or 3 of the cationic cellulose, or to an anionic substrate attached to a polyethylene glycol linker terminal bound thereto. For example, when a reactive dye is used as an active ingredient, the reactive dye can be transferred to hair, and thus can be used for dyes or strengthening dyes.

In addition, since coacervates are formed when the polymer is diluted in water along with an anionic surfactant, due to the quaternized ammonium bonded to the carbon 6 of the cationic cellulose, it can be used as a washing agent having a dye-strengthening function at the same time without inhibiting the hair dyeing effect by the anionic surfactant.

When the composition containing 1% to 3% by weight of the polymer has a viscosity of 20 cps to 500 cps, specifically 50 cps to 500 cps, it has a property of enhancing hair color when repeatedly used. When it has a viscosity higher than 500 cps, the adsorption layer may not increase by repeated use, and when it has a viscosity lower than 20 cps, it may be difficult to exhibit adsorption.

The viscosity of the composition is referred to as a value that increases as the molecular weight of the polymer increases, and the composition may exhibit excellent adsorption and desorption capacities when the molecular weight of the polymer is 300,000 to 400,000. Specifically, when the molecular weight of the cationic cellulose polymer exceeds 400,000 and when additional adsorption occurs, additional adsorption is prevented by mutual electric repulsive force between the cationic polymer layer, which is adsorbed on the substrate, and the cationic polymer to be adsorbed. Further, the polymer adsorption layer already having a high molecular weight in the rinsing process has a hydrophilic property for a large area, and thereby shows a property of being easily dissolved in water and rather be desorbed. In particular, when the molecular weight of the polymer is high, adsorption trains (adsorption region) and loops (non-adsorption region) increase in the state in which the polymer is adsorbed onto the anionic substrate, thereby increasing entropy, so that the polymer exhibits a property of being easily desorbed in contact with water molecules. When the molecular weight is less than 10,000, the adsorbed area decreases, so that it may be difficult to detect color change and the smooth conditioning effect.

As used herein, the term "anionic substrate" refers to a substrate that exhibits anionic properties of hair, skin, and fibers, *etc.* The anionic substrate can be adsorbed by the electrostatic attraction with the polymer layer exhibiting cationic property, so that the active ingredient bound to the polymer can be easily transferred.

The active ingredient may be a reactive dye.

As used herein, the term "reactive dye" refers to a dye that chemically reacts with a functional group of hair, skin, or fiber, and is dyed by a covalent bond or ionic bond. The reactive dyes are known to have a property of excellent fastness because the functional group of the reactive dye forms covalent bonds with hair, skin, or fibers.

The reactive dye as described above is not limited to its type as long as it is known in the art. The detailed description thereof is disclosed in Industrial Dyes (K.Hunger edition, Wiley VCH 2003) *etc.,* and the commonly used reactive dyes are listed in color indexes [Society of Dyers and Colorists and American Association of Textile Chemists and Colorists].

Specifically, the covalent reactive dye includes reactive groups selected from dichlorotriazinyl, difluorochloro pyrimidine, monofluorotriazinyl, dichloroquinoxaline, vinyl sulfone, difluorotriazine, monochlorotriazinyl, bromoacrylamide, and trichloropyrimidine. More specifically, the reactive dye includes reactive groups selected from monochlorotriazinyl, dichlorotriazinyl and vinylsulfonyl.

In addition, the reactive dye preferably includes a chromophore selected from azo, anthraquinone, phthalocyanine, formazan, and tripendioxazine.

The reactive dye may contain one to four SO₃Na groups, thereby generating negative charges at an effective level.

Examples of the reactive dye include reactive blue No. 4, reactive black No. 5, reactive blue No. 19, reactive red No. 2, reactive blue No. 59, reactive blue No. 269, reactive blue No. 11, reactive yellow No. 17, reactive orange No. 4, reactive orange No. 16, reactive green No. 19, reactive brown No. 2, reactive brown No. 50, *etc.,* but is not limited thereto.

As the ionic reactive dye, basic raw materials notified according to the Ministry of Food and Drug Safety Notification No. 2016-49 can be selected. The cation or cation portion of zwitterion in the basic raw materials can be adsorbed to anionic hair via an ionic bond, and the basic material may include basic brown No. 16, basic blue No. 99, basic red No. 76, basic brown No. 17, basic brown No. 87, basic brown No. 57, basic red No. 51, basic orange No. 31, *etc.,* but is not limited thereto. In addition, there are acidic raw materials, which have an anionic charge, but can be adsorbed by being in a part of the hydrophobic portion of the hair and can be attached to the cationic adsorption polymer adsorbed onto the hair, and the acidic raw materials may include acidic red No. 52, acidic red 92, *etc.,* but are not limited thereto.

In the present embodiment, in order to determine the position of the polymer adsorption, a reactive blue No. 4 dye and 5-([4,6-dichlorotriazin-2-yl] amino) fluorescein, which are used as phosphors and can simultaneously utilize the dyeing function, were used as a reactive dye, but these are not limited to the reactive dyes used as a phosphor.

The polymer may be mixed with an anionic surfactant and used as a washing composition.

Specifically, the washing composition may be treated on an anionic site such as hair or skin. The washing composition may be applied to fibers such as hair to exhibit a dyeing function or to enhance the dyed color.

Specifically, when the washing composition is treated on undyed hair, it may be dyed. In addition, it may be possible to enhance the color already dyed by washing the hair with the washing composition on the hair dyed by another dyeing method. In this case, a washing composition including a polymer, to which a dye exhibiting the same or similar color is bound, may be used.

As the anionic surfactant, sodium lauryl sulfate, sodium laureth sulfate, polyoxyethylene sodium lauryl sulfate, ammonium lauryl sulfosuccinate, ammonium myreth sulfate, disodium laureth sulfosuccinate, disodium C₁₂-C₁₄ pareth-2 sulfosuccinate, or mixtures thereof, *etc.* can be used. The anionic surfactant may be included in an amount of 1% to 30% by weight, specifically 3% to 20% by weight relative to the total weight of the composition. The amphoteric surfactant may be included in an amount of 0.1% to 20% by weight, specifically 2% to 15% by weight relative to the total weight of the composition.

The washing composition may further include a nonionic surfactant and/or an amphoteric surfactant. Examples of the nonionic surfactant include lauric acid diethanolamide, palm oil fatty acid diethanolamide, and palm oil fatty acid monoethanolamide, and examples of the amphoteric surfactant include betaines, cocamidopropyl betaine, alkyl glycinate, and alkyl aminopropionate, but these are not limited thereto. The nonionic surfactant and amphoteric surfactant may be included in an amount of 0% to 15% by weight, specifically 1% to 15% by weight, and more specifically 3% to 10% by weight relative to the total weight of the composition.

The washing composition may include oil used for providing glossiness to the hair or moisturizing the hair. Any oil that can change the moisture content in the hair by adsorbing and penetrating into the hair can be used without limitation.

Non-limiting examples of the oil may include components commonly referred to as silicones such as water-insoluble non-volatile dimethicone, cyclomethicone, aminated silicone, trimethylsilyl amodimethicone, or vinyl silicone, *etc.,* and derivatives thereof, vegetable oils such as *Simmondsia Chinensis* seed oil, vegetable fats and oils, animal oils, animal oils and fats, hydrocarbon oils, or synthetic ester oils, *etc.* The hydrocarbon oil may include liquid paraffin, isoparaffin, or hydrogenated polydecene, *etc.,* and the ester oil may include isopropyl myristate, isopropyl palmitate, isostearyl isostearate, C₁₂₋₁₅ alkyl benzoate, triethylhexanoin, squalane, palm oil, *Olea europaea* oil, PPG-3 caprylyl ether, capric/caprylic triglyceride, isostearyl isostearate, *Cocos nucifera,* polyglyceryl-6 octacaprylate, hydrogenated polydecene, *Simmondsia Chinensis* seed oil, DI-C₁₂₋₁₃ alkyl malate, *etc.,* but is not limited thereto. In one embodiment of the present invention, *Simmondsia Chinensis* seed oil was used as the oil.

The oil may be included in an amount of 0.05% to 4% by weight, specifically 0.1% to 2% by weight.

The washing composition may be used as a shampoo. In this case, the composition may further include additional adjuvants used in the art within a range that does not impair the object of the present invention so as to further enhance the conditioning effect. For example, it may further include any one or more selected from the group consisting of fatty substances, organic solvents, solubilizing agents, thickening agents, gelling agents, softening agents, antioxidants, suspending agents, stabilizing agents, foaming agents, flavoring agents, surfactants, water, ionic or non-ionic emulsifiers, fillers, sequestering agents, preservatives, vitamins, blockers, wetting agents, essential oils, dyes, pigments, hydrophilic or lipophilic active agents, *etc.* commonly used in hair compositions.

Additionally, the composition may further include additives that provide beneficial properties to the human body. For example, it may further include additives for imparting beneficial properties to the human body such as washing, volumizing, trimming, protection, blocking, moisturizing, dyeing, coloring, discoloration, restriction, deodorization, antiseptic, cooling, hair removal, hair growth, anti-dandruff, hair loss prevention, hair tonic, anti-inflammatory, fragrance, aroma, whitening, anti-aging, wrinkle improvement, astringency, relaxation, shrinkage, sebum control, keratin exfoliation, sterilization, antiphlogistic, antipruritic, deodorization, antihistamine, anti-seborrhea, blood circulation promotion, UV protection, or skin metabolism promotion, *etc.*

In addition, the composition may further contain preservatives, thickeners, viscosity modifiers, pH modifiers, flavoring agents, dyes, or conditioning agents, *etc.,* which can be commonly used as components of hair compositions, and these can be commercially and easily purchased and used. Examples of preservatives include benzoic acid and salts, methyl paraoxybenzoate, a mixture of methylchloroisothiazolinone or methylisothiazolinone (trade name: Kathon CG, manufacturer: The Dow Chemical Company), *etc.* As a thickener and viscosity modifier, hydroxypropylmethylcellulose, hydroxymethylcellulose, sodium chloride, ammonium chloride, propylene glycol, hexylene glycol, or sodium xylene sulfonate, ammonium xylene sulfonate, *etc.* may be used. As a pH modifier, citric acid, sodium hydroxide, or triethanolamine, *etc.* may be used. As a dye, water-soluble tar color, *etc.* may be used. In addition, as a conditioning agent, animal and vegetable extracts, proteins and protein variants, higher fatty acids, *etc.* may be used.

The composition can be prepared in the form of a general emulsifying or solubilizing formulation.

In addition, the composition can be prepared in any formulation that can be applied to the scalp, such as liquid, cream, paste, or solid.

In addition, the composition may be formulated into any one selected from the group including shampoo, hair conditioner, hair lotion, hair essence, hair gel, hair pack, patch, and spray, *etc.*

It may be utilized as a method for dyeing hair, including the step of washing the hair using the composition.

Specifically, the hair may be repeatedly washed 1 to 10 times using the washing composition. Through repeated washing, the degree of dyeing of the hair may be enhanced.

Methods of dyeing hair include dyeing undyed hair and enhancing color on already dyed hair.

### 4. Composition for Providing the Use for Transferring Active Ingredients to Hair, Skin, or Fiber, including Substance to which the Cationic Polymer of the Present Invention and Active Ingredients are Bound

In still another aspect of the present invention to achieve the objects, there is provided a composition for transferring active ingredients to hair, skin, or fiber, including a substance to which the cationic polymer and active ingredients are bound.

The cationic polymer used herein has a molecular weight of 1,300,000 to2,900,000, and the cationic polymer exhibits the property of being substituted with a cationic polymer already adsorbed on hair, fiber, or skin and adsorbed.

The cationic polymer may be present by being bonded with an appropriate anion via an ionic bond, and herein, the appropriate anion group may be a halogen group, for example, Cl⁻, Br⁻, *etc.*

The cationic cellulose polymer is used for the purpose of transferring active ingredients.

As used herein, the expression "for transferring active ingredients", "transfer of active ingredients", *etc.* means that when the cellulose polymer is adsorbed on a substrate, the active ingredient bound to the cellulose polymer via an ion or covalent bond is dissociated or it shows the efficacy of the active ingredients in the substrate while being bound to the polymer. The substrate is preferably a substrate capable of forming an electrostatic attraction with the cellulose polymer, and more specifically, an anionic substrate may be preferred.

In one embodiment, the cellulose polymer, to which the hair dye is bonded via a covalent bond, is intended to transfer the hair dye to the substrate, and the cellulose polymer can be applied to the hair to exhibit a dyeing effect resulting from the hair dye on the hair.

In another embodiment, the cellulose polymer, to which a moisturizing oil for hair is bonded via a covalent bond, is intended to transfer the oil for hair to the substrate, and the cellulose polymer can be applied to the hair to exhibit a continuous moisturizing effect on the hair.

The inventors of the present invention have found that by binding of an active ingredient to a cellulose polymer modified by a cation through an ion or covalent bond, it is possible to improve the efficiency and sustainability of transferring the active ingredient to an anionic substrate such as skin or hair.

The cationic cellulose polymer modified with a cation by the quaternized amine group may be formed by covalently binding a quaternized amine group to a linker (L) at the carbon 6 in the glucose monomer in the cellulose monomer. Here, (CH₂CH₂O)x may be used as the linker, wherein x is an integer from 1 to 10. In addition, the introduction of the linker at the carbon 6 can be performed through methods known in the art.

In one embodiment, the cellulose polymer for transferring active ingredients may be a polymer represented by Chemical Formula 2 below.

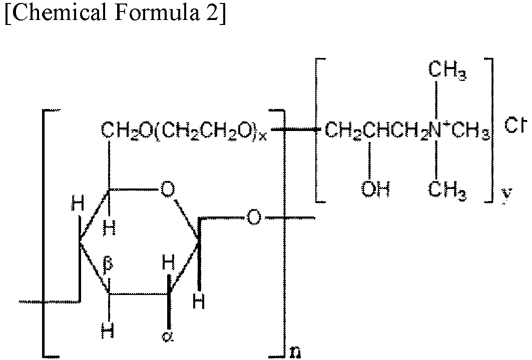

In Chemical Formula 2 above, x is an integer from 1 to 10; when n is 1, y is 0.3 to 0.7; and α and β are each independently -OH, -CH₂OH, or -CH₂CH₂OH.

The active ingredient, which is bound to the cellulose polymer for transferring the active ingredient and transferred to the substrate, may preferably exhibit a functional component for skin or a functional component for hair. The functional component for skin may be a sun screening agent, a dye, a wrinkle-improving agent, an elasticity-reinforcing agent, a whitening agent, an agent for preventing or removing dead skin cells, or a wrinkle-masking agent, *etc.* More specifically, the functional component for skin may be carbonate selected from the group consisting of ammonium carbonate, potassium carbonate, calcium carbonate, sodium carbonate, sodium bicarbonate, potassium bicarbonate, potassium sesquicarbonate, calcium sesquicarbonate, and sodium sesquicarbonate; mineral wax selected from the group consisting of ceresin wax, paraffin wax, vaseline wax, petroleum wax, ozokerite, montan wax, and microcrystalline wax; animal wax selected from the group consisting of beeswax and lanolin; vegetable wax selected from the group consisting of candelilla, ouricurry, carnauba wax, Japan wax, cocoa butter, cork fiber, and sugarcane wax; hydrogenated oil which is solid at 25°C.; fatty esters and glycerides; synthetic wax selected from the group consisting of polyethylene wax and wax obtained by the Fischer-Tropsch synthesis; and silicone wax, natural or synthetic triglycerides, ester oils, and hydrocarbon oils.

The functional component for hair may be preferably a hair-strengthening agent, a dye, a conditioning agent, a coating agent, a lipid component, or an internal crosslinking agent, and more specifically a lipid component selected from the group consisting of an amphoteric polymer such as an amino acid polymer, such as polylysine, a polyamine polymer, a polycarboxylic acid polymer, a methacryloyl ethyl betaine/methacrylate copolymer, or an octylacrylamide/acrylate/butylaminoethyl methacrylate copolymer; a nonionic polymer such as polyvinylpyrrolidone, a PVP/vinyl acetate (VA) copolymer, a PVP/dimethylaminoethyl methacrylate copolymer, or polyurethane; and an anionic polymer such as an acrylate/methacrylate copolymer or a VA/crotonate/vinyl neodecanoate copolymer, natural or synthetic triglycerides, ester oils and hydrocarbon oils, but is not limited thereto.

In one embodiment, a cellulose polymer in which reactive blue No. 4 dye, as an active ingredient, was a covalently bonded at the α position was prepared, thereby confirming the fluctuation of the adsorption layer formed on the substrate. In addition, it was confirmed that the active ingredient can be continuously transferred to the substrate.

In one embodiment, a cellulose polymer in which 5-([4,6-dichlorotriazin-2-yl] amino) fluorescein, as an active ingredient, formed a covalent bond with the -OH group at the α position was prepared, thereby confirming that the active ingredient was continuously transferred to the substrate.

In one embodiment, a cellulose polymer in which an oil composed of an alkyl group having 15 to 20 carbon atoms, as an active ingredient, is covalently bonded to the α position was prepared, thereby confirming that the moisturizing effect can be continuously exhibited by the active ingredient in the hair in which the hair composition including the polymer is used.

In one aspect, the cationic polymer may have a molecular weight of 1,300,000 to 2,900,000. The cellulose polymer may form an adsorption layer having a thickness of certain range on the substrate. Additionally, when the cellulose polymer within the molecular weight range above is used, it may exhibit an excellent effect of continuously transferring the active ingredient through the fluctuation of the adsorption layer formed on the substrate. When the molecular weight is less than 1,300,000, the height of the adsorption layer may be constant or the fluctuation may be insignificant, and accordingly, there may be a problem that efficiency and sustainability of transferring effective ingredients may be deteriorated. In addition, when the molecular weight exceeds 4,000,000, the spreadability of the polymer may be poor, so that it may be difficult to achieve homogenous adsorption of the polymer onto the substrate, and the high cationic polymer having a high molecular weight with a large surface area may cause a mutual repulsive force, which weakens the adsorption force, thereby raising a problem that the adsorption layer is easily destroyed upon contact with water including rinsing.

The present inventors have found that the thickness of the adsorption layer formed by adsorbing the polymer used for the compositions for skin or hair onto the substrate changes according to the electrostatic properties and molecular weight of the polymer. More specifically, they have found that an adsorption layer formed by the cationic polymer adsorbed onto an anionic substrate such as skin or hair can be formed to have a thickness in a certain range depending on a specific molecular weight range of the cationic polymer. In particular, the cellulose polymer for transferring effective ingredients having a molecular weight of 1,300,000 to 2,900,000 according to the present invention may form an adsorption layer having a thickens in a fixed range when the viscosity of the 2% aqueous solution is 8,000 cps to 100,000 cps when measured using a viscometer (Brookfield, US) with a spindle condition of 50 rpm at 25°C. The inventors of the present invention have confirmed that the adsorption layer formed after binding dye, as an active ingredient, to the cellulose polymer can continuously transfer the active ingredients to the substrate by being adsorbed onto the substrate, through the adsorption layer fluctuation phenomenon, rather than being fixed.

As used herein, the expression "adsorption layer fluctuation" was used to indicate that the molecular position in the adsorption layer formed by adsorbing the polymer onto the substrate is not fixed, but there is a shift in the molecular position in the adsorption layer. The adsorption layer fluctuation is related to the molecular weight, viscosity, degree of cationization, and degree of crosslinking of the cellulose polymer. More specifically, the cationic cellulose polymer forms an adsorption layer on the anionic substrate such as skin, hair, scalp, *etc.* by electrostatic action. When the cationic cellulose polymer is adsorbed onto a substrate, and additional adsorption is induced by further using a cationic cellulose polymer in the state in which an adsorption layer is already formed, the polymer may exhibit a property of suppressing or preventing the additional adsorption between the already formed adsorption layer and the newly applied cationic cellulose polymer by the electrostatic repulsion. Meanwhile, when the cationic polymer is adsorbed onto the substrate and forms an adsorption layer, an adsorption train portion (adsorbed portion) and a loop portion (non-adsorbed portion) are present in the formed adsorption layer. As the molecular weight of the cationic polymer increases, the viscosity increases, and the homogeneity of adsorption decreases. That is, the adsorption train portion and the loop portion are increased, which leads to high entropy in the adsorption layer. Therefore, the cationic polymer having a molecular weight in a certain range or more exhibits a property of being easily desorbed by contact with water molecules. Due to such overall action, the cationic cellulose polymer having a molecular weight in a certain range shows the property of desorbing the adsorption layer already formed on the anionic substrate, and adsorbing the newly applied adsorption layer onto the anionic substrate, thereby leading to the adsorption layer fluctuation between the conventionally formed adsorption layer and the newly formed adsorption layer. Herein, the effect of continuously transferring the active ingredient to the substrate can be exhibited through the adsorption layer fluctuation of the cationic cellulose polymer.

In the present invention, there may be provided a cellulose polymer for transferring the active ingredients; and a composition for skin or hair, which contains an active ingredient which is bound to at least one of the -OH group present at the α or β position of the cellulose polymer by an ion or a covalent bond. The composition may exhibit an effect of continuously transferring the active ingredient with excellent efficiency, including a cellulose polymer to which the active ingredient is bound.

In the composition, the content of the cellulose polymer for transferring active ingredients is not limited, but may be 0.01% to 10% by weight, preferably 0.1% to 5% by weight relative to the total weight of the composition. When the polymer is included within the content above, it is possible to provide a composition having an excellent molecular weight and phase stability.

Here, the composition for skin may be formulated into foundation, solution, ointment for external use, cream, foam, nutrient cosmetic water, softening cosmetic water, pack, makeup base, primer, essence, sunscreen cream, sun oil, sunscreen stick, suspension, emulsion, paste, gel, lotion, oil, powder, powder foundation, emulsion foundation, wax foundation, patch, or spray, but is not limited thereto. For reference, the powder formulation can continuously transfer the active ingredient using sweat or moisture that comes into contact with the skin.

Here, the composition for hair may be formulated into hair shampoo, hair rinse, hair treatment, hair conditioner, hair tonic, hair lotion, hair cream, hair nutrition cosmetic water, hair essence, hair spray, hair gel, patch, spray, or hair pack, but is not limited thereto.

### 5. Composition for Providing the Use for Preventing Loss of Hair, Skin, or Fiber, including Cationic Polymer of the Present Invention, Crosslinking-mediating Component having Carboxyl Group or Amine Group, and Carbodiimide-Based Compound

In still another aspect of the present invention to achieve the objects above, there is provided a composition for providing the use for preventing the loss of hair, skin, or fiber, including the cationic polymer, a crosslinking-mediating component having a carboxyl group or an amine group, and a carbodiimide-based compound.

The cationic polymer used herein has a molecular weight of 600,000 to 2,500,000, and exhibits a property of preventing the loss of amphipathic internal components or internal components having a relatively low hydrophobicity in the washing process, and thus can provide the use for preventing the loss of hair, skin, or fiber components by being included together with a crosslinking-mediating component having a carboxyl group or an amine group, and a carbodiimide-based compound.

The composition providing the use for preventing the loss of hair, skin, or fiber components may include at least one selected from the group consisting of a crosslinking-mediating component having a carboxyl group or an amine group, and a carbodiimide-based compound; a cationic cellulose polymer; and a polar oil.

Additionally, the composition providing the use for preventing the loss of hair, skin, or fiber components may include a crosslinking-mediating component having a carboxyl group or an amine group, a carbodiimide-based compound, and a cationic cellulose polymer; a polar oil; or a cationic cellulose polymer and a polar oil.

Here, the "hair, skin, or fiber component" refers to a component constituting hair, skin, or fiber. More preferably, it refers to a component that constitutes the hair, skin, or fiber or is included therein, that is, an internal component. Here, it can be used interchangeably with internal components, constituents or components in the hair, skin, or fibers. Specific examples of internal components include keratin proteins, lipids, moisture, and trace elements, but are not limited to these components. The trace elements include, but are not limited to, nickel, titanate, iron, molybdenum, copper, cobalt, iron, *etc.* In the washing process, the internal components of the hair, skin, or fiber are lost due to friction pressure, adsorption by a surfactant, *etc.,* thereby causing a problem in that the hair, skin, or fiber may become rough or brittle. Additionally, there may also be a problem in that the strength is deteriorated due to the loss of the internal components, leading to breakage and cracking. The composition has an effect of preventing such internal components from being lost during the washing process, and thereby can keep the hair, skin, or fibers healthy.

The composition prevents the movement of the surfactant by crosslinking the components in the hair, skin, or fiber, thereby preventing elution thereof to the outside, and forms a protective layer against the surfactant that comes in contact from the outside during the washing process, suppressing the infiltration of the surfactant to the inside, ultimately preventing the loss of internal components. In particular, in one embodiment, when treating the composition according to an embodiment to the hair, it was confirmed that the loss of lipids in the hair was significantly prevented, and accordingly, the hair was further strengthened. In this aspect, the internal component may be lipid, and the composition may be a composition for preventing the loss of lipid in hair, skin, or fiber.

The inventors of the present invention divided the mechanism of the loss of lipids among the above internal components into two, and they have made a mechanistic prediction that the hydrophobic lipids with hydrophobic properties would be lost together by the movement of surfactant micelles in the hair, skin, or fiber, and in the case of lipids having amphipathicity or relatively low hydrophobicity, diffusion would occur from the inside of the hair toward the surface of the hair, and the lipid component eluted out of the hair would lost by binding to the surfactant, and have continued on research to prevent such phenomenon. As a result, it was confirmed that the loss of lipids could be effectively prevented using a crosslinking-mediating component having a carboxyl group or an amine group, and a carbodiimide-based compound; a cationic cellulose polymer; and/or a polar oil.

Here, the hydrophobic lipid group refers to ones having a relatively high hydrophobicity compared to other lipids, and examples thereof include squalene and wax ester, but are not limited thereto. In addition, amphipathic or relatively low hydrophobic lipid groups include fatty acids (myristyl acid, C₁₄, palmitic acid (C₁₆), stearic acid (C₁₈), oleic acid (C₁₈₌₁)), cholesterol, *etc.,* but are not limited thereto.

The hydrophobic lipid group referred herein is a lipid whose solubility in water at room temperature is indicated as insoluble as indicated by CAMEO Chemicals, and the lipids in the relatively low hydrophobic group is a substance which is not insoluble. In fact, these materials have a measurable value for solubility in water at room temperature. For example, myristyl acid has a solubility of 22 mg/L (Handbook of Aqueous Solubility Data, CRC Press LLC, FL. (2003), p. 987), palmitic acid has a solubility of 0.04 mg/L (Aust J Chem 19: 2281-4 (1966)), stearic acid has a solubility of 0.597 mg/L (Handbook of Chemistry and Physics 86TH Edition. CRC Press, FL (2005), p. 3-462), oleic acid has a solubility of 0.01 mg/L (US EPA; Estimation Program Interface (EPI) Suite. Ver.3.12. Nov 30, 2004), and cholesterol has a solubility of 0.095 mg/L (The AQUASOL dATAbASE of Aqueous Solubility. Ver 5. Tucson, AZ: Univ AZ, College of Pharmacy (1992)).

The composition may include a crosslinking-mediating component, which has a carboxyl group or an amine group, and a carbodiimide-based compound.

The crosslinking-mediating component having an carboxyl group or an amine group means a component that serves to form a crosslink between amino acids in hair, skin, or fiber, and it may play a role in forming a crosslink by directly binding to the amino acid, or in mediating therebetween so that the amino acids can be bound to each other. Specifically, it can bind to a carbodiimide-based compound to form a crosslink between internal components. Alternatively, one side of the mediating component may directly bind to the internal component, and another side may bind to a carbodiimide-based compound to form a crosslink between the internal components. The composition including a crosslinking-mediating component, which has a carboxyl group or an amine group, and a carbodiimide-based compound forms a crosslink between amino acids in hair, skin, or fiber, thereby effectively preventing the elution of the internal components to the surface or to the outside in the washing process by a surfactant. **In** particular, the loss of hydrophobic internal components can be effectively prevented through crosslinking the internal components of hair, skin, or fiber.

As confirmed in the specific Test Example, 60% of the lipid in the hair was lost when washing was performed 10 times using a conventional washing solution, while the degree of loss of hydrophobic lipid components was lowered even after performing washing 10 times, when a crosslink was formed between the internal components of hair using the mediating component and the carbodiimide-based compound.

Here, the crosslinking-mediating component having a carboxyl group or an amine group may include all those which are capable of reacting with biological amino acids on the protein surface of hair, skin, or fibers and which include carboxyl groups or amine groups. Specifically, the crosslinking-mediating component having a carboxyl group or an amine group may include at least one selected from the group consisting of natural extracts, amino acids, peptides, proteins, polymers, silicones, fatty alcohols, fatty acids, waxes, esters, and derivatives thereof, but is not limited thereto. The crosslinking-mediating component of the present invention forms a covalent bond with a protein residue (thiol, hydroxyl, carboxyl, or amine) of hair, skin, or fiber, thereby enabling crosslinking between the internal components; further, the crosslinking-mediating component of the present invention also increases the reaction efficiency with the carbodiimide-based compound, allowing the crosslinking between the internal components to be formed more efficiently.

The natural extract may include a *Rhodiola rosea* extract, a *Camellia japonica* leaf extract, ursolic acid, a *Rhus javanica* extract, an algae extract, a *Helianthus annuus* seed extract, *a Sophora flavescens* extract, a *Panax ginseng* root extract, a *Coptis chinensis* root extract, a *Calendula officinalis* extract, *Betula platyphylla* sap, a *Betula alba* extract, a *Zanthoxylum bungeanum* maxim extract, a *Luffa* cylindrical extract, a *Monarda didyma* extract, a *Chamaecyparis obtusa* extract, a *Rhodiola rosea* extract, a *Sophora flavescens* extract, an *Atractylodes* rhizome extract, a *Centella asiatica* extract, a *Coptis chinensis* extract, red *ginseng* root water, a *Fritillaria ussuriensis* extract, a *Convallaria keiskei* extract, a honeycomb extract, a cassis extract, a pomegranate extract, a lemon extract, a pine bud extract, a green tea extract, a broccoli extract, a honey extract, a cranberry extract, a berry extract, a lavender extract, a lentil extract, a ginger water extract, *etc.* The protein and the peptide may be a protein and a peptide which are obtained from Chun-zam silk, silk, polylysine, algae, wool, hair, or wheat.

The amino acid may include glycine, alanine, valine, leucine, isoleucine, threonine, serine, cysteine, cystine, methionine, aspartic acid, asparagine, glutamic acid, diiodotyrosine, lysine, arginine, histidine, phenylalanine, tyrosine, tryptophan, proline, oxyproline, *etc.*

The peptide may be a peptide consisting of 2 to 200 amino acids, and the protein may include keratin, collagen, gelatin, a vegetable protein, a hydrolysate thereof. The keratin is preferably hydrolyzed keratin, and more preferably has a molecular weight of 200 Da to 150,000 Da.

The polymer may include a linear/branched chain-type or network-type polymer compound having a molecular weight of about 1,000 to 1,000,000, and may include a double bond or various ring-structural substituents between carbon atoms as necessary. Additionally, it is preferably included in the molecule at least one residue having reactivity in order to attach a bioreactive functional group such as -COONa, -COOK, -COOH, -NH₂, -NHR, -NR₂, -Cl, -Br, -I, or -F for easily attaching the bioreactive functional group to any one terminus of the molecule. More preferably, a compound which is a linear/branched chain-type polymer having about 10,000 to 500,000 carbon atoms and includes in the molecule at least one residue with reactivity such as -COONa, -COOK, -COOH, -NH₂, -NHR, -NR₂, -Cl, -Br, - I, or -F for easily attaching the bioreactive functional group to any one terminus of the molecule may be used. For example, the polymer may be an amphoteric polymer such as an amino acid polymer, such as polylysine, a polyamine polymer, a polycarboxylic acid polymer, a methacryloyl ethyl betaine/methacrylate copolymer, or an octylacrylamide/acrylate/butylaminoethyl methacrylate copolymer; a nonionic polymer such as polyvinylpyrrolidone, a PVP/vinyl acetate (VA) copolymer, a PVP/dimethylaminoethyl methacrylate copolymer, or polyurethane; and an anionic polymer such as an acrylate/methacrylate copolymer or a VA/crotonate/vinyl neodecanoate copolymer, but is not limited thereto.

The silicone may be, for example, a compound such as dimethicone, trimethicone, phenyl trimethicone, amodimethicone, amodi phenyl trimethicone, amodi-penta phenyl trimethicone, dimethylpolysiloxane, methylphenylpolysiloxane, decamethylcyclopentasiloxane, methyl trimethicone, phenyl trimethicone, methicone, cyclomethicone, an alkyl methyl siloxane, dimethicone copolyol, or trimethylsilylamodimethicone, but is not limited thereto.

The fatty alcohol is a C_{10 to 50} linear/branched fatty alcohol compound, and examples thereof preferably include lauryl alcohol, cetyl alcohol, stearyl alcohol, isostearyl alcohol, behenyl alcohol, *etc.,* but is not limited thereto.

The fatty acid is a C_{10 to 50} linear/branched fatty acid compound, and examples thereof preferably include 18-methyl eicosanoic acid, lauric acid, stearic acid, isostearic acid, *etc.,* but is not limited thereto.

The wax may be, for example, candelilla wax, carnauba wax, ricebran wax, beeswax, lanoline, ozokerite, ceresin wax, paraffin wax, microcrystalline wax, polyethylene wax, *etc.,* but is not limited thereto.

The ester may be, for example, isopropyl myristate, butyl myristate, isopropyl palmitate, ethyl stearate, isopropyl linoleate, decyl myristate, cetyl myristate, cetyl palmitate, hydrogenated polyisobutene, *etc.,* but is not limited thereto.

Here, the carbodiimide-based compound includes all compounds having a N=C=N structure, and more specifically, it may be selected from the group consisting of N,N'-methylene-bis-(4-isocyanatocyclohexane)-, homopolymer, polyethylene glycol mono-Me-ether-blocked; N,N'-dicyclohexylcarbodiimide; N,N'-diisopropylcarbodiimide; N-ethyl-N'(3-dimethylaminopropyl)carbodiimidehydrochloride; N-cyclohexyl, N'-isopropylcarbodiimide; N-tert-butyl, N'-methylcarbodiimide; N-tert-butyl, N'-ethylcarbodiimide; N,N'-dicyclopentylcarbodiimide; bis[[4-(2,2-dimethyl-1,3-dioxolyl)]methyl]carbodiimide; N-ethyl, N-phenylcarbodimide; N-phenyl,N-isopropylcarbodiimide, and derivatives thereof, but is not limited thereto.

The crosslinking-mediating component and the carbodiimide-based compound can form crosslinks in two ways. Aspartic acid and glutamic acid, which are amino acids having a carboxyl group on the protein surface of hair, skin, or fiber, primarily react with a carbodiimide-based compound to form a reactive ester, followed by reacting with a crosslinking-mediating component having an amine residue to form a covalent bond. Alternatively, a crosslinking-mediating component having a carboxyl group may primarily react with a carbodiimide-based compound to form a reactive ester, followed by reacting with an amino acid having an amine residue in the living body to form a covalent bond.

In a preferred embodiment, the crosslinking-mediating component having a carboxyl group is directly converted into a reactive ester crosslinking-mediating component having a bioactive reactivity, or an amino acid (e.g., aspartic acid, glutamic acid) existing in excess in the hair (17.5% to 21.9%) is converted into an ester (reactive hair-strengthening component) using a carbodiimide-based compound that can be targeted by a crosslinking-mediating component including an amine group via reactive esterification to increase the reactive efficiency, thereby significantly increasing the effect.

Further, the crosslinking-mediating component may further include in the molecule a functional group such as carbodiimide, imidoester, aryl azide, diazirine, hydroxymethyl phosphine, pentafluorophenyl ester, pyridyl disulfide, sulfo-hydroxysuccinimide ester, alkoxy amine, hydrazide, haloacetyl, and azide, which may target other amino acids. In this case, more bonds can be formed, and the loss of internal components can be more effectively prevented.

In a more specific example, the composition may be prepared by primarily reacting a carbodiimide-based compound in the form of a polymer or a carbodiimide-based compound such as 1-ethyl-3-(3-dimethylaminopropyl) carbodiimide.HCl with a crosslinking-mediating component having a carboxyl group on the protein surface of hair/skin/fiber to form reactive esters, followed by reacting the formed reactive ester functional group again with a crosslinking-mediating component having an amine or reacting with an amine on the surface of the hair/skin/fiber to form a bond between the components, thereby obtaining an excellent hair/skin/fiber strengthening effect.

The reaction may preferably be carried out at pH 2 to 10, or at pH 3 to 9, or it may have the more excellent reaction efficiency when reacted in an acidic to weakly acidic aqueous solution of pH 4 to 5

The composition may include the crosslinking-mediating component having a carboxyl group or an amine group in an amount of 0.0001% to 10% by weight relative to the total composition and the carbodiimide-based compound in an amount of 0.0001% to 10% by weight relative to the total composition, or may contain the crosslinking-mediating component having a carboxyl group or an amine group in an amount of 0.01% to 10% by weight relative to the total composition and the carbodiimide-based compound in an amount of 0.01% to 10% by weight relative to the total composition.

The composition may include a polar oil and/or a cationic cellulose polymer. The composition can prevent the loss of components in hair, skin, or fiber through the polar oil and/or cationic cellulose polymer, and in particular, it can effectively prevent the loss of internal components having amphipathicity or low hydrophobicity. It prevents the loss of the internal components by binding of the polar oil or cationic cellulose polymer to a surfactant instead of the internal components during cleaning, thereby increasing the charge density of hair, skin, or fiber and reducing the interfacial tension of the surface of hair, skin, or fibers, which in turn reduces the contact angle of the internal components so that when the internal components having a weak hydrophobicity or amphipathicity or are exposed to the surface of hair, skin, or fibers, the loss thereof by the surfactant is prevented.

In particular, although the composition including the crosslinking-mediating component and the carbodiimide-based compound effectively prevented the loss of hydrophobic components, the effect of preventing the loss of relatively low hydrophobic or amphipathic components was relatively weak. Accordingly, the composition may further include a polar oil and/or a cationic cellulose polymer, together with the crosslinking-mediating component and the carbodiimide-based compound. In this case, the loss due to penetration of a surfactant and diffusion of internal components can be effectively prevented. In particular, when a polar oil and/or a cationic cellulose polymer is included together with the crosslinking-mediating component and the carbodiimide-based compound, it can be confirmed through an embodiment of the present invention that the effect of preventing the loss of internal components is significantly superior as compared to the case where each of the components are only treated.

Here, the oil may be transported to hair, skin, or fiber by coacervates which are formed when diluted in micelles of the cosmetic composition or water. Any oil can be included in the present invention without limitation as long as it can adsorb/penetrate into hair, skin, or fiber, and it may be preferably a polar oil. The polar oil may be included in an amount of 0.0001% to 10% by weight; 0.01% to 10% by weight; or 0.1% to 5% by weight in the composition.

Here, the degree of polarity can be evaluated according to the degree of polarity commonly used in the technical field of the present invention, and any of those classified as polar oils by a conventional evaluation method in the art can be included in the present invention. More specifically, the value inversely proportional to the interfacial tension value in water and oil may be evaluated according to the polarity evaluation method of Evonik, which is presented as a classification standard for oil polarity. More specifically, the interfacial tension of the oil may be evaluated by forming an oil droplet in a water tank using a drop shape analyzer (KRUESS, Germany), measuring the length and diameter of the droplet and the formed angle through image analysis, and calculating by the Young-Laplace equation of General Formula 1 below.

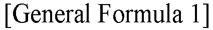

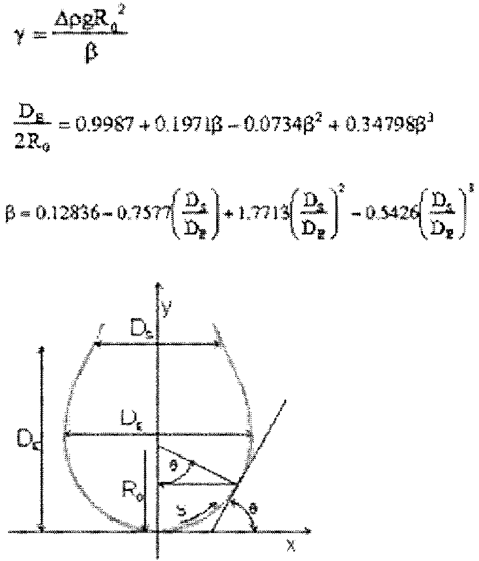

Herein, the polar oil may be an oil having an interfacial tension of 150 mN/m or less based on an interfacial tension value of 80 mM/m to 180 mM/m measured by the above-described method.

The polar oil may be selected from the group of saturated and/or unsaturated, branched and/or unbranched alcohols having a chain length of 3 to 30 carbon atoms, esters of saturated and/or unsaturated, branched and/or unbranched alkane carboxylic acids having a chain length of 3 to 30 carbon atoms, or may be selected from the group of saturated and/or unsaturated, branched and/or unbranched alcohols having a chain length of 3 to 30 carbon atoms or esters of aromatic carboxylic acids. Such ester oils may be selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate, dicaprylyl carbonate (Cetiol CC), cocoglyceride (Myritol 331), butylene glycol dicaprylate/dicaprate, dibutyl adipate, and synthetic, semi-synthetic, plant, animal, and mineral natural mixtures of these esters (e.g., jojoba oil), and more preferably, the oil may be those having an interfacial tension of 150 mN/m or less.

In one embodiment, when a washing including TEGOSOFT APM (PPG3-myristyl ether, manufactured by Evonik) as a polar oil was treated on the hair, it was confirmed that the oil penetrated into the hair, thereby preventing the loss of the low hydrophobic or amphipathic internal components during the washing process. Specifically, when a non-polar oil having an interfacial tension value exceeding 150 mN/m was used in the composition, the oil did not penetrate into the hair but rather remained on the surface, and was washed off with water during the washing process, and thus, the loss of lipids in the hair could not be prevented. However, the polar oil penetrated into the hair and as the low hydrophobic or amphipathic lipids diffused and moved toward the surface of the hair, the polar oil moved together, allowing the binding of the surfactant and the polar oil in the washing process, and thus, the loss of lipids by being eluted out of the hair can be prevented.

The cationic cellulose polymer has a nitrogen content of 2.3% to 3.2% by weight relative to the total weight of the polymer. When the nitrogen content in the polymer is 1.5% by weight or more, the loss of hair, skin, or fiber components may be more effectively prevented.

The cationic cellulose polymer may be one into which a cationic quaternized ammonium, in which ethylene oxide (EO) or OH bonded to carbons 2 and 3 of the glucose monomer in the cellulose skeleton is capable of hydrogen bonding, and the OH site bonded to carbon 6 is not capable of hydrogen bonding through a linker, is introduced.

The cationic cellulose polymer may preferably be a quaternized ammonium salt including hydroxyethyl cellulose and trimethylamine.

The cationic cellulose polymer may be a polymer in which ethylene oxide (EO) is substituted at α and β positions in the glucose ring.

The cationic cellulose polymer can be synthesized according to the method described in the present invention, and a commercially available cellulose polymer can be used. The commercially available cellulose polymer may be one having, for example, a total nitrogen content of 1.5% by weight or more among polyquaternium-10.

In one embodiment, when the cationic cellulose polymer has a nitrogen content of 2.8% by weight, it was confirmed that the loss of amphipathic internal components or internal components having a relatively low hydrophobicity was prevented in the washing process. As confirmed in the present invention, the amphipathic internal components or internal components having a relatively low hydrophobic are eluted by diffusion as time elapses in the washing process, and are lost upon contact with an anionic surfactant on the surface of the skin, hair or fiber, and thus, it was confirmed that the loss of lipids was prevented by binding of the cationic cellulose polymer to the anionic surfactant on the surface of skin, hair, or fibers, instead of lipids, upon contact. Additionally, as in the General Formula 1, it can be interpreted that when the surface charge is increased, the interfacial tension of the surface is lowered, so that the roll-up phenomenon of the lipid is suppressed and the contact angle (wetting) is reduced, thereby preventing the loss of lipids.

The cationic cellulose polymer has a molecular weight of 300,000 to 2,900,000, or 600,000 to 2,500,000, or 700,000 to 1,500,000.

The cationic cellulose polymer may produce a quaternized ammonium salt obtained by quaternization reaction of hydroxyethyl cellulose with chlorohydroxypropyl trimethylamine. Specifically, it may be prepared by adding sodium hydroxide and urea in a weight ratio of 7 to 9: 10 to 12: 75 to 85 (sodium hydroxide: urea: water) relative to water as a solvent, and then dissolving hydroxyethyl cellulose in a concentration of 1% to 3% by weight and further adding a quaternized ammonium-based compound. More specifically, it may be prepared by adding sodium hydroxide and urea in a weight ratio of 7.5: 11: 81.5 (sodium hydroxide: urea: water) relative to water as a solvent and then dissolving hydroxyethyl cellulose in a concentration of 2% by weight. The solvent of the above composition helps to improve the solubility and stability of hydroxyethyl cellulose, thereby helping to improve the uniformity of modification in the reaction process [Bi Xiong, Dissolution of cellulose in aqueous NaOH/urea solution: role of urea, Cellulose (2014) 21: 1183-1192]. Subsequently, the quaternized ammonium-based compound may be further added at an appropriate concentration (1 mole or higher moles compared to the glucose monomer of cellulose), and the reaction may be proceeded according to the room temperature or heated temperature conditions to carry out modification, thereby obtaining the cationic cellulose polymer.

There are three positions in the glucose ring of the cationic cellulose polymer, *i.e.,* C2, C3, and C6, that have hydroxyl groups which can be modified. Among these, the primary alcohol at C6 has minimum steric hindrance, and thus has excellent positional selectivity for modification reactions. In contrast, the difference in the preference for the modification reaction between C2 and C3 may vary depending on the conditions such as a solvent system [Mitsuru Abe, Regioselectivity in Acetylation of Cellulose in Ionic Liquids, Materials Science Inc. Nanomaterials & Polymers (2016) 1: 2474-2478].

In the case of adding EO to the α and β positions in the glucose ring, the addition of EO may be carried out by proceeding gas-phase polymerization using epoxide, spraying the reactants in the chamber thereto and then proceeding a heated and pressurized reaction. In the case of alkylation, a reaction may be performed so that a desired alkyl group can be added using a reactant having a halohydrin, aldehyde, or epoxide functional group at the end.

In one embodiment, EO was added to the α or β position in glucose of the cationic cellulose polymer substituted with trimethyl amine in the Chemical Formula 1.

Additionally, in order to increase the nitrogen content of the quaternized ammonium salt in the cationic polymer, the content of the degree of cationization can be controlled by repeatedly controlling the addition reaction process of the quaternized ammonium compound of Reaction 1 described above, ultimately controlling the nitrogen content, while changing the amount of trimethylamine used and controlling the temperature conditions, and this was confirmed through an elemental content analysis.

The composition may include the cationic cellulose polymer and/or polar oil in an amount of 0.001% to 10% by weight, 0.01% to 10% by weight, or 0.1% to 5% by weight relative to the total composition.

Preferably, the composition may include all of a crosslinking-mediating component having a carboxyl group or an amine group and a carbodiimide-based compound; a cationic cellulose polymer; and a polar oil. In the case of the composition, it was experimentally confirmed that the effect of preventing the loss of components in hair, skin, or fibers was remarkably excellent.

Specifically, in one embodiment, in the case of the composition including all three components, it was confirmed that the loss of hydrophobic lipids, low hydrophobic lipids, and amphipathic lipids was significantly prevented, and it was experimentally confirmed that the effect of the present invention was found to be more remarkable than the effects obtained when the composition includes a cationic cellulose polymer or a polar oil, and a crosslinking-mediating component having a carboxyl group/amine group and a carbodiimide-based compound.

The composition may include the cationic cellulose polymer in an amount of 0.0001% to 10% by weight; the polar oil in an amount of 0.0001% to 10% by weight; the sum of the crosslinking-mediating component having a carboxyl group or amine group and the carbodiimide-based compound in an amount of 0.0001% to 10% by weight, relative to the total composition, and each component may be included in an amount of 0.01% to 10% by weight. In the case of the composition including all of the above components, the effect of preventing the loss of internal components in hair, skin, or fibers is highly excellent.

The composition may further include at least one selected from the group consisting of a cationic polymer, a surfactant, and an oil.

The cationic polymer means a polymer additionally included in addition to the cationic cellulose polymer. For example, it may be any one or more selected from the group consisting of a cellulose-based polymer, a guar-based polymer, and a synthetic material-based polymer. The cellulose-based polymer may be JR125, JR400, JR30M, POLYQUAT-3000KC, LR-400R-LO, LR30M, Ucare LK, Catinal HC100, Catinal HC200, *etc.* In another specific example, the guar-based polymer may be guarhydroxypropyltrimonium chloride or hydroxypropyl guarhydroxypropyltrimonium chloride. The synthetic material-based polymer may be one or more selected from the group consisting of polyquaternium-22, polyquaternium-47, polyquaternium-53, dimethyldiallylammonium chloride polymer, acrylamide-dimethyldiallylammonium chloride copolymer, polyvinylpyrrolidone (PVP)-dimethylaminoethylmethacrylate copolymer, acrylic acid-dimethyldiallylammonium chloride copolymer, acrylamide-dimethylamino ethylmethacrylate methyl chloride copolymer and trimethylaminoethylmethacrylate polymer, *etc.,* but is not limited thereto. When the composition includes the cationic polymer, it may not include a cationic surfactant. In this case, it is possible to provide a composition having no skin irritation caused by a conventional cationic surfactant used to give a softening effect to hair, skin, or fiber.

As the 'surfactant', any one or more selected from the group consisting of an anionic surfactant, an amphoteric surfactant, and a nonionic surfactant may be used in the composition. The surfactant basically exhibits a washing power effect as used in conventional compositions. As the surfactant, those that form micelles together with C₁₀₋₂₀ alkyl group of alkyl cellulose described above may be used.

The anionic surfactant may include sodium lauryl sulfate, sodium laureth sulfate, ammonium lauryl sulfosuccinate, ammonium myreth sulfate, disodium laureth sulfosuccinate, disodium C₁₂-C₁₄ pareth-2 sulfosuccinate, or mixtures thereof. In addition, the amphoteric surfactant may be any one or more selected from the group consisting of betaine, cocamidopropyl betaine, amido propyl betaine, and coco amphocarboxy glycinate. Additionally, the nonionic surfactant may be any one or more selected from the group consisting of caprylyl/capryl glucoside, coco glucoside, lauramide DEA, cocamide DEA, cocamide methyl MEA, and glyceryl monostearate.

The anionic surfactant may be included in an amount of 1% to 30% by weight, specifically 3% to 20% by weight relative to the total weight of the composition. The amphoteric surfactant may be included in an amount of 0.1% to 20% by weight, specifically 2% to 15% by weight relative to the total weight of the composition.

An oil may be further used in the composition. Non-limiting examples of the oil may include components commonly referred to as silicones such as water-insoluble non-volatile dimethicone, cyclomethicone, aminated silicone, trimethylsilyl amodimethicone or vinyl silicone and derivatives thereof, vegetable oils, vegetable fats and oils, animal oils, animal oils, hydrocarbon oils, or synthetic ester oils, *etc.* The hydrocarbon oil may include liquid paraffin, isoparaffin, or hydrogenated polydecene, and the ester oil may include isopropyl myristate, isopropyl palmitate, isostearyl isostearate, C₁₂₋₁₅ alkyl benzoate, triethylhexanoin, squalane, palm oil, *Olea europaea* oil, PPG-3 caprylyl ether, capric/caprylic triglyceride, isostearyl isostearate, *Cocos nucifera,* polyglyceryl-6 octacaprylate, hydrogenated polydecene, *Simmondsia Chinensis* seed oil, DI-C₁₂₋₁₃ alkyl malate, *etc.,* but is not limited thereto.

The composition may be used as a shampoo. In this case, the composition may further include additional adjuvants used in the art within a range that does not impair the object of the present invention so as to further enhance the conditioning effect. For example, it may further include any one or more selected from the group consisting of fatty substances, organic solvents, solubilizing agents, thickening agents, gelling agents, softening agents, antioxidants, suspending agents, stabilizing agents, foaming agents, flavoring agents, surfactants, water, ionic or non-ionic emulsifiers, fillers, sequestering agents, preservatives, vitamins, blockers, wetting agents, essential oils, dyes, pigments, hydrophilic or lipophilic active agents, *etc.* commonly used in hair compositions.

Additionally, the composition may further include additives that provide beneficial properties to the human body. For example, it may include additives for imparting beneficial properties to the human body such as washing, volumizing, trimming, protection, blocking, moisturizing, dyeing, coloring, discoloration, restriction, deodorization, antiseptic, cooling, hair removal, hair growth, anti-dandruff, hair loss prevention, hair tonic, anti-inflammatory, fragrance, aroma, whitening, anti-aging, wrinkle improvement, astringency, relaxation, shrinkage, sebum control, keratin exfoliation, sterilization, antiphlogistic, antipruritic, deodorization, antihistamine, anti-seborrhea, blood circulation promotion, UV protection, or skin metabolism promotion, *etc.*

In addition, the composition may further contain preservatives, thickeners, viscosity modifiers, pH modifiers, flavoring agents, dyes, or conditioning agents, *etc.,* which can be commonly used as components of hair compositions, and these can be commercially and easily purchased and used. Examples of preservatives include benzoic acid and salts, methyl paraoxybenzoate, a mixture of methylchloroisothiazolinone or methylisothiazolinone (trade name: Kathon CG, manufacturer: The Dow Chemical Company). As a thickener and viscosity modifier, hydroxypropylmethylcellulose, hydroxymethylcellulose, sodium chloride, ammonium chloride, propylene glycol, hexylene glycol, sodium xylene sulfonate, or ammonium xylene sulfonate, etc. may be used. As a pH modifier, citric acid, sodium hydroxide, or triethanolamine may be used. As a dye, water-soluble tar color, etc. may be used. Further, as a conditioning agent, animal and vegetable extracts, proteins and protein derivatives, fatty acids, *etc.* may be used.

The term "bending strength" as used herein to express the effect means the degree of becoming strong as something is not well bent, and is a slightly different characteristic from stiffness caused by an increase in the frictional force on the surface. As a standard for measuring the bending strength, the 'bending strength' referring the degree of becoming strong as the longitudinal axis is not well bent is used. As the bending strength increases, the strength of hair increase. In a specific Test Example of the present invention, the shampoo composition was treated to the hair and rinsed so as to evaluate the bending strength using a bending strength evaluation device. As a result, it was confirmed that the bending strength of the hair treated with the composition was higher than the bending strength of the untreated hair.

The composition can effectively prevent the loss of internal components in hair, skin, or fibers during the washing process. In this aspect, the present invention provides a composition for treating hair, skin, or fiber that can prevent the loss of internal components.

The composition for treating hair, skin, or fiber may be one for preventing the loss of lipids in hair, skin, or fiber.

The composition may be treated alone for the above-mentioned purposes, or may be treated in a form included in washing agents, cosmetics, or preparations for external use.

In this aspect, the present invention provides a washing composition including the composition for preventing the loss of hair, skin, or fiber components. The washing composition is intended to include both a composition for washing the human body, including skin and hair, and a composition for washing fibers or objects including clothes, dishes, *etc.* The washing composition may be commercialized into a washing agent, and examples of the washing agent include cleansing foam, cleansing lotion, cleansing cream, body lotion, body cleanser, disinfectant cleaner, shower foam, cleaning tissue, detergent soap, hand wash, detergent, conditioning agent, face wash, or shampoo, but are not limited thereto.

Additionally, the present invention provides a cosmetic composition including the composition for preventing loss of hair, skin, or fiber components. The cosmetic composition may further contain a cosmetically acceptable carrier, diluent, adjuvant, colorant, stabilizer, flavor, surfactant, oil, moisturizer, alcohol, thickener, antioxidant, pH modifier, UV blocker for ease of use and handling. When used as a composition for external use on the skin, the composition may be used in any form that can be applied to skin such as liquid, oil, cream, ointment, stick, pack, paste, powder, *etc.* These may be used alone or in combination of two or more thereof.

When the cosmetic composition is formulated into cosmetics, it may include a known dermatologically acceptable excipient that acts as a carrier for the active ingredients. Specifically, a reference can be made to the contents disclosed in International cosmetic ingredient dictionary, 6th ed., The cosmetic, Toiletry and Fragrance Association, Inc., Washington, 1995.

The composition may further include an acceptable carrier, diluent, adjuvant, colorant, stabilizer, flavor, surfactant, oil, moisturizer, alcohol, thickener, antioxidant, pH modifier, UV blocker for ease of use and handling. Specific examples thereof include those conventionally used in the technical field of the present invention.

Here, the composition may be in the form of a general emulsifying formulation or solubilizing formulation. It may be formulated into powder, emulsion, suspension, oil, spray, ointment, cream, paste, gel, foam or liquid, and may be solid or semi-solid upon drying or concentration. As a specific example, the composition of the present invention may be any one formulation selected from the group consisting of shampoo, hair conditioner, hair lotion, hair essence, hair gel, hair pack, patch, and spray.

When the composition is commercialized for dermatological treatment or improvement, the composition may include each component at a high concentration as compared to when commercialized as a cosmetic product that is routinely applied to skin. When the composition is commercialized as cosmetics, in the case of a wash-off type cosmetic such as a make-up removing agent, a washing agent, *etc.,* in which the active ingredients remain on the skin for a short period of time, it may contain a relatively high concentration of each component. In contrast, in the case of a leave-on type cosmetic such as cosmetic water, milky lotion, cream, essence, *etc.,* in which the active ingredients remain on the skin for a long period of time, it is safe to include each component at a lower concentration than wash-off type cosmetics.

### [Advantageous Effects]

The cationic cellulose polymer of the present invention can be utilized for various purposes by controlling the molecular weight.

The composition including the cationic cellulose polymer of the present invention, which exhibits the property of imparting flexibility to hair, skin, or fiber, prevents the cumulative adsorption of the polymer onto the hair and fiber, even when repeatedly applied to the hair and fiber, improves the smoothness of the hair, and reduces the stiffness of the hair and fiber, thereby providing a remarkably excellent conditioning effect of hair.

The composition including the cationic cellulose polymer of the present invention, which exhibits the property of being cumulatively adsorbed onto hair, fiber, or skin, can exhibit the hair dye-strengthening effect by binding active ingredients such as a hair dye.

The composition including the cationic cellulose polymer of the present invention, which exhibits the property of being substituted by a cationic polymer already adsorbed onto hair, fiber, or skin and adsorbed, can exhibit the effect of continuously transferring active ingredients to the substrate with excellent efficiency.

The compositions including the cationic cellulose polymer of the invention, which exhibits the property of preventing the loss of amphipathic internal components or relatively low hydrophobic internal components in the washing process has effects of protecting hair, skin, or fibers from damage and irritation resulting from the washing process and maintaining health.

### [Brief Description of Drawings]

Fig. 1 is a nanoscale image obtained by imaging the polymers of Comparative Example 8 and Example 2 with AFM.
Fig. 2 is a graph showing the film thicknesses of the polymers of Comparative Example 8 and Example 2 according to the number of adsorptions.
Fig. 3 shows numerical values obtained by evaluating the changes in adsorption thickness according to an increase in the rinsing power with AFM, illustrating values obtained by changing the adsorption condition of (A) Comparative Example 10 and (B) Example 2.
Fig. 4 is a graph showing the thickness of the polymer film adsorbed on mica by AFM according to the number of adsorptions. Fig. 4 (A) shows the thickness variation of Example 9, and Fig. 4 (B) shows the thickness variation of Comparative Example 23.
Fig. 5 is a confocal fluorescence microscope image. Fig. 5 (A) shows the bottom and top images of the adsorption layer of Example 9, and Fig. 5 (B) shows the bottom and top images of the adsorption layer of Comparative Example 23.
Fig. 6 is an image obtained by applying a dye to hair, and Fig. 6 (A) shows Example 9 and Fig. 6 (B) shows Comparative Example 23.

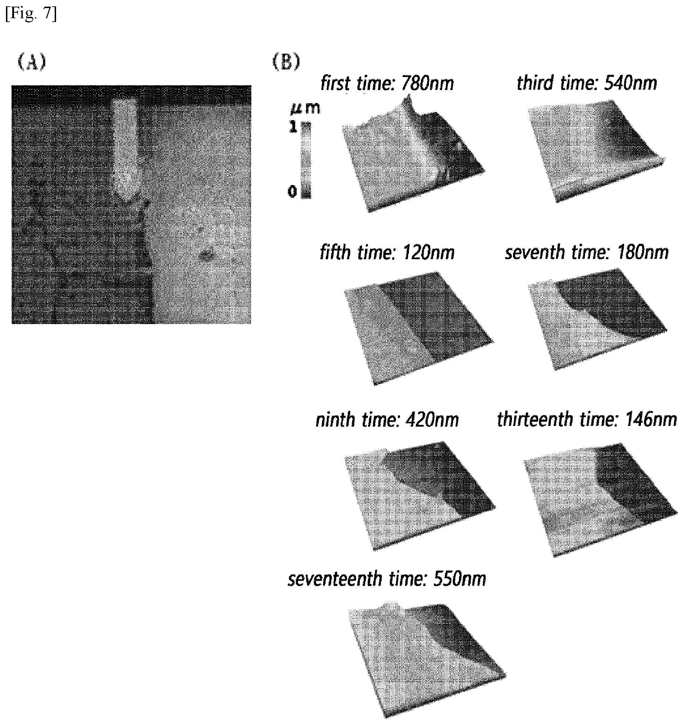

Fig. 7 is a nanoscale image of Comparative Example 25 obtained by imaging the polymer with AFM (XE-100, Park Systems, Korea). Fig. 7 (A) is an image at the time of AFM scanning, where the left side illustrates the polymer adsorption layer, and the right side illustrates the bottom of the mica disk as a substrate based on the boundary. The cantilever (NSC 36C, MikroMasch, Germany) measures the step between the polymer adsorption layer and the bottom of the mica disk. Fig. 7 (B) is an image with a z-axis resolution of 0.1 nm obtained through (A).
Fig. 8 is a graph showing the thickness of the adsorption layer according to the number of adsorptions. Fig. 8 (A) is a graph of Comparative Examples 25 to 27, and Fig. 8 (B) is a graph of Examples 11 to 13.
Fig. 9 is aa confocal fluorescence microscope image (LSM710, Carl Zeiss, Germany). Fig. 9 (A) is an image of Comparative Example 25, and Fig. 9 (B) is an image of Example 11, where the bottom illustrates the bottom image of the adsorption layer, and up illustrates the top image of the adsorption layer.
Fig. 10 is an image showing the results of using Comparative Example 25 (A) and Example 11 (B) for hair dyeing. The results obtained by using once, twice, and third time are shown in the order from left to right.
Fig. 11 is a schematic diagram showing the mechanism by which ingredients in the hair are lost by anionic surfactants. Group A shows components having low hydrophobicity or amphipathicity in the components of hair, and Group B shows components having hydrophobicity in the components of hair.
Figs. 12 (A) and 12 (B) are graphs showing the values obtained by quantifying the loss of lipids in hair by gas chromatography according to washing in an embodiment of the present invention as a relative value (%) to untreated hair. Experimental groups 1 to 9 are as follows in the order below: C_{14:} Myristyl acid, C_{16:} Palmitic acid, C_{18:} Stearic acid, C_{18 = 1}: oleic acid, cholesterol, squalene, C₁₄-C₁₄ lipid, C₁₄-C₁₆ lipid, and C₁₆-C₁₆, C₁₈-C₁₆ lipid.
Fig. 13 is a graph showing the results of confirming the effect of preventing the loss of lipids in hair by treatment with the composition according to an embodiment of the present invention. Group A shows lipids having low hydrophobicity or amphipathicity in the components of hair, and Group B shows lipids having hydrophobicity in the components of hair.

### [Detailed Description of Preferred Embodiments]

Hereinafter, the present invention will be described in detail by way of Examples to help understanding of the present invention. However, the Examples according to the present invention may be modified in various forms, and the scope of the present invention should not be interpreted as being limited thereto. These examples of the present invention are provided to more fully explain the invention to those having ordinary knowledge in the art to which the invention belongs.

### 1. Preparation of Cationic Polymer

In the following Examples and Comparative Examples, raw materials synthesized by the present inventors were used, and raw materials manufactured by the Dow Chemical Company were used as needed. The molecular weight was controlled by varying the content of ethyl cellulose in the polymerization process, and the nitrogen content was controlled by varying the amount of trimethylamine used to eventually control the amount of cationization degree.

A quaternized ammonium salt obtained by a quaternization reaction of hydroxyethyl cellulose with chlorohydroxypropyl trimethylamine was prepared. Specifically, sodium hydroxide and urea as solvents were added at a concentration in a weight ratio of 7.5: 11: 81.5 relative to water, and then, hydroxyethyl cellulose was dissolved therein at a concentration of 2% by weight. Thereafter, the quaternized ammonium-based compound was further added at an appropriate excess concentration (molar number of 3, 6, or 9 times relative to the glucose ring of cellulose) and the reaction was carried out according to temperature conditions (25, 45, or 60°C) for modification to prepare a quaternized cationic cellulose polymer of Chemical Formula 1.

Further, referring to Reference Document (WO 2016085099 A1), EO addition or alkylation was carried out to prepare a structure including an alkyl group. Specifically, the addition of EO was carried out by proceeding gas-phase polymerization in order to introduce ethylene oxide (EO) to the α and β positions in the glucose ring of the cationic cellulose polymer using an epoxide, spraying the reactants in the chamber thereto, and then proceeding a reaction by applying temperature and pressure. In the case of alkylation, the reaction was carried out using reactants having a halohydrin, aldehyde, or epoxide functional group at the end so as to introduce a desired alkyl group, and as a result, the OH groups at the α and β positions in the glucose ring were substituted with alkyl.

### 2. Property of Imparting Flexibility to Hair, Skin, or Fiber of Cationic Polymer

### (1) Change in Thickness of Cumulative Adsorption According to Substituents at α and β Positions During Polymer Adsorption

In order to confirm the cumulative adsorption characteristics of polymers and the impact of rinsing power on the polymers according to the substituent groups at the α and β positions in the glucose ring of the cationic polymer, polymer samples of Examples and Comparative Examples in which C2 (α) and C3 (β) were substituted in the glucose ring were prepared, as shown in Table 1. In Comparative Example 1, distilled water was used as a control group. The cationic cellulose polymers of Examples and Comparative Examples were synthesized with a molecular weight of 800,000 and a nitrogen content (% by weight) of 2.7% by weight. Table 1 below shows substitution positions and substituent groups.

**[Table 1]**

| Samples | Example1 | | Example 2 | | Example3 | | Comparative Example2 | | Comparative Exampl e3 | | Comparative Exampl e4 | | Comparative Exampl e5 | | Comparative Exampl e6 | | Comparative Example 7 | |
|---|---|---|---|---|---|---|---|---|---|---|---|---|---|---|---|---|---|---|
| Substition | α | β | α | β | α | β | α | β | α | β | α | β | α | β | α | β | α | β |
| Substituent Groups | EO | OH | Divalent or higher valent EO | OH | Divalent or higher valent EO | Mono valent or higher valent EO | OH | OH | Alkyl havi ng C5 or less | OH | Alkyl havig C5 Or more and C10 or less | OH | Alkyl havig C10 Or more and C18 or less | OH | Alkyl havig C18 or more | OH | Quaternized ammonium | EO |

In order to evaluate the adsorption state of cationic polymers, mica, which has a Zeta potential of tens of mV, generally similar to that of hair and wool, was selected as the substrate using the method practiced by many companies including Dow Corning, a raw-material manufacturing company for conditioning agents, and the cationic polymers were adsorbed onto the mica substrate to determine the adsorption characteristics of the polymers. Since there exist many variables such as an interval between hair cuticles, cuticle thickness, cuticle state, hair hydrophobicity, hair thicknesses, etc. for each hair even within the same person, the method of comparing the cationic polymers by directly adsorbing the same onto the hair cannot be a proper evaluation method. Therefore, in this experiment, cumulative adsorption was evaluated by selecting mica discs as a substrate and measuring the adsorption thickness of the cationic polymers.

12 mm diameter mica discs (TED PELLA, Canada) were used as an adsorbent. The polymers were placed and adsorbed onto only half of the mica discs using 10 ml of distilled water containing 0.5% of polymers, and then placed and washed with distilled water. The amount of polymer adsorption is determined by the electrical attraction and repulsion between the polymers, the mica, and the polymers adsorbed onto the mica. Since the desorption proceeds due to the hydrophilicity of the polymers and water, the change in the polymer content is not a factor affecting the adsorption amount. Therefore, the polymer content of 0.5% commonly used in the shampoo was selected as an experimental condition.

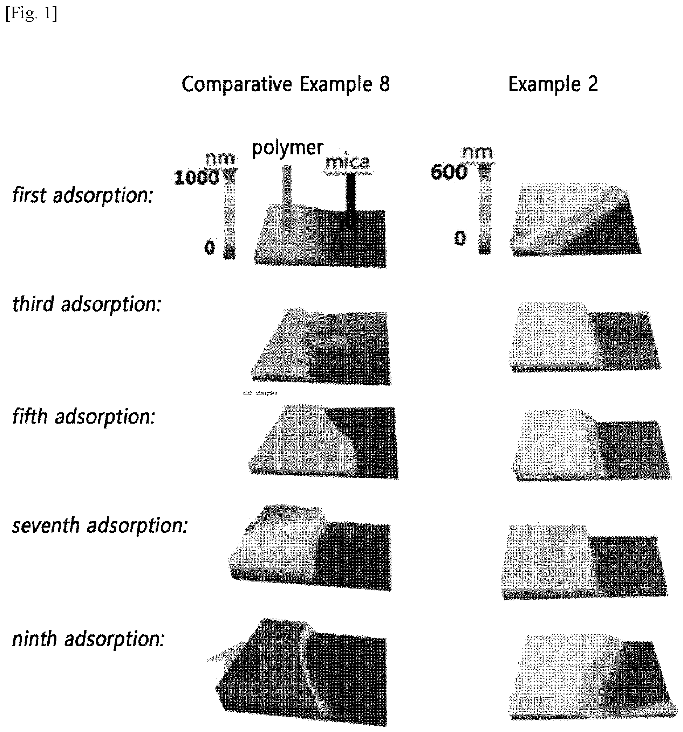

The interface between the adsorbed polymer layer and the mica bottom was observed by the atomic force microscopy (AFM, Model Systems XE-100, Korea) using the topography of the contact mode of the tip (NSC 36C, Mikro Masch, Germany), and the thickness thereof was evaluated as shown in Fig. 1, and the average thickness is shown in Table 2.

Comparative Example 1 in Table 2 shows the measured values of the thickness of mica immersed only in distilled water containing no polymer. As can be seen from the results of Comparative Example 1, the cumulative adsorption is resulted from the polymer adsorption, and the thickness difference is resulted from the difference in the polymer adsorption amount.

Comparative Example 2 is a cellulose polymer having a molecular weight of 800,000 and a nitrogen content of 2.7% by weight, wherein α is an OH group and β is an OH group in the Chemical Formula 1, and Comparative Example 3 is a cellulose polymer having a molecular weight of 800,000 and a nitrogen content of 2.7% by weight, wherein α is an alkyl group having 5 carbon atoms or less and β is an OH group in the Chemical Formula 1.

Comparative Example 4 is a cellulose polymer having a molecular weight of 800,000 and a nitrogen content of 2.7% by weight, wherein α is an alkyl group having 5 to 10 carbon atoms and β is an OH group in the Chemical Formula 1, and Comparative Example 5 is a cellulose polymer having a molecular weight of 800,000 and a nitrogen content of 2.7% by weight, wherein α is an alkyl group having 10 to 18 carbon atoms, and β is an OH group in the Chemical Formula 1.

Comparative Example 6 is a cellulose polymer having a molecular weight of 800,000 and a nitrogen content of 2.7% by weight, wherein α is an alkyl group having 18 or more carbon atoms and β is an OH group in the Chemical Formula 1, and Comparative Example 7 is a cellulose polymer having a molecular weight of 800,000 and a nitrogen content of 2.7% by weight, wherein α is a quaternized ammonium and β is an OH group in the Chemical Formula 1, wherein the quaternized ammonium is the same as the quaternized ammonium connected to the ethylene oxide (EO) group of the glucose ring C5 of the Chemical Formula 1.

Example 1 is a cellulose polymer having a molecular weight of 800,000 and a nitrogen content of 2.7% by weight, wherein α is an ethylene oxide (EO) group and β is an OH group in the Chemical Formula 1, and Example 2 is a cellulose polymer having a molecular weight of 800,000 and a nitrogen content of 2.7% by weight, wherein α is a divalent or higher valent ethylene oxide (EO) group and β is an OH group in the Chemical Formula 1.

Example 3 is a cellulose polymer having a molecular weight of 800,000 and a nitrogen content of 2.7% by weight, wherein α is a divalent or higher valent ethylene oxide (EO) group and β is a monovalent or higher valent EO group in the Chemical Formula 1. Table 2 shows thickness measurements according to polymer conversion (unit: µm).

**[Table 2]**

| Nu mb er of ad sor pti on s | Exam ple1 | Exam ple2 | Exa mple 3 | Com parat ive Exa mple 1 | Com parat ive Exa mple 2 | Com parat ive Exa mple 3 | Comp arativ e Exam ple4 | Comp arativ e Exam ple5 | Comp arativ e Exam ple6 | Comp arativ e Exam ple7 |
|---|---|---|---|---|---|---|---|---|---|---|
| 0 | 0 | 0 | 0 | 0 | 0 | 0 | 0 | 0 | 0 | 0 |
| 1 | 0.562 | 0.525 | 0.53 8 | 0 | 0.58 3 | 0.63 5 | 0.682 | 0.735 | 0.625 | 0.373 |
| 2 | 0.672 | 0.620 | 0.62 2 | 0 | 0.68 2 | 0.74 8 | 0.713 | 0.815 | 0.735 | 0.367 |
| 3 | 0.725 | 0.447 | 0.52 7 | 0 | 0.69 2 | 0.94 5 | 0.824 | 0.863 | 0.795 | 0.318 |
| 4 | 0.430 | 0.378 | 0.37 4 | 0 | 0.72 6 | 1.13 3 | 0.924 | 0.928 | 0.835 | 0.283 |
| 5 | 0.472 | 0.358 | 0.34 6 | 0 | 0.80 1 | 1.28 5 | 1.015 | 1.139 | 0.935 | 0.273 |
| 6 | 0.452 | 0.374 | 0.34 8 | 0 | 0.84 0 | 1.42 8 | 1.215 | 1.257 | 1.015 | 0.235 |
| 7 | 0.448 | 0.387 | 0.35 0 | 0 | 0.83 4 | 1.62 3 | 1.368 | 1.367 | 1.213 | 0.231 |
| 8 | 0.428 | 0.389 | 0.34 2 | 0 | 0.88 3 | 1.93 2 | 1.626 | 1.547 | 1.254 | 0.217 |
| 9 | 0.463 | 0.391 | 0.34 7 | 0 | 0.89 2 | 2.04 5 | 1.935 | 1.782 | 1.269 | 0.169 |
| 10 | 0.438 | 0.381 | 0.36 2 | 0 | 0.90 2 | 2.15 3 | 2.035 | 1.824 | 1.330 | 0.105 |
| 11 | 0.452 | 0.378 | 0.36 0 | 0 | 0.93 7 | 2.32 6 | 2.386 | 1.946 | 1.394 | 0.061 |

From the results shown in Table 2, it can be seen that the thicknesses adsorbed in Examples 1 to 3 were constant. From this, it can be seen that the effect of preventing cumulative adsorption was exhibited when EO was added to carbon 2 or 3 of the glucose ring unit in the cellulose skeleton.

In the above Examples, the thickness of the adsorption layer increased during repeated adsorption from one to three times, implying that the cationic polymers were steadily adsorbed as a single layer onto the space above the negatively charged surface. The adsorption thickness of the cation polymers decreased from 3 and 4 times of adsorption, indicating that the anionic surface was fully covered by the adsorption of the cation polymers. When the cationic polymers were adsorbed onto a single layer having an overall negative charge, the adsorption force was reduced due to the cationic repulsive force with the already adsorbed polymers. Therefore, the polymers of the adsorption layer on the single layer were washed away by the hydrophilic force with water in the rinsing process.

In Comparative Examples 2 to 6, it was observed that the adsorption thickness of the adsorption layer was increased as the number of adsorptions was increased. Considering that the degree of cationization was similar compared to those of Examples 1 to 3, although the adsorption layer and the cationic polymer had a similar cationic repulsive force therebetween, it seems that the cumulative adsorption was proceeded with relatively reduced rinsing power with respect to the bonding relationship with water. In fact, it can be seen that the hydrophobicity increased as the length of the alkyl group increased, thereby reducing the hydrophilic force with water molecules, resulting in a cumulative adsorption phenomenon.

In Comparative Example 7, it can be seen that the thickness of the adsorption layer was remarkably reduced as the number of adsorptions increased. This may be illustrated as a phenomenon in which the cationic polymers adsorbed in the rinsing process after the polymer has been adsorbed onto the surface of the hair was washed away by binding to water molecules. That is, when two or more ammonium were present in the quaternized cellulose, the rinsing power was excessively increased, thereby preventing the adsorption of the polymer.

### (2) Change in Thickness of Cumulative Adsorption layer According to Molecular Weight and Degree of Cationization During Polymer Adsorption

In order to confirm the effect of the molecular weight on the adsorption characteristics of the cationic polymer, polymer samples of Examples and Comparative Examples having molecular weights and nitrogen contents (% by weight) as shown in Table 3 below were prepared. In Comparative Example 8, a Ucare polymer JR125 manufactured by the Dow Chemical Company was used, and the polymer of Comparative Example 9 was prepared. As the polymer of Comparative Example 10, a Ucare polymer JR30M manufactured by the Dow Chemical Company was used, and the polymers of Comparative Examples 11 to 14 were prepared. Table 3 below shows samples for evaluation of the thickness of cumulative adsorption layers.

**[Table 3]**

| Samp les | Exam ple 2 | Exam ple 4 | Exam ple 5 | Comp arativ e Exam ple 8 | Compar ative Exampl e 9 | Comp arativ e Exam ple 10 | Comp arativ e Exam ple 11 | Comp arativ e Exam ple 12 | Comp arativ e Exam ple 13 | Comp arativ e Exam ple 14 |
|---|---|---|---|---|---|---|---|---|---|---|
| Poly mer | Cellul ose | Cellul ose | Cellul ose | Cellul ose | Cellulos e | Cellul ose | Cellul ose | Cellul ose | Cellul ose | Cellul ose |
| Mole cular Weig ht | 800,0 00 | 1,800, 000 | 2,500, 000 | 250,0 00 | 250,000 | 800,0 00 | 1,800, 000 | 2,500, 000 | 3,000, 000 | 3,000, 000 |
| N% | 2.7 | 2.7 | 2.7 | 1.8 | 2.7 | 1.8 | 1.8 | 1.8 | 1.8 | 2.7 |

In Comparative Example 8, a cationic cellulose polymer having a molecular weight of 250,000 and a nitrogen content of 1.8% by weight was used, and in Comparative Example 9, a cationic cellulose polymer having a molecular weight of 250,000 and a nitrogen content of 2.7% by weight was used

In Comparative Example 10, a cationic cellulose polymer having a molecular weight of 800,000 and a nitrogen content of 1.8% by weight was used, and in Comparative Example 11, a cationic cellulose polymer having a molecular weight of 1,800,000 and a nitrogen content of 1.8% by weight was used.

In Comparative Example 12, a cationic cellulose polymer having a molecular weight of 2,500,000 and a nitrogen content of 1.8% by weight was used, and in Comparative Example 13, a cationic cellulose polymer having a molecular weight of 3,000,000 and a nitrogen content of 1.8% by weight was used.

In Comparative Example 14, a cationic cellulose polymer having a molecular weight of 3,000,000 and a nitrogen content of 2.7% by weight was used, and in Example 2, a cationic cellulose polymer having a molecular weight of 800,000 and a nitrogen content of 2.7% by weight was used.

In Example 4, a cationic cellulose polymer having a molecular weight of 1,800,000 and a nitrogen content of 2.7% by weight was used, and in Example 5, a cationic cellulose polymer having a molecular weight of 2,500,000 and a nitrogen content of 2.7% by weight was used. Table 4 below shows the results of evaluating the thickness of cumulative adsorption layers (unit: µm).

**[Table 4]**

| Nu mb er of ad sor ption s | Exam ple 2 | Exam ple 4 | Exam ple 5 | Comp arativ e Exam ple 8 | Comp arativ e Exam ple 9 | Com parat ive Exa mple 10 | Com parat ive Exa mple 11 | Comp arativ e Exam ple 12 | Comp arativ e Exam ple 13 | Comp arativ e Exam ple 14 |
|---|---|---|---|---|---|---|---|---|---|---|
| 0 | 0 | 0 | 0 | 0 | 0 | 0 | 0 | 0 | 0 | 0 |
| 1 | 0.525 | 0.552 | 0.589 | 0.253 | 0.385 | 0.36 3 | 0.74 3 | 0.737 | 0.933 | 1.291 |
| 2 | 0.620 | 0.783 | 0.843 | 0.327 | 0.483 | 0.47 3 | 0.52 8 | 0.748 | 0.982 | 1.425 |
| 3 | 0.378 | 0.483 | 0.503 | 0.527 | 0.627 | 0.66 3 | 0.83 5 | 0.964 | 1.067 | 1.283 |
| 4 | 0.378 | 0.419 | 0.479 | 0.592 | 0.593 | 0.73 0 | 0.72 5 | 1.089 | 1.263 | 1.532 |
| 5 | 0.358 | 0.485 | 0.495 | 0.683 | 0.705 | 0.87 3 | 1.35 6 | 1.125 | 1.472 | 1.853 |
| 6 | 0.374 | 0.483 | 0.467 | 0.795 | 0.864 | 0.94 8 | 1.63 2 | 1.236 | 1.628 | 1.902 |
| 7 | 0.387 | 0.538 | 0.519 | 0.877 | 0.935 | 1.04 8 | 1.85 3 | 1.467 | 1.836 | 2.105 |
| 8 | 0.389 | 0.513 | 0.506 | 0.963 | 0.864 | 1.29 4 | 2.03 3 | 1.774 | 1.929 | 2.075 |
| 9 | 0.391 | 0.496 | 0.542 | 1.043 | 0.926 | 1.38 5 | 2.18 5 | 2.086 | 2.148 | 2.532 |
| 10 | 0.381 | 0.551 | 0.553 | 1.142 | 1.426 | 1.49 2 | 2.53 2 | 2.332 | 2.436 | 2.938 |
| 11 | 0.378 | 0.527 | 0.548 | 1.204 | 1.583 | 1.58 5 | 2.96 3 | 2.406 | 2.839 | 3.502 |

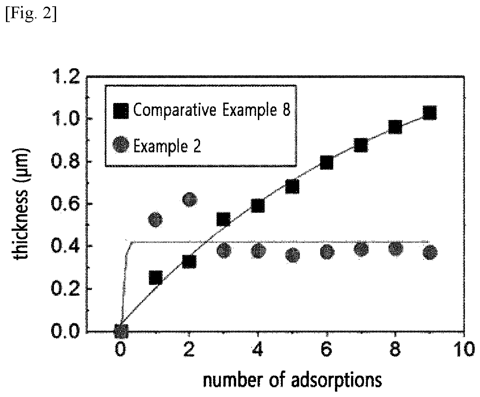

From the results shown in Table 4, it can be seen that the adsorbed thicknesses in Example 2 and Examples 4 and 5 were constant. It can be confirmed therefrom that the cumulative adsorption phenomenon could be prevented by adjusting the molecular weight and the nitrogen content (degree of cationization). Further, it was confirmed from the Comparative Examples 8 to 14 that the adsorption thickness increased when the number of adsorptions of the polymer was increased (Figs. 1 and 2).

It seems that when adsorbed onto the surface of the anionic hair, the polymers, due to the high degree of cationization, were uniformly adsorbed in a constant amount. In fact, in the Examples except Example 2 and Examples 4 and 5, the cases in which the thickness layer of the polymers was not uniform were also observed. In the Examples, it seems that the increase in the adsorption thickness when adsorption was carried out for 1 to 2 times attributes to the continued adsorption of the cationic polymers on the hair surface. From the third time of the adsorption, the thickness decreased and became uniform, and the adsorption was prevented by the mutual electric repulsive force between the cationic polymer layer adsorbed onto the hair and the cationic polymers to be adsorbed. Meanwhile, the portion onto which the polymer was adsorbed in a constant amount due to the molecular weight was formed, as the polymer adsorption layer with a high degree of cationization had hydrophilicity and was bound to water molecules, and washed away in the rinsing process. As a result, it seems that the cumulative adsorption phenomenon was prevented when the conditions of cellulose molecular weight in the range of 800,000 to 2,500,000 and nitrogen content of 2.7% by weight of Example 2 and Examples 4 to 5 were applied.

### (3) Change in Thickness of Cumulative Adsorption Layer According to Rinsing Power

In order to confirm whether the phenomenon of the cumulative adsorption prevention shown in Table 4 was caused by the rinsing power, and an experiment was conducted to confirm the adsorption thickness by changing the rinsing power, while polymer adsorption was conducted as shown in Table 5 under the adsorption conditions of Comparative Example 10 and Example 2. In the cases of Comparative Example 10 and Example 2, the molecular weight of each polymer was the same as 800,000, but their nitrogen content was different, which was 1.8 and 2.7% by weight, respectively. Table 5 below shows samples for evaluating the thickness of cumulative adsorption layers.

**[Table 5]**

| Experimental Category | Exper iment #1 | Exper iment #2 | Exper iment #3 | Exper iment #4 | Exper iment #5 | Exper iment #6 | Exper iment #7 | Exper iment #8 |
|---|---|---|---|---|---|---|---|---|
| Nitrogen Content | 1.8% (Com parati ve Exam ple10 ) | 1.8% (Com parati ve Exam ple10) | 1.8% (Com parati ve Exam ple10) | 1.8% (Com parati ve Exam ple10) | 2.7% (Exa mple 2) | 2.7% (Exa mple 2) | 2.7% (Exa mple 2) | 2.7% (Exa mple 2) |
| rpm | 0 | 20 | 40 | 60 | 0 | 20 | 40 | 60 |

Experiments #1 to #4 were performed with the polymer of Comparative Example 10, and Experiments #5 to #8 were performed with the polymer of Example 2. All experiments were carried out as follows: the polymers were adsorbed onto the mica discs, placed thereon, and then washed, and subsequently, each Petri dish was added to a shaker (Jeio Tech, SI-900R, Korea) and shaken at 20, 40, and 60 rpm to exhibit rinsing power. After varying the rinsing power, the thickness of the adsorption layer was measured and shown as in Table 6.

**[Table 6]**

| Exp erim ental Cate gory | Experim ent #1 | Experim ent #2 | Experim ent #3 | Experim ent #4 | Experim ent #5 | Experim ent #6 | Experim ent #7 | Experim ent #8 |
|---|---|---|---|---|---|---|---|---|
| 0 | 0 | 0 | 0 | 0 | 0 | 0 | 0 | 0 |
| 1 | 0.335 | 0.315 | 0.351 | 0.421 | 0.525 | 0.453 | 0.402 | 0.352 |
| 2 | 0.521 | 0.521 | 0.583 | 0.482 | 0.620 | 0.513 | 0.452 | 0.423 |
| 3 | 0.663 | 0.692 | 0.678 | 0.613 | 0.447 | 0.392 | 0.358 | 0.317 |
| 4 | 0.801 | 0.731 | 0.752 | 0.714 | 0.378 | 0.341 | 0.309 | 0.271 |
| 5 | 0.873 | 0.862 | 0.898 | 0.811 | 0.358 | 0.331 | 0.308 | 0.261 |
| 6 | 1.024 | 0.972 | 0.942 | 0.936 | 0.374 | 0.325 | 0.305 | 0.285 |
| 7 | 1.048 | 0.992 | 1.032 | 1.154 | 0.387 | 0.352 | 0.318 | 0.285 |
| 8 | 1.294 | 1.236 | 1.262 | 1.248 | 0.389 | 0.359 | 0.308 | 0.278 |
| 9 | 1.325 | 1.392 | 1.402 | 1.346 | 0.391 | 0.342 | 0.308 | 0.274 |
| 10 | 1.492 | 1.493 | 1.457 | 1.438 | 0.381 | 0.343 | 0.319 | 0.285 |
| 11 | 1.585 | 1.502 | 1.544 | 1.526 | 0.378 | 0.351 | 0.301 | 0.294 |

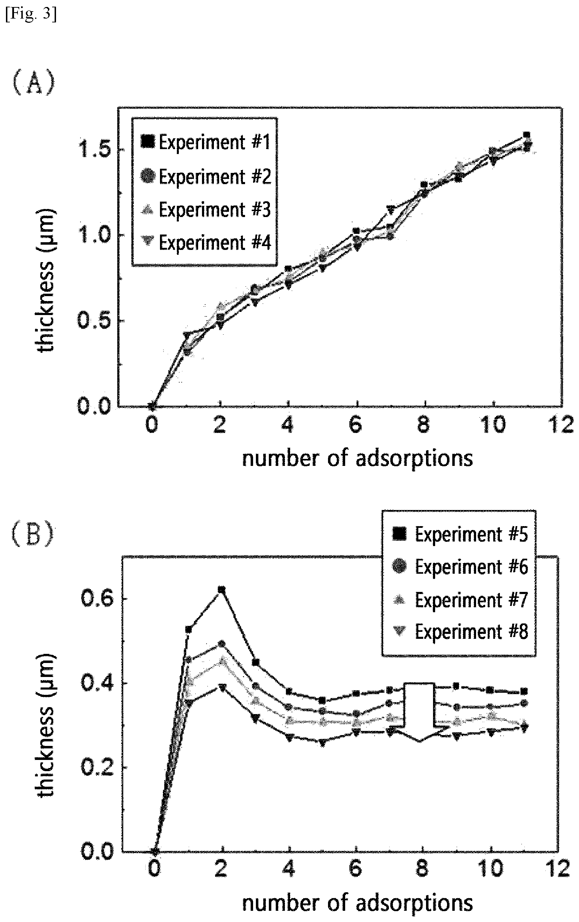

The thickness variations in Table 6 are shown as a graph in Fig. 3. Fig. 3 (A) shows the result of Experiment #1 to Experiment #4, and Fig. 3 (B) shows the result of Experiment #5 to Experiment #8. When (A), which has a relatively low nitrogen content, was compared to (B), which has a high nitrogen content, cumulative adsorption occurred. In this case, there was no variation of the adsorption thickness even when the rinsing power was increased. However, in the case where the cumulative adsorption was prevented as shown in (B), the adsorption thickness was decreased and thus varied with an increase in the rinsing power.

From the above results, it can be seen that the phenomenon of cumulative adsorption prevention of the adsorption layer was associated with the high-nitrogen polymer, and the high degree cationization, which reflects the high-nitrogen portion, and the hydrophilicity acting on the water play a crucial role in providing such effect.

That is, it was found that the cumulative adsorption phenomenon could be prevented by adsorbing polymers having a high nitrogen content in order to utilize the rinsing power. In other words, the adsorption of polymers having a high nitrogen content was proved to be effective in preventing cumulative adsorption by hydrophilicity

### (4) Change in Thickness of Cumulative Adsorption Layer According to Degree of Cationization During Polymer Adsorption

Since it was confirmed that cumulative adsorption did not occur in Example 2 and Examples 4 and 5 of Table 3, polymers were prepared by variously applying the degree of cationization based on the molecular weight of 800,000 of the cationic polymer of Example 2 as shown in Table 7. The degree of cationization is determined by the nitrogen content. Table 7 below shows samples for evaluating the thickness of cumulative adsorption layer according to the degree of cationization.

**[Table 7]**

| Samples | Example 6 | Example 7 | Comparative Example 15 | Comparative Example 16 | Comparative Example 17 |
|---|---|---|---|---|---|
| Polymer | Cellulose | Cellulose | Cellulose | Cellulose | Cellulose |
| Molecular Weight | 800,000 | 800,000 | 800,000 | 800,000 | 800,000 |
| N% | 2.3 | 3.0 | 1.7 | 2.0 | 3.3 |

With respect to the samples of Table 7, the evaluation of adsorption thickness was carried out according to the degree of cationization, and the results are shown in Table 8 below (unit: µm).

**[Table 8]**

| Number of adsorptions | Example 6 | Example 7 | Comparative Example 10 | Comparative Example 15 | Comparative Example 16 | Comparative Example 17 |
|---|---|---|---|---|---|---|
| 0 | 0 | 0 | 0 | 0 | 0 | 0 |
| 1 | 0.453 | 0.463 | 0.363 | 0.438 | 0.427 | 0.478 |
| 2 | 0.648 | 0.436 | 0.473 | 0.502 | 0.453 | 0.592 |
| 3 | 0.683 | 0.437 | 0.663 | 0.581 | 0.464 | 0.482 |
| 4 | 0.437 | 0.410 | 0.730 | 0.627 | 0.502 | 0.412 |
| 5 | 0.378 | 0.408 | 0.873 | 0.653 | 0.489 | 0.403 |
| 6 | 0.354 | 0.431 | 0.948 | 0.684 | 0.517 | 0.382 |
| 7 | 0.387 | 0.420 | 1.048 | 0.699 | 0.537 | 0.371 |
| 8 | 0.348 | 0.397 | 1.294 | 0.753 | 0.583 | 0.352 |
| 9 | 0.349 | 0.392 | 1.385 | 0.799 | 0.573 | 0.321 |
| 10 | 0.338 | 0.441 | 1.492 | 0.831 | 0.628 | 0.296 |
| 11 | 0.376 | 0.404 | 1.585 | 0.863 | 0.649 | 0.269 |

As shown in Table 8, in the case of Comparative Example 10, Comparative Examples 15 and 16, the adsorption thickness increased with an increase in the number of adsorptions, and thus, it can be seen that the cumulative adsorption phenomenon occurred because the degree of cationization due to the electric repulsive force generated during the adsorption described above was insufficient.

In the cases of Examples 6 to 7, no increase in adsorption thickness according to an increase in the number of adsorptions was observed, and it was confirmed that cumulative adsorption was prevented when the nitrogen content was in a range of 2.3 to 3.2% by weight while the molecular weight was 800,000. It can be seen therefrom that, in the case of a low molecular weight, the additional adsorption could be suppressed by the electric repulsive force only when the degree of cationization reaches a certain level.

However, in the case of Comparative Example 17 in which the nitrogen content determining the degree of cationization was higher than 3.2% by weight based on the weight of the polymer, although the cumulative adsorption phenomenon was prevented, the adsorption layer was rather decreased as the number of adsorptions increased, resulting in the interference with the amount of adsorption which imparts a conditioning effect. Accordingly, it was determined that desorption resulted from hydrophilicity with water in the rinsing process due to a high degree of cationization.

### (5) Change in Thickness of Cumulative Adsorption Layer According to Polymer Base During Polymer Adsorption

For the evaluation of the thickness of the cumulative adsorption layer according to polymer bases, the cumulative adsorption phenomenon was observed with respect to the cellulose and other polymers shown in Table 9 below, and the results are shown in Table 10 below.

In Comparative Example 18, N-Hance CCG45 manufactured by Ashland, a guar hydroxypropyltrimonium chloride, was used. In Comparative Example 19, OaSense Care CT400 manufactured by Ashland, a hydroxypropyl guar hydroxypropyltrimonium chloride, was used.

In Comparative Examples 20 and 21, a synthetic polymer of ethyltrimonium chloride methacrylate/propyltrimonium acrylamide/dimethylacrylamide copolymer of KR Patent No. 10-1291693 was used. Table 9 below shows samples for evaluating the cumulative adsorption through the polymer bases.

**[Table 9]**

| Samples | Comparative Example 18 | Comparative Example 19 | Comparative Example 20 | Comparative Example 21 |
|---|---|---|---|---|
| Polymer | Guar | Guar | Synthetic | Synthetic |
| Molecular Weight | 800,000 | 800,000 | 800,000 | 800,000 |
| N% | 1.4 | 2.7 | 1.4 | 2.7 |

Table 10 below shows the results of cumulative adsorption evaluation for the samples of Table 9 (unit: µm).

**[Table 10]**

| Number of adsorptions | Comparative Example 18 | Comparative Example 19 | Comparative Example 20 | Comparative Example 21 |
|---|---|---|---|---|
| 0 | 0 | 0 | 0 | 0 |
| 1 | 0.935 | 1.027 | 0.673 | 0.783 |
| 2 | 1.025 | 1.453 | 0.693 | 0.892 |
| 3 | 1.284 | 1.364 | 0.783 | 0.904 |
| 4 | 1.257 | 1.502 | 0.893 | 0.978 |
| 5 | 1.563 | 1.789 | 0.834 | 1.025 |
| 6 | 1.375 | 1.853 | 0.904 | 1.064 |
| 7 | 1.694 | 1.762 | 1.024 | 1.194 |
| 8 | 1.792 | 1.981 | 1.083 | 1.285 |
| 9 | 1.802 | 1.972 | 1.184 | 1.304 |
| 10 | 1.894 | 2.247 | 1.283 | 1.503 |
| 11 | 2.005 | 2.283 | 1.306 | 1.603 |

As shown in Table 10, no phenomenon of cumulative adsorption prevention was observed in the polymers other than cellulose. This is because the chemical structure of the cellulose has a linear ethyl cellulose structure, while the guar polymer has a branched structure, and accordingly, in the guar polymers, the desorption effect, which occurs during rinsing, was remarkedly reduced due to the hydrophilicity after adsorption, thus failing to prevent the cumulative adsorption phenomenon. In the case of the synthetic polymers, it is interpreted that the repulsive force during adsorption was different from that of cellulose due to the difference in the bonding position of the cationic nitrogen.

### (6) Sensory Evaluation of Rinsing and Conditioning According to Polymer

The shampoo compositions were prepared in a conventional manner according to the prescription shown in Table 11 below.

**[Table 11]**

| Mixing Ingredients | Weight Ratio (%) |
|---|---|
| Polymer | 0.5 |
| Sodium laureth sulfate | 8 |
| Cocamidopropyl betaine | 4.5 |
| EDTA 4Na, citric acid monohydrate | 0.1 |
| Other ingredients | 16 |
| Purified water | Residual amount (to 100) |

Since cellulose-based polymers have excellent solubility, after adding the polymers, surfactants were added in sequence and dissolved and then the pH was neutralized by adding EDTA-4Na and citric acid monohydrate. As other ingredients, preservatives, fragrances, dispersants, viscosity modifiers, and pH modifiers were added.

1 g of hair tress was added to 100 mL of a 10% diluted shampoo solution and stirred for 5 minutes at 150 rpm and then rinsed for 2 minutes with a flow rate of 4 ml/sec, and this whole process was repeated 5 times. Sensory evaluation was performed to evaluate the stiffness of the treated hair, and the results are shown in Table 12 below. The experiment was conducted on 15 male and 15 female subjects, and they were required to evaluate the conditioning effect of the hair after shampooing based on the evaluation criteria of freshness. The results are shown in Table 12 below as average values.

Experiment #9 was the result of treatment with sodium lauryl sulfate, an anionic surfactant, without the treatment of any polymer, and Experiment #10 was treated with the hair in which the cumulative adsorption phenomenon occurred after shampooing using the cationic cellulose polymer of Comparative Example 10. Additionally, in Experiment #11, the polymer according to the present invention (Example 2) was applied as a conditioning polymer.

Evaluation Criteria-The order of hair freshness was measured, and the opposite represents roughness.

### (5: Very good); (4: Good); (3: Moderate); (2: Bad); (1: Very bad)

**[Table 12]**

| Experimental Category | **Experiment** #9 | Experiment #10 | Experiment #11 |
|---|---|---|---|
| When wet | 1.3 | 1.9 | 3.7 |
| After drying | 2.1 | 1.6 | 4.2 |

As shown in Experiment #11 of Table 12, it can be seen that the polymer in which the cumulative adsorption was prevented provided freshness and was escaping from providing the sense of stiffness.

### (7) Evaluation of Frictional Force

The conditioning effect of the cationic polymer was applied to shampoo, and the frictional force was quantified by device evaluation.

The shampoo composition of Table 11 was added to the burex hair tress in an amount of 10% by weight, and the hair was lathered for 15 seconds, rubbed for 20 seconds, rinsed for 15 seconds with running water at 37°C, wiped with a towel to remove moisture, and then evaluated for the frictional force using an MTT 175 Miniature Tensile Tester (Diastron, GB).

The other hair treated with the shampoo was rinsed for 2 minutes and then dried with a dryer for 2 minutes, and was maintained at a temperature of 25°C in a constant temperature and humidity chamber under a condition of 50% humidity for one day, so that the moisture in the hair was kept constant. Thereafter, the frictional force was measured in the same temperature and humidity chamber and the results are shown in Table 13 below.

Herein, the hair tress was washed with sodium lauryl sulfate, an anionic surfactant, which was used as a reference value, and the value of the frictional force of the hair was calculated by comparing the difference from the corresponding measured value based thereon.

**[Table 13]**

| Experimental Category | Experiment #12 (Polymer of Comparative Example) | Experiment #13 (Polymer of Example 2) |
|---|---|---|
| During rinsing | 21 | 55 |
| After drying | 13 | 21 |

From the results of Table 13, the shampoo, to which the polymer presented in the present invention was applied, showed reduced rinsing time during rinsing, and the frictional force was remarkably reduced even after drying, thereby improving the smooth conditioning effect. In addition, as can be seen from the above Examples, the polymer prepared in the present invention imparts the effect of preventing cumulative adsorption when applied as a conditioning agent, thereby overcoming the problem of the cumulative adsorption that gives a feeling of stiffness, and accordingly, it was confirmed that it provides freshness to the hair and is an excellent component for hair conditioning cosmetic compositions.

Subsequently, in order to confirm whether the phenomenon of cumulative adsorption prevention due to repeated use was applied to the hair, and at the same time, whether the stiffness caused by the accumulation prevention phenomenon was improved, the shampoo composition was treated 60 times to evaluate the frictional force as shown in Table 14.

**[Table 14]**

| Experimental Category | Experiment #14 (Polymer of Comparative Example) | Experiment #15 (Polymer of Example 2) |
|---|---|---|
| During rinsing | 20 | 56 |
| After drying | 4 | 21 |

The decrease of the change in the frictional force on the hair surface in Experiment #14, which occurs when the shampoo according to the prescription of Table 11 was repeatedly applied for washing of the hair, can be understood as a decrease in the degree of smoothness due to the texture of the polymer accumulated in the hair when the shampoo is repeatedly used. In Experiment #15, the effect of no change in the frictional force even after repeated washing and adsorption reflects that there is no change in the polymer layer.

### 3. Property of Cumulatively Adsorbing Active Ingredients of Cationic Polymer, to which the Active Ingredients are Bound, onto Hair, Skin, or Fiber

### (1) Preparation of Cationic Polymer to which Hair Dye is Bound

Cationic cellulose polymers were prepared according to the above-mentioned 1.

One of the reactive blue No. 4 dye and 5-([4,6-dichlorotriazin-2-yl]amino) fluorescein was selected and allowed to bind to the carbon atom at position 2 of the glucose monomer as shown in Reaction Schemes 1 and 2 below. The dyes can be used as a phosphor and a hair dye, and thus can grasp the polymer adsorption position of the active ingredient and utilize the dye function at the same time. In the reactive blue dye No. 4, both the fluorescence emission color and the visible light reflection color are blue, whereas in 5-([4,6-dichlorotriazin-2-yl] amino) fluorescein, the fluorescence emission color is green but it takes on a bright reddish orange color under the visible light.

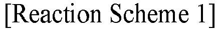

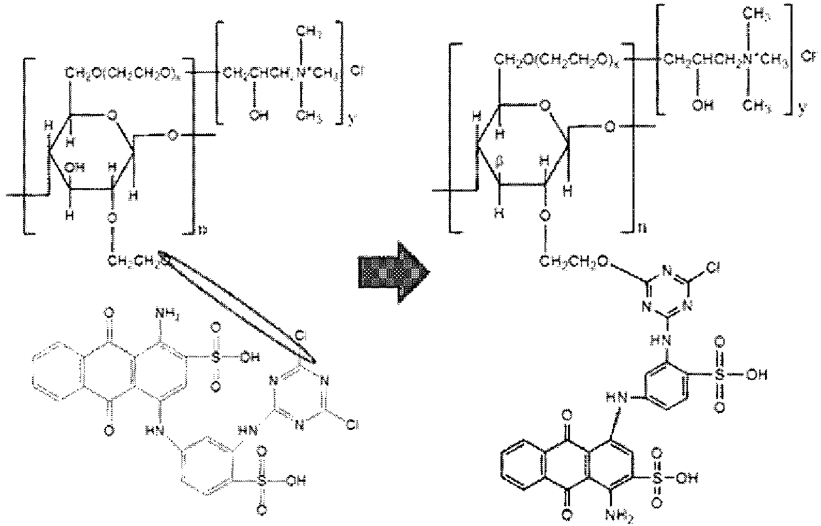

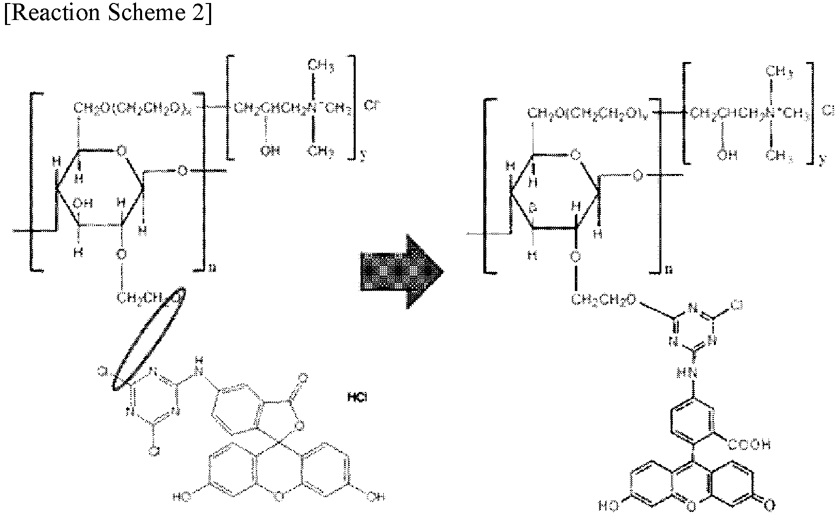

As a preparation method, the pH was lowered using KOH, and the dye was stirred with the polymer at 500 rpm, filtered with HPLC-grade ethanol, and then subjected to freeze-drying (Bondiro, Ilshin) for one week. However, as shown in Reaction Scheme 2, a binding between the amine group of the polymer and the carboxyl group of 5-([4,6-dichlorotriazin-2-yl] amino) fluorescein may occur, leading to aggregation. In this case, the aggregate can be decomposed by stirring with a homo mixer, if necessary.

### (2) Change in Thickness of Adsorption Layer According to Viscosity and Molecular Weight

In order to evaluate the adsorption state of the cationic polymer bound with reactive blue No. 4, mica, which has a Zeta potential of tens of mV, similar to that of hair and wool, was selected as the substrate, and the cationic polymers were adsorbed onto the mica substrate to determine the adsorption characteristics of the polymers, using the method practiced by many companies including Dow Corning, a raw-material manufacturing company for conditioning agents.

12 mm diameter mica discs (TED PELLA, Canada) were used as an adsorbent. The polymers were placed and adsorbed onto only half of the mica discs using 10 ml of distilled water containing 0.5% of each polymer, and then washed with distilled water. The amount of polymer adsorption is determined by the electrical attraction and repulsion between the polymer, the mica, and the polymer adsorbed onto the mica. Since the desorption proceeds due to the hydrophilicity between the polymer and water, the change in the polymer content is not a factor affecting the adsorption amount. Therefore, the polymer content of 0.5% commonly used in the shampoo was selected as an experimental condition.

The interface between the adsorbed polymer layer and the mica bottom was observed by the atomic force microscopy (AFM, Model Systems XE-100, Korea) using the topography of the contact mode of the tip, and the result of evaluating the thickness is shown in Fig. 1.

Examples 8 to 10 and Comparative Examples 22 to 24 having the viscosity of Table 15 were prepared for thickness variation experiments according to viscosity and molecular weight of the polymers.

**[Table 15] (Examples 8 and 9 not according to the invention)**

| Samples | Exampl e 8 | Exampl e 9 | Exampl e 10 | Comparative Example 22 | Comparative Example 23 | Comparative Example 24 |
|---|---|---|---|---|---|---|
| Viscosity (2%) | 50 | 200 | 500 | 600 | 700 | 800 |
| Molecular Weight | 100,000 | 200,00 0 | 300,00 0 | 500,000 | 700,000 | 900,000 |

Table 16 shows the change in the thickness of adsorption layer according to the molecular weight of the polymers.

**[Table 16]**

| Number of adsorptions | Exampl e 8 | Exampl e 9 | Exampl e 10 | Comparative Example 22 | Comparative Example 23 | Comparative Example 24 |
|---|---|---|---|---|---|---|
| 0 | 0 | 0 | 0 | 0 | 0 | 0 |
| 1 | 0.32 | 0.18 | 0.19 | 0.52 | 0.68 | 0.69 |
| 3 | 0.72 | 0.51 | 0.36 | 0.37 | 0.40 | 0.42 |
| 5 | 0.82 | 0.76 | 0.46 | 0.35 | 0.37 | 0.39 |
| 7 | 0.92 | 0.82 | 0.52 | 0.38 | 0.38 | 0.40 |
| 9 | 1.01 | 0.87 | 0.58 | 0.37 | 0.42 | 0.41 |
| 11 | 1.10 | 0.93 | 0.64 | 0.38 | 0.37 | 0.40 |
| 13 | 1.14 | 1.02 | 0.74 | 0.37 | 0.38 | 0.41 |
| 15 | 1.21 | 1.13 | 0.83 | 0.40 | 0.39 | 0.39 |
| 17 | 1.26 | 1.22 | 0.92 | 0.39 | 0.41 | 0.40 |

| | | | | | | |
|---|---|---|---|---|---|---|
| (unit: µm) | | | | | | |

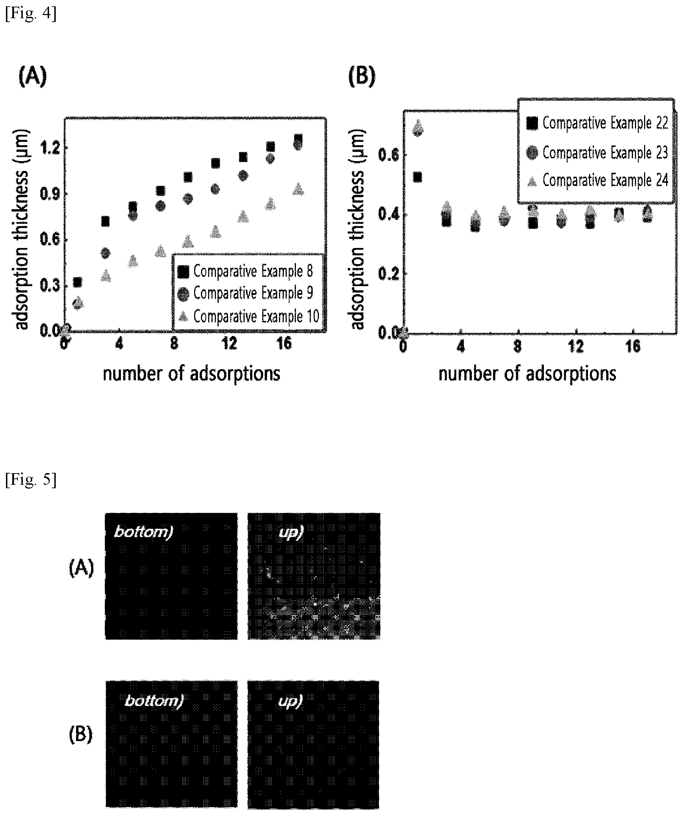

From the results shown in Table 16 and Fig. 4, in Examples 8 to 10 relating to the polymers having a viscosity of 50 to 500 in an aqueous solution state and a molecular weight of 100,000 to 300,000, it can be seen that the thickness of the polymers being adsorbed increased with additional adsorption.

In Comparative Examples 22 to 24, as the number of adsorptions increased, the adsorption thickness of the adsorption layer remained constant, and accordingly, it appears that the additional adsorption was not proceeded by the cationic repulsive force between the adsorption layer and the cation polymer, and the rinsing power with respect to the binding relationship with water.

### (3) Analysis of Adsorption Layer Components Using Confocal Fluorescence Microscopy

After finding out that the polymer adsorption patterns can be divided into three types as seen in the results of Table 16 above, the confocal fluorescence microscopy was used to confirm as to why such variation phenomenon occurred.

A cellulose polymer bound with a fluorescent dye was examined using a confocal fluorescence microscope (Carl Zeiss, LSM710). With respect to the polymers of Example 9 and Comparative Example 23, the polymer bound with the reactive blue No. 4 dye was first adsorbed onto mica discs and washed three times, and subsequently, the polymer bound with the green fluorescent dye (5-([4,6-dichlorotriazin-2-yl] amino) fluorescein) was continuously adsorbed onto the mica discs and washed. A light laser with an excitation light wavelength of 420 nm and 510 nm was irradiated thereto, and the fluorescence spectrum was mapped onto the bottom with an area of 100 µm × 100 µm. Then, the first top part and the bottom part of the fluorescence spectrum were focused and mapped as shown in Fig 5.

As shown in Fig. 5 (A), the polymer of Example 9 had a blue bottom, but no green color was detected. However, the blue fluorescent polymer was not detected at the top part, but only the green part was detected. That is, considering that the green-bound polymer was cumulatively adsorbed onto the blue-bound polymer, it is suggested that the cumulative adsorption was continuously carried out on the conventional adsorption layer whenever the polymer was adsorbed.

The polymer of Comparative Example 23 is shown in Fig. 5 (B). Only the blue fluorescent polymer was detected at both the top and bottom parts, and the green fluorescent polymer was not detected. This implies that the there was no further adsorption after the adsorption of the blue polymer was proceeded.

### (4) Application of Accumulation Phenomenon to Hair

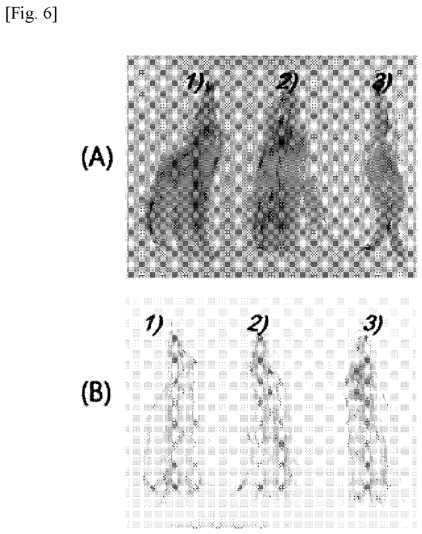

It was confirmed whether the variation phenomenon occurs after polymer adsorption by simultaneously applying an anionic surfactant-based shampoo to the hair, which is an actual biological substrate as shown in Fig. 6.

The shampoo composition was prepared in a conventional manner according to the prescription shown in Table 17 below.

**[Table 17]**

| Mixing Components | Weight ratio (%) |
|---|---|
| Jojoba oil | 0.1 |
| Polymer | 0.5 |
| Sodium laureth sulfate | 8 |
| Cocamidopropyl betaine | 4.5 |
| EDTA 4Na, citric acid monohydrate | 0.1 |
| Other ingredients | 16 |
| Purified water | Residual amount (to 100) |

Since cellulose-based polymers have excellent solubility, after adding the polymers, surfactants were added in sequence and dissolved and then the pH was neutralized by adding EDTA-4Na and citric acid monohydrate. As other ingredients, preservatives, fragrances, dispersants, viscosity modifiers, and pH modifiers were added.

1 g of hair tress was treated with the shampoo for 50 seconds and rinsed for 2 minutes with a flow rate of 4 ml/sec. Fig. 3 1), 2), and 3) are the results of washing after a single treatment, two-repeated treatments, and three-repeated treatments, respectively.

In the case of the polymer of Example 9 in which adsorption proceeded according to the number of adsorptions, the hair color was strengthened depending on the dye color as the number of treatments increased as shown in Fig. 6 (A). In contrast, in the case of the polymer of Comparative Example 23 in which further adsorption was prevented, the polymer constantly appeared in blue, which was the first dye color, regardless of additional dyeing as shown in Fig. 6 (B).

Through the above Examples, it can be seen that the dyeing was actually strengthened when the polymer was applied to hair.

Table 18 shows the results of treating hair with the shampoo prepared using the polymer having a viscosity of 200 cps used in Example 9 to which the reactive blue No. 4 dye was bound, and the polymer having a viscosity of 700 cps used in Comparative Example 23, to which the reactive blue No. 4 dye was bound, as shown in Table 17, and the results of color change are expressed as a CIE lab value through a color difference meter.

**[Table 18]**

| Samples | Example 9 | | | Comparative Example 23 | | |
|---|---|---|---|---|---|---|
| | Blue | Green | Yellow | Blue | Green | Yellow |
| 1 | 1.25 | 1.13 | 0.64 | 1.35 | 1.21 | 0.53 |
| 3 | 1.53 | 1.25 | 0.73 | 1.28 | 1.02 | 0.54 |
| 5 | 1.59 | 1.29 | 0.79 | 1.15 | 1.12 | 0.56 |
| 7 | 1.63 | 1.35 | 0.84 | 1.29 | 1.07 | 0.49 |
| 9 | 1.75 | 1.42 | 0.86 | 1.32 | 1.10 | 0.53 |

| | | | | | | |
|---|---|---|---|---|---|---|
| Color difference value, ΔE=(L²+a²+b²)^{1/2} | | | | | | |

As can be seen from Table 18, it can be seen that Example 9 had a color-strengthening effect according to repeated use.

### (5) Sensory Evaluation

Meanwhile, a sensory evaluation was performed to evaluate the smoothness of the treated hair. The results are shown in Table 19 below. The experiment was conducted on 15 male and 15 female subjects, and they were required to evaluate the conditioning effect of the hair based on the evaluation criteria of smoothness. Experiment #16 is the results of treating the hair with the anionic surfactant sodium lauryl sulfate without treatment of a polymer. Experiment #17 is the results of sensory evaluation of hair which was washed once with the shampoo prepared using the polymer of Example 9, and Experiment #18 is the results thereof using the polymer of Comparative Example 23.

Evaluation Criteria-The order of hair freshness was measured, and the opposite represents roughness.

(5: Very good); (4: Good); (3: Moderate); (2: Bad); (1: Very bad)

**[Table 19]**

| Experimental Category | Experiment #16 | Experiment #17 | Experiment #18 |
|---|---|---|---|
| Sensory Evaluation | 1.5 | 4.5 | 4.5 |

As shown in Table 19, it can be seen that excellent effect was actually exhibited even in terms of the conditioning effect. This is because the molecular weight of the polymer was much larger than the molecular weight of the hair dye components bound to the glucose ring, and thus the hair dye component did not interfere with the surface smoothness after adsorption to hair, which can be confirmed from the fact that there was no difference between Experiments #17 and #18.

### 4. Properties of Transferring Active Ingredient of Cationic Polymer to which the Active Ingredient are Bound to Hair, Skin, or Fiber

### (1) Preparation of Cationic Polymers Bound with Hair Dyes

Cationic cellulose polymers were prepared according to the above-mentioned 1. One of the reactive blue No. 4 dye and 5-([4,6-dichlorotriazin-2-yl]amino) fluorescein was selected and allowed to bind to the carbon at position 2 of the glucose monomer as shown in Reaction Schemes 1 and 2 below. The dyes can be used as a phosphor and a hair dye, and thus can grasp the polymer adsorption position of the active ingredient and utilize the dye function at the same time. The cellulose polymer bound with the reactive blue No. 4 is represented by Chemical Formula 3, and the cellulose polymer bound with 5-([4,6-dichlorotriazin-2-yl]amino) fluorescein is represented by Chemical Formula 4. The polymer of Chemical Formula 3 has a fluorescence emission color and a visible light reflection color of blue, and the polymer of Formula 4 has a fluorescence emission color of green while having a visible light reflection color of orange.

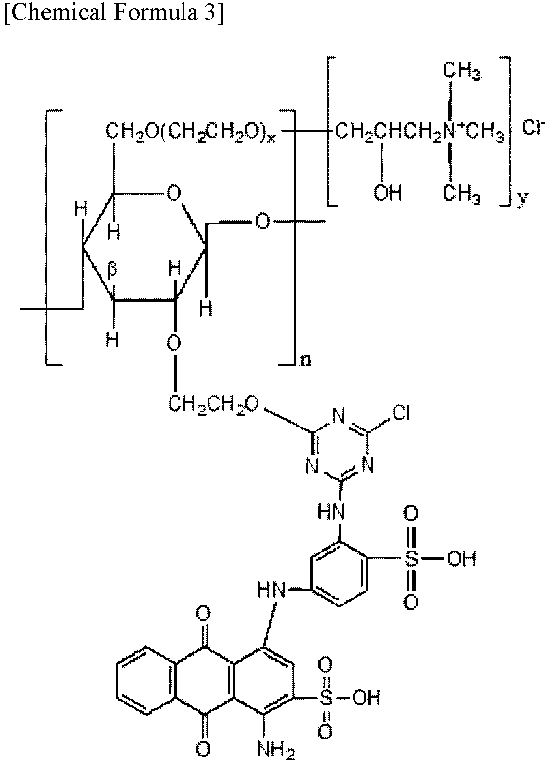

In Chemical Formula 3, x is an integer of 1 to 10, and when y is 1, n is 0.3 to 0.7.

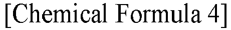

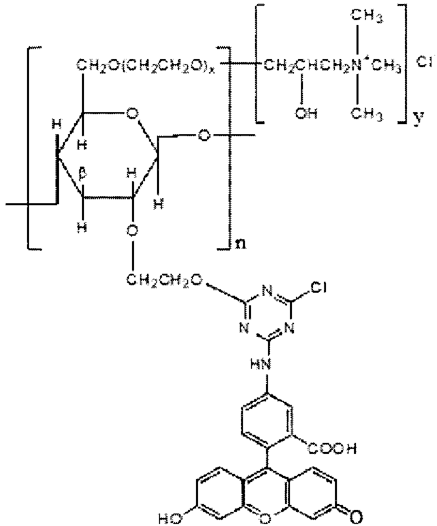

In Chemical Formula 4, x is an integer of 1 to 10, and when y is 1, n is 0.3 to 0.7.

The cellulose polymer prepared as described above was adjusted of its pH using KOH, and then stirred at 500 rpm, filtered with HPLC-grade ethanol, and subjected to freeze-drying (Bondiro, IlshinBio) for one week. When aggregation occurs due to the binding between the amine group and the carboxyl group of 5-([4,6-dichlorotriazin-2-yl]amino) fluorescein in the cellulose polymer represented by Chemical Formula 4, the aggregate can be decomposed by stirring with a homo mixer, if necessary.

### (2) Change in Adsorption Layer Thickness according to Molecular Weight

In order to evaluate the adsorption characteristics of cationic polymers to the substrate, the method practiced in the art including Dow Corning, a raw-material manufacturing company for conditioning agents, was used. More specifically, mica, which has a Zeta potential of tens of mV, similar to those of hair and wool, was selected as the substrate, and the cationic polymers were adsorbed onto the mica substrate to determine the adsorption characteristics of the polymers.

12 mm diameter mica discs (TED PELLA, Canada) were used as an adsorbent. The polymers were placed and adsorbed onto only half of the mica discs using 10 ml of distilled water containing 0.5% of polymers, and then washed with distilled water. The adsorption of cationic cellulose polymers is determined by the electrostatic action between the mica and the polymer adsorbed onto the mica. Since the content of the polymer in an aqueous solution does not affect the amount of adsorption, 0.5% aqueous solution, which is a mixing amount conventionally used in the compositions for hair, was selected and used.

The interface between the adsorbed polymer layer and the mica bottom was observed by the atomic force microscopy (AFM, Model Systems XE-100, Korea) using the topography of the contact mode of the tip, and the result is shown in Fig. 1.

The molecular weight of the polymers used to evaluate the adsorption layer characteristics in the 0.5% aqueous solution is shown in Table 20 below.

**[Table 20] (Example 13 not according to the invention)**

| Samples | Comparati ve Example 25 | Comparati ve Example 26 | Comparati ve Example 27 | Example 11 | Example 12 | Example 13 |
|---|---|---|---|---|---|---|
| Molecular Weight | 600,000 | 1,000,000 | 1,200,000 | 1,500,000 | 2,100,000 | 3,000,000 |

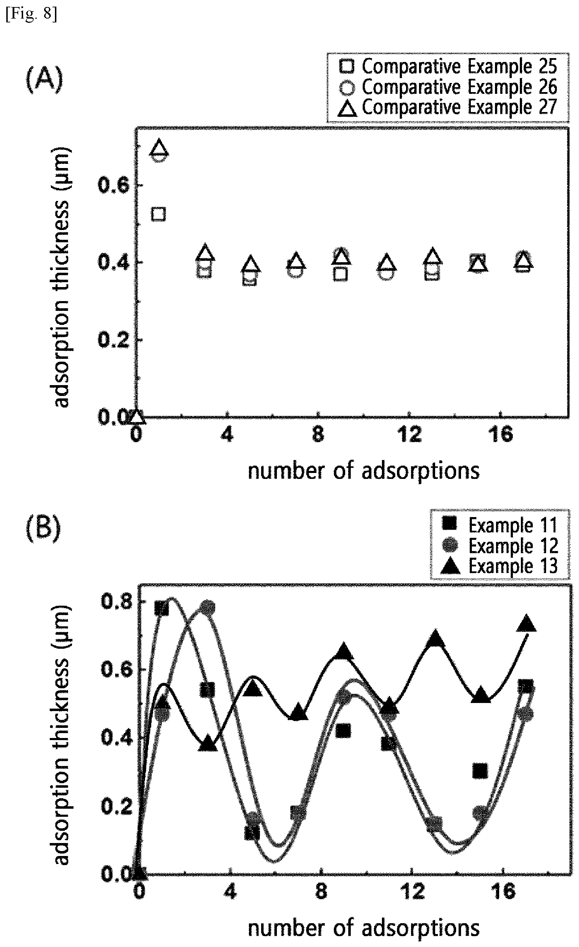

The change in the thickness of the adsorption layer according to the molecular weight of polymers was measured according to the number of adsorptions and is shown in Table 21 and Fig. 8. (Unit: µm).

**[Table 21]**

| Number of adsorptions | Comparati ve Example 25 | Comparati ve Example 26 | Comparati ve Example 27 | Example 11 | Example 12 | Example 13 |
|---|---|---|---|---|---|---|
| 0 | 0 | 0 | 0 | 0 | 0 | 0 |
| 1 | 0.52 | 0.68 | 0.69 | 0.78 | 0.47 | 0.50 |
| 3 | 0.37 | 0.40 | 0.42 | 0.54 | 0.78 | 0.38 |
| 5 | 0.35 | 0.37 | 0.39 | 0.12 | 0.16 | 0.54 |
| 7 | 0.38 | 0.38 | 0.40 | 0.18 | 0.18 | 0.47 |
| 9 | 0.37 | 0.42 | 0.41 | 0.42 | 0.52 | 0.65 |
| 11 | 0.38 | 0.37 | 0.40 | 0.38 | 0.47 | 0.49 |
| 13 | 0.37 | 0.38 | 0.41 | 0.14 | 0.15 | 0.69 |
| 15 | 0.40 | 0.39 | 0.39 | 0.30 | 0.18 | 0.52 |
| 17 | 0.39 | 0.41 | 0.40 | 0.55 | 0.47 | 0.73 |

As can be seen from the results of Table 21 and Fig. 8, it was confirmed that Comparative Examples 25 to 27 showed a constant adsorption thickness despite repeated number of adsorptions, whereas Examples 11 to 13 showed that the thickness of the adsorption layer fluctuated within a particular range. These results confirmed that the cellulose polymers according to the present invention having a molecular weight within a particular range exhibited a phenomenon of adsorption layer fluctuation.

More specifically, Comparative Examples 25 to 27 showed the highest adsorption thickness for one-time adsorption, and it can be understood therefrom that the cationic cellulose polymers were constantly adsorbed onto the space on the negatively charged surface. However, starting from the third-time adsorption, the thickness of the adsorption layer started to decrease, and it can be understood therefrom that the adsorption force was lowered due to the cationic repulsive force with the adsorption layer already formed, and thus the additional adsorption layer was washed down during the rinsing process, in the case in which the anionic surface was fully covered with the cationic cellulose polymer and then the cationic cellulose polymer was further adsorbed onto a single layer having an overall negative charge

### (3) Analysis of Adsorption Layer Components using Confocal Fluorescence Microscopy

In order to confirm the phenomenon of the adsorption layer fluctuation estimated by the fluctuation of the adsorption layer thickness in (2) above, the following experiment was performed.

First, an experiment was performed on the cellulose polymers to which a fluorescent dye was bound using a confocal fluorescence microscope (LSM710, Carl Zeiss). The cellulose polymers of Comparative Example 25 and Example 11 were used. First, the polymer bound with a blue fluorescent dye was adsorbed onto mica discs and washed three times, and then the polymer bound with a green fluorescent dye was continuously adsorbed onto mica discs and washed three times.

Then, the thus-treated mica discs were irradiated with a laser light having a wavelength of 420 nm and 510 nm, and the fluorescence spectrum was mapped onto the bottom with an area of 100 µm X 100 µm. Thereafter, the most top and most bottom parts of the treated mica discs were focused and mapped, and the results are shown in Fig. 3.

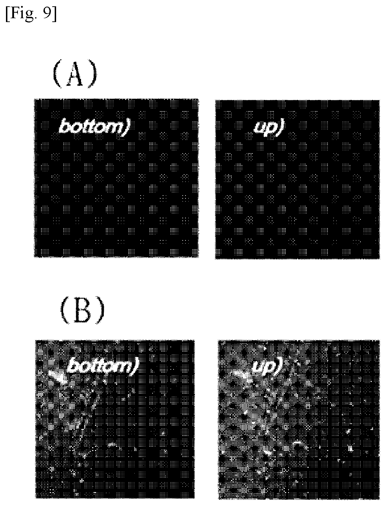

As can be confirmed from Fig. 9, in the case of the polymer (A) of Comparative Example 25, only the blue fluorescent polymer was detected at both the bottom and top parts of the mica discs, and no green fluorescence was detected. Through these results, it was confirmed that after the polymer of Comparative Example 25 was adsorbed onto the mica discs, the cellulose polymer bound with the green fluorescent dye was not further adsorbed onto the mica discs.

In contrast, in the case of the polymer (B) of Example 11, both blue fluorescence and green fluorescence were detected at both the bottom and top parts of the mica discs. Accordingly, it was confirmed that the polymer bound with the blue fluorescence and the polymer bound with the green fluorescence formed an adsorption layer due to the fluctuation of the adsorption layer of the polymer of Example 11. From these results, it can be seen that the previously adsorbed cationic polymer was substituted with the cationic cellulose of the present invention having a molecular weight of 1,500,000 or more thus being capable of transferring the active ingredients.

### (4) Confirmation of Phenomenon of Adsorption Layer Fluctuation Through Hair Dyeing

In order to confirm the effect of the cellulose polymer according to the present invention when it was applied to hair, which is an actual biological substrate, the following experiment was conducted.

The polymers used in the experiment were polymers having the molecular weights of Comparative Example 25 and Example and 11. The result obtained from the use of Comparative Example 15 was denoted by (A) and the result obtained from the use of Example 11 was denoted by (B).

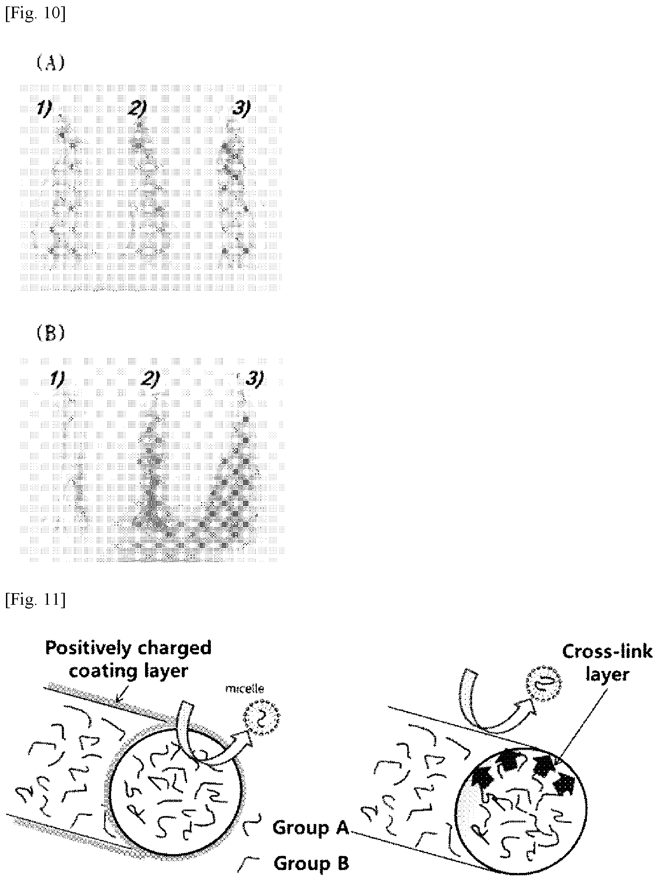

First, a 0.5% aqueous solution of the cellulose polymer bound with a blue dye was applied in an amount of 1 ml per 1 g of yak hair, and after 30 seconds, the yak hair was washed with running water for 30 seconds and the water thereon was removed using a dryer. The hair at this time was denoted as 1) in each of A and B in Fig. 10.

Then, the cellulose polymer bound with an orange dye was additionally applied under the same conditions as in Example 11 of (2) above , and after 30 seconds, the hair was washed with running water for 30 seconds and the water thereon was removed using a dryer. The hair at this time was denoted as 2) in each of A and B in Fig. 10.

Finally, the cellulose polymer bound with the blue dye was applied, and after 30 seconds, the hair was washed with running water for 30 seconds and the water thereon was removed using a dryer. The hair at this time was denoted as 3) in each of A and B of Fig. 10.

As can be seen from Fig. 10, it was confirmed that, in the case of Comparative Example 25, no additional adsorption layer was formed when an additional polymer was applied according to the adsorption of 1) to 3). In contrast, in the case of Example 11, a violet dye, which is an interference color between a blue dye and an orange dye, was observed, and accordingly, it was confirmed that the color of hair dye was changed through the adsorption layer fluctuation of the polymer. This result reflects that the orange dye layer adsorbed for the second time and the blue dye layer adsorbed for the third time were mixed rather than being accumulated in order.

Through this experiment, it was confirmed that the active ingredients can be continuously transferred through the fluctuation of the cellulose polymer adsorption layer in the actual biological materials.

### (5) Evaluation of Continuous Conditioning Effect

The conditioning effect was evaluated by applying cellulose polymers, to which hair oil was bound, to the hair.

The cellulose polymers of Comparative Example 25 and Example 11 were used in the experiment, and C₁₅ to C₂₀ alkyl groups were added using a halohydrin at the α position in each glucose monomer to prepare cellulose polymers bound with oil.

The prepared polymers were used to prepare a shampoo composition with the composition shown in Table 22 below. The preparation of the shampoo composition was carried out according to a conventional method. After the cellulose polymers were dissolved in purified water, a surfactant was added in sequence and dissolved, and then the pH was adjusted by adding EDTA 4Na and citric acid monohydrate. As other ingredients, preservatives, fragrances, dispersants, viscosity modifiers, and pH modifiers were added.

**[Table 22]**

| Ingredients (wt%) | Shampoo Preparation Example 1 | Shampoo Preparation Example 2 |
|---|---|---|
| Jojoba oil | 0.1 | 0.1 |
| Polymer (Example of using the polymer of Comparative Example 25) | 0.5 | |
| Polymer (Example of using the polymer of Example 11) | | 0.5 |
| Sodium laureth sulfate | 8 | 8 |
| Cocamidopropyl betaine | 4.5 | 4.5 |
| EDTA 4Na, citric acid monohydrate | 0.1 | 0.1 |
| Other ingredients | 16 | 16 |
| Purified water | Residual amount (to 100) | Residual amount (to 100) |

1 g hair tress was treated with shampoo for 50 seconds using each of the above Shampoo Preparation Example 1 and Shampoo Preparation Example 2 prepared with the above composition and then rinsed with running water at a flow rate of 4 ml/sec for 2 minutes, and this whole process was repeated 60 times. After heating the hair for 60 minutes at 65°C using an evaporator (Mettler Toledo, US), the change in hair mass of 0.5 g was measured to evaluate the change in moisture content of the hair. The results are shown in Table 23 below.

**[Table 23]**

| | Shampoo Preparation Example 1 | Shampoo Preparation Example 2 |
|---|---|---|
| Mass Change % | 19.2 | 23.2 |

As confirmed in Table 23, the change in hair mass was large when the Shampoo Preparation Example 2 was used. This indicates that the amount of moisture contained in the hair was relatively increased. That is, it was confirmed that the oil transferred by the cellulose polymer was continuously transferred to the hair surface cuticle due to the fluctuation phenomenon inside the adsorption layer, and the moisture release from the hair was prevented, thereby providing a moisturizing effect to the hair.

Additionally, sensory evaluation was conducted on 15 men and 15 women with respect to the hair treated with the Shampoo Preparation Examples 1 and 2 and the control as described above for softness. As the control, hair, which was treated with a 10% aqueous solution of sodium lauryl sulfate as an anionic surfactant in the same manner as above, was used without treatment of any polymer.

### [Evaluation Criteria]

5- Very good (soft), 4- Good, 3- Moderate, 2- Bad, 1- Very bad (rough)

**[Table 24]**

| | Use of Shampoo Preparation Example 1 | Use of Shampoo Preparation Example 2 | Use of Control |
|---|---|---|---|
| Sensory Evaluation | 3.2 | 4.6 | 1.7 |

As can be seen in Table 24, the conditioning effect was observed when the Shampoo Preparation Examples 1 and 2 were used, and it was confirmed that the effect was remarkably excellent in the case of using the Shampoo Preparation Example 2 containing the polymer that exhibits the adsorption layer fluctuation.

### 5. Property of Preventing Loss of Hair, Skin, or Fiber Components of Cationic Polymers

### (1) Preparation of Cationic Polymers

Cationic cellulose polymers were prepared according to the above-mentioned 1. Specifically, a cationic cellulose polymer having a molecular weight of 800,000 and a nitrogen content (% by weight) of 2.7%, wherein α is EO and β is OH in the Chemical Formula 1; a cationic cellulose polymer having a molecular weight of 800,000 and a nitrogen content (% by weight) of 2.7%, wherein α is EO (divalent or higher) and β is OH; a cationic cellulose polymer having a molecular weight of 800,000 and a nitrogen content (% by weight) of 2.7%, wherein α is EO (divalent or higher) and β is EO (monovalent or higher); a cationic cellulose polymer having a molecular weight of 1,800,000 and a nitrogen content (% by weight) of 2.7%, wherein α is EO (divalent or higher) and β is OH; and a cationic cellulose polymer having a molecular weight of 2,500,000 and a nitrogen content (% by weight) of 2.7%, wherein α is EO (divalent or higher) and β is OH were synthesized and used in the following tests. In addition, under the same conditions as above, a cationic cellulose polymer having a nitrogen content of 1.7% by weight was also synthesized and used in subsequent experiments.

### (2) Preparation of Washing Solution Composition

The shampoo washing solution composition was prepared in a conventional manner according to the prescription shown in Table 25 below. The composition was prepared by varying the amount of the polymer and/or oil included in the washing solution according to the following Examples or Comparative Examples. Specifically, after adding the polymer, surfactants were added in sequence and dissolved, and the pH was neutralized by adding EDTA-4Na and citric acid monohydrate. As other ingredients, preservatives, fragrances, dispersants, viscosity modifiers, and pH modifiers were added.

**[Table 25]**

| **Mixing Ingredients** | **Weight (%)** |
|---|---|
| Polymer and/or oil | 1.0 |
| Sodium lauryl sulfate (SLS) | 8 |
| Cocamidopropyl betaine | 4.5 |
| EDTA 4Na, citric acid monohydrate | 0.1 |
| Other ingredients | 16 |
| Purified water | Residual amount (to 100) |

Next, a conditioner was prepared as shown in Table 26.

**[Table 26]**

| **Mixing Ingredients** | **Weight (%)** |
|---|---|
| Purified water | 75 |
| Glyceryl stearate | 0.1 |
| Cetearyl alcohol | 5 |
| Behentrimonium chloride | 2 |
| Purified water | Residual amount (to 100) |

### (3) Lipid Quantification through GC/MS Spectrum

### 1) Evaluation Method

For the shampoo treatment, 1 g of hair tress was treated with 1 ml of shampoo, lathered for 45 seconds by rubbing, and rinsed with water for 2 minutes at a flow rate of 4 ml/sec, and this whole process was repeated 5 times. Next, the hair was allowed to stand at 50% RH condition in a constant temperature and humidity chamber for one day and then finely crushed with intervals of 1 mm. Lipids were extracted with a solvent mixture of chloroform and methanol using an ultrasonic washing machine (Branson, 3510E) for 4 days and analyzed by gas chromatography GC/MS (Agilent 5977B GC/MSD) in a SIM mode.

For GC/MS analysis, marker materials were prepared by mixing with a solution prepared by mixing chloroform and methanol in a ratio of 2:1 and at 5000 ppm. As the internal solution, tricosanoic acid was used at 50 ppm. Ionization voltage was 70 eV at 250°C, and HP-5ms UI column (30 m × 0.25 mm × 0.25 µm) was used. The temperature was raised from 80°C to 320°C by 5°C per minute. In all experiments, the population was n = 10.

### 2) Evaluation According to Polymer Type

### Experiments #9 to #22

The hair was treated 10 times with the shampoo composition of Table 25, which includes LR30M as a polymer, and the quantitative value obtained by GC/MS analysis was calculated as a relative value based on the GC/MS quantitative value of un-shampooed hair, which was set to 100%, and the analysis results are shown in Table 27. The group A includes myristyl acid (C₁₄), palmitic acid (C₁₆), stearic acid (Cis), oleic acid (C₁₈₌₁), and cholesterol, and the group B includes lipids having strong hydrophobicity, such as squalene, wax esters of C₁₄-C₁₄, C₁₄-C₁₆, C₁₆-C₁₆, C₁₈-C₁₆. The results in Table 27 mean the content of each lipid, indicating the relative value (%) based on the control group (GC/MS quantitative value of un-shampooed hair).

**[Table 27]**

| Type of Lipids | | Content for short-term treatment (%) | | Content for long-term treatment (%) | |
|---|---|---|---|---|---|
| | | **Experiment** #19 | **Experiment** #20 | **Experiment** #21 | **Experiment** #22 |
| Lipid Group A | C₁₄ | 75±1 | 68±2 | 65±3 | 59±1 |
| | C₁₆ | 72±1 | 66±1 | 63±2 | 57±1 |
| | C₁₈ | 70±1 | 68±1 | 63±2 | 59±1 |
| | C₁₈₌₁ | 75±1 | 73±1 | 62±3 | 59±1 |
| | Cholesterol | 76±1 | 74±1 | 73±2 | 72±1 |
| Lipid Group B | Squalene | 57±2 | 82±1 | 43±1 | 75±1 |
| | C₁₄-C₁₆ | 61±2 | 76±9 | 45±3 | 45±3 |
| | C₁₆-C₁₆ | 55±1 | 58±6 | 42±2 | 36±1 |
| | C₁₈-C₁₆ | 55±1 | 71±1 | 48±6 | 46±6 |
| | C₁₄-C₁₄ | 57±3 | 77±6 | 48±4 | 67±5 |

Specifically, for Experiment #19, a 10% diluted shampoo solution in the composition of Table 25 was used, and the hair was washed 10 times by hand under constant physical pressure. The washing was performed by lathering for 50 seconds with the shampoo solution and washing for 2 minutes. Since the hair was brought into contact with the actual shampoo solution for 10 minutes, Experiment #20 was carried out by stirring in a shaker (Jeio Tech, SI-900R, Korea) for 10 minutes under the same concentration conditions. Experiment #20 was different from Experiment #19 in that the washing was performed without physical pressure.

Experiment #21 was performed for 30 minutes using the treatment of Experiment #19, and Experiment #22 was performed under the same conditions as Experiment #20 by increasing the treatment time by 30 minutes.

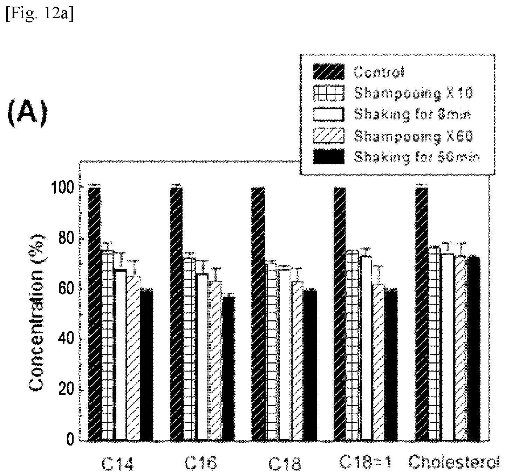

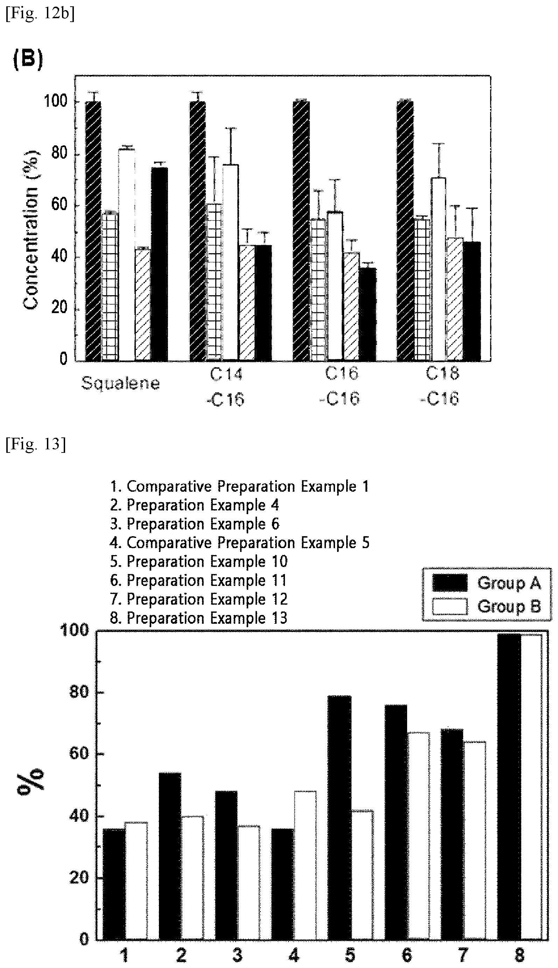

As a result, as shown in Table 27 and Fig. 12, it was observed that the lipid loss patterns of Experiments #19 and #21, in which infiltration of the surfactants were induced by applying physical pressure in the short-term and long-term treatments, were different from the lipid loss patterns of Experiments #20 and #22, which were performed without physical pressure.

It was confirmed that, in Experiments #19 and #21, lipids of group B with hydrophobicity were lost in larger amounts as compared to those in group A with relatively low hydrophobicity or amphipathicity, whereas in Experiments #20 and #22, the lipids of the group A with amphipathicity or relatively low hydrophobicity (particularly lower than the lipids of group B) were lost in larger amounts than the lipids of group B.

Although Experiments #19 and #21 have the same concentration of shampoo solution as Experiments #20 and #22, aggregation occurred by coacervation in Experiments #19 and #21, so that the hair was brought into contact with the shampoo solution at a high concentration, that is, the anionic surfactant at a high concentration. Additionally, since friction and physical pressure were applied to the hair by hand, the surfactants could more easily infiltrate into the hair, and the infiltrated surfactants could easily bind to the hydrophobic components inside the hair. Regarding the infiltration of the molecules into the hair, it has been reported previously that when the ratio of the amphoteric surfactant to the anionic surfactant is 2: 1, spherical micelles are formed instead of cylindrical micelles (Langmuir, 20, (2004) 571) at 150 mM or below, and it is not known whether hair micelles infiltrate directly into the hair, or whether surfactant monomolecules infiltrate into the hair and re-form micelles, but it was proved that the surfactants infiltrate into the hair by imaging the surfactant micelles with CryoTEM (transmission electron microscopy) at a size of 6.0 ± 0.6 nm (Langmuir, 4 (1988) 1066). For reference, the largest size of micelle observed was 4.8 nm (Langmuir, 4 (1988) 1066) and the presence of micelles in the outer part of the hair was confirmed using energy-dispersive X-ray spectroscopy (EDX) via imaging (Int. J. Cos. Sci. 26 (2004) 61). Therefore, according to the results of the above conventional studies, if it were assumed that the anionic surfactant infiltrated into the hair during the washing process and formed micelles, it was predicted that lipids of the group B having hydrophobicity would be captured by the micelles which infiltrated inside of the hair, and would be lost by exiting the hair.

As shown in Experiments #20 and #22, the lipids of the group A slowly exited at a relatively low concentration over time and were lost in large amounts. In the washing process, since the lipids of the group A in the hair are not completely hydrophobic, there is a high probability that not all of the lipids would be captured in the micelles formed by the surfactant. Therefore, the lipids of the group A are not lost by the surfactant in the inside, but are eluted to the outside by the phenomenon in which the lipids inside the hair escape to the outside, that is, by the diffusion of the lipids (J. Cosmet. Sci., 49, (1998) 223). It was confirmed that the lipids of the group A migrated towards the outside of the hair by diffusion and exited and then were lost when the lipids were brought into contact with the anionic surfactant present outside the hair.

### (4) Hair Surface Treatment

It was confirmed that the lipids of the group A were lost in the outer layer of the hair by the anionic surfactants. Therefore, in order to reduce the probability of the contact of the anionic surfactant with the lipids by suppressing the diffusion of the lipids of the group A, the shampoo composition of Table 25 and the conditioner compositions of Table 26 were prepared according to the prescription shown in Table 28 and applied on the hair. The conditioner was also applied once at a time after shampooing, and the washing was repeated 10 times in total. After each washing cycle, the hair was completely dried with a dryer to promote diffusion by heat.

Various sebum and grease residues remain on the hair, and the hair washed once with sodium laurate sulfate (SLES) was set as Comparative Preparation Example 1. As reported in the previous studies, in the case of light washing, lipids at a depth of 3 nm of hair remained (J, Cosmet Sci. 61 (6) (2010), 467-77), and thus, the washing of Comparative Preparation Example 1 was carried out on the assumption that the internal lipids were not eluted.

In Comparative Preparation Example 2, a Ucare LK cationic cellulose polymer PQ10 having a nitrogen content of 0.5% by weight was used as the polymer in the mixing of Table 25, and in Comparative Preparation Example 3, an LR30M cationic cellulose polymer PQ10 having a nitrogen content of 1.0% by weight was used as the polymer in the mixing of Table 1.

In Comparative Preparation Examples 4 and 5, Abil WAX 9840 and WAX9801 manufactured by Evonik were used, which had an interfacial tension of 120 mN/m or higher in the mixing of Table 25, respectively.

In Preparation Examples 3 (not according to the invention) and 4, cationic cellulose polymers (α: EO (bivalent or higher), β: OH, and molecular weight of 800,000) having a nitrogen content of 1.7% and 2.8% by weight, respectively, were synthesized and used as the polymer in the mixing of Table 25. In Preparation Examples 5 and 6, were treated with shampoo, which selectively included TEGOSOFT APM and TEGOSOFT E having an interfacial tension between water/oil of 100 mN/m or less, respectively, and was stirred.

**[Table 28]**

| Samples | Comp arative Prepar ation Exam ple 1 | Comp arative Prepar ation Exam ple 2 | Comp arative Prepar ation Exam ple 3 | Prepar ation Exam ple 3 | Prepar ation Exam ple 4 | Comp arative Prepar ation Exam ple 4 | Comp arative Prepar ation Exam ple 5 | Prepar ation Exam ple 5 | Prepar ation Exam ple 6 |
|---|---|---|---|---|---|---|---|---|---|
| N content in polymer (wt%) | - | 0.5 | 1.0 | 1.7 | 2.8 | 1.0 | 1.0 | 1.0 | 1.0 |
| Polar oil (mM/m) | - | - | - | - | - | 160 | 120 | 100 | 90 |

**[Table 29]**

| Lipids | Comp arative Prepar ation Exam ple 1 | Comp arative Prepar ation Exam ple 2 | Comp arative Prepar ation Exam ple 3 | Prepar ation Exam ple 3 | Prepar ation Exam ple 4 | Comp arative Prepar ation Exam ple 4 | Comp arative Prepar ation Exam ple 5 | Prepar ation Exam ple 5 | Prepar ation Exam ple 6 |
|---|---|---|---|---|---|---|---|---|---|
| Total content of Group A | 100% | 36% | 36% | 52% | 54% | 36% | 37% | 47% | 48% |
| Total content of Group B | 100% | 38% | 38% | 39% | 40% | 37% | 37% | 37% | 37% |

Table 29 shows the average content of the lipids of the group A and the lipids of the group B (amount remained in hair after washing) as a percentage relative to the control value (Comparative Preparation Example 1). According to the results of Preparation Examples 3 and 4 of Table 29, it can be seen that when the shampoo treatment was carried out using highcationic polymers, the loss of the lipids of the group A could be prevented, while the lipids of the group B could not be protected. In Preparation Examples 5 and 6, similarly, it can be seen that the lipids of the group A were better protected when oils having an interfacial tension of 100 mN/m or less were used. When comparing Preparation Examples 3 and 4 with Preparation Examples 5 and 6, a more excellent effect of preventing lipid loss was exhibited when the polymer was included rather than the polar oil. This is because the anionic surfactants were bound to other oils and polymers instead of the lipids diffused out of the hair. Even when the cationic cellulose polymers were adsorbed onto the surface of the hair and bound to the surfactants, it would be difficult for the polymers to be eluted out due to the properties of polymers, and accordingly, the film layer would not be lost in the hair. Additionally, the polar oil or the polymers having a high nitrogen content increased the charge density on the surface of the hair, which lowered the interfacial tension on the surface of the hair, thereby reducing the contact angle of the lipids, and consequently, the lipids of the group A, which had weak hydrophobicity, was prevented from being lost by surfactants that came into contact with them when exposed to the surface.

### (5) Protection of Lipids of Group B: Internal Treatment of Hair

As a crosslinking-mediating component, polylysine, polyamine, or soy protein was used for forming an amine bond with hair, and polycarbodiimide (PCI) was used for crosslinking them together (Table 30). According to the prescription of Table 25, a composition including a crosslinking-mediating component and polycarbodiimide of Table 30 below was added to a shampoo composition prepared by including a PQ10 polymer (LR30M) having a nitrogen content of 1.0% as a polymer, and the resultant was stirred at room temperature for 10 minutes at 400 rpm. Then, the lipid content in the hair was measured in the same manner as in (4) above.

Each lipid content of the hair, treated with the shampoo to which the crosslinking-mediating component and PCI of Table 30 were added, was calculated as a relative value (%) based on the lipid content value in the hair washed once with sodium laurate sulfate (SLES) of Comparative Preparation Example 1, and the results are shown in Table 31. For easy comparison of the behavioral properties according to the lipids, the average content of the five lipid components including myristyl acid, palmitic acid, stearic acid, oleic acid, and cholesterol is shown by the content of the group A lipids, and the average content of the four lipid components including squalene, wax esters of C₁₄-C₁₄, C₁₄-C₁₆, C₁₆-C₁₆, C₁₈-C₁₆, is shown by the content of the group B lipids.

**[Table 30]**

| Samples | Preparation Example 7 | Preparation Example 8 | Preparation Example 9 |
|---|---|---|---|
| Polylysine | 0.5% | - | - |
| Polyamine | - | 0.5% | - |
| Soy protein (WS-SP) | - | - | 0.5% |
| PCI | 0.5% | 0.5% | 0.5% |

**[Table 31]**

| Lipid Content | Preparation Example 7 | Preparation Example 8 | Preparation Example 9 |
|---|---|---|---|
| Lipids of Group A | 36% | 36% | 37% |
| Lipids of Group B | 42% | 47% | 46% |

As shown in Table 31, it was confirmed that the crosslinking was effective in protecting the lipids of the group B. However, the crosslinking had no protective effect on the loss of the group A lipids. That is, it was confirmed that when the inside of the hair was densely filled with crosslinking, the elution of the hydrophobic lipids of the group B by infiltration of surfactant micelles into the hair and capturing the lipids could be prevented, while the elution of the relatively less hydrophobic lipids of the group A by diffusion into the outside could not be prevented. Accordingly, the following experiment was conducted to provide a composition capable of preventing the loss of both the hydrophobic components and the amphipathic/low hydrophobic components.

### (6) Simultaneous Protection of Lipids of Groups A and B

In Tables 29 and 31, it was confirmed that the loss of lipids of the groups A and B was prevented, and in order to confirm the protective effect for all of the lipids, shampoos and conditioner compositions of Tables 25 and 26 were prepared according to the prescription of Table 32. Table 33 shows the values of GC/MS quantitative analysis by washing the hair tress 10 times in the same manner as in (4) described above. In Preparation Example 11, a cationic cellulose polymer having a nitrogen content of 1.7% by weight (α: EO (bivalent or higher) and β: OH, molecular weight of 800,000) was synthesized and used, and in Preparation Example 13, a cationic cellulose polymer having a nitrogen content of 2.8% by weight (α: EO (bivalent or higher) and β: OH, molecular weight of 800,000) was synthesized and used. In Preparation Examples 10 and 12 to 13, TEGOSOFT APM (PPG-3 myristyl ether) manufactured by TEGOSOFT having an interfacial tension of 100 mM/m was used as oil. The lipid contents shown Table 33 were averaged as a relative value (%) based on the control value of Comparative Production Example 1. The content of each component in Table 32 means the content of the component relative to the total composition as a weight percent.

**[Table 32]**

| Samples | Preparation Example 10 | Preparation Example 11 | Preparation Example 12 | Preparation Example 13 |
|---|---|---|---|---|
| Polymer | 0.5 | 0.5 | - | 0.34 |
| Oil | 0.5 | - | 0.5 | 0.33 |
| Polylysine & PCI | - | 0.5 | 0.5 | 0.33 |

**[Table 33]**

| Lipid Content | Preparation Example 10 | Preparation Example 11 | Preparation Example 12 | Preparation Example 13 |
|---|---|---|---|---|
| Lipids of Group B | 79% | 76% | 68% | 99% |
| Lipids of Group A | 46% | 67% | 64% | 99% |

It can be seen that the prescription containing a high cationic polymer and a polar oil maintained the lipid content of the group A very efficiently (Preparation Example 10), but the loss of lipids of the group B was only slightly protected. The effect of preventing the lipid loss was only slightly increased in the group B than in Preparation Examples 3 to 6 of Table 29, these results appear to be because the frictional force was reduced during the washing process, thereby reducing the damage of cuticle layer, and at the same time, the oil content decreased the infiltration of the surfactant micelles, resulting in a decrease in the loss of the lipids in the group B.

In Preparation Examples 11 to 12 of Table 33, the effect of protecting both the loss of lipids of the groups A and B was exhibited. Particularly, in Preparation Example 13, a surprising effect of protecting most of the lipids was exhibited. The reason for the occurrence of a synergistic effect in the protection be interpreted that the outer monomolecular oil or polymer cationic coating film reduces the number of micelles infiltrating into the inside as well as to the outside, and simultaneously, that when the polar oil infiltrates into the inside as well as to the surface, it interferes with the flow of the lipids diffused therein together with the tightly bound crosslinks, ultimately almost completely protecting the lipids during the washing process.

### (7) Evaluation of Physical Properties by Treatment with Composition of Preparation Example 13

### 1) Evaluation of Bending Strength of Hair

In order to confirm the effect of protecting the loss of lipids on the hair, and in order to evaluate the strength of the hairs treated with each of the compositions of Comparative Preparation Examples 1 (experimental group in which no elution of the internal lipids occurred) and 2, or Preparation Example 11, Preparation Example 12, and Preparation Example 13, the bending strength was evaluated using KES-FB2-S (KATO TECH, Japan), a bending strength tester. The strength of the hair was increased as the bending strength increased.

A sample tress was prepared in which 200 hairs of shampoo-treated hair having a thickness of 70 to 80 µm were attached into a 10 cm size without gaps. This evaluation method was carried out according to the manual recommended by KATO TECH, and when evaluating the bending strength of fiber tress, measurement was performed according to the evaluation method adopted as a standard evaluation by the public institution for fiber evaluation.

### Results of Bending Strength Evaluation

The results evaluated with the fiber strength tester are shown in Table 34 below.

**[Table 34]**

| Samples | Comparative Preparation Example 1 | Comparative Preparation Example 2 | Preparation Example 11 | Preparation Example 12 | Preparation Example 13 |
|---|---|---|---|---|---|
| Bending Strength (Unit: gf cm) | 0.576 | 0.466 | 0.513 | 0.489 | 0.573 |

As shown in Table 34, the bending strength was significantly lowered after 10 washings as in Comparative Production Example 2, that is, it was found that the strength of the hair was significantly reduced when the hair was washed 10 times. However, in Preparation Example 13, the strength of hair was almost at the same level as compared to that before washing.

### 2) Sensory Evaluation of Hair

Based on the above results, shampoos were prepared and sensory evaluation was carried out. The experiment was conducted on 15 male and 15 female subjects, and they were required to evaluate the hair-strengthening effect after shampooing based on the following evaluation criteria of smoothness.

The strength was determined by the following criteria in the order of hair strength (the opposite was stiff): (5: Very flexible); (4: Flexible); (3: Moderate); (2: Stiff); (1: Very stiff)

The trimness and calmness were evaluated by the following evaluation criteria according to the degree of trimness seen from the upper head (the opposite was puffy): (5: Very calm); (4: Calm); (3: Moderate); (2: Puffy); (1: Very puffy)

L80KC, a guar gum polymer generally prescribed for commercially available hair-strengthening shampoos, was prescribed as a polymer of Table 1 to prepare a composition, which was treated in Experiments #23 and #24, and in Experiment #25 with the prescription of Preparation Example 13.

The results are shown in Table 35 below as average values.

**[Table 35]**

| Experimental Category | Experiment #23 | Experiment #24 | Experiment #25 |
|---|---|---|---|
| Strongness | 3.6 | 3.8 | 4.6 |
| Calmness | 2.4 | 2.5 | 4.8 |

As can be seen from Experiment #25 in Table 35, it can be seen that the shampoo of the present invention significantly increased the strength compared to the conventional hair-strengthening shampoos. Additionally, the effect of hair trimness and calmness was also observed, and in Experiment #25, an effect of observing no baby hair was exhibited as it tended to stick to the hair regardless of whether it was curly, half-curly, or straight hair. Accordingly, it can be seen therefrom that the active ingredients were efficiently transferred to various forms of human hair by the adsorption according to the composition of the present invention.

### 3) Evaluation of Surface Friction of Hair

Subsequently, for the shampoos having the polymer composition of the present invention, the frictional force was quantified through device evaluation in order to evaluate the conditioning effect for imparting softness and feeling of use.

### Experimental Method

Comparative Preparation Examples 1 and 2, a hair-strengthening shampoo of LG Household & Health Care, and the composition of Preparation Example 13 were added to a burex hair tress at 10% by weight based on the weight of hair, and the hair was lathered for 15 seconds, rubbed for 20 seconds, and rinsed for 15 seconds with running water at 37°C, and then wiped with a towel to remove moisture. Thereafter, the friction was evaluated using an MTT 175 Miniature Tensile Tester (DiaSTRONG, GB).

The shampoo-treated hair was rinsed for 2 minutes and then dried with a dryer for 2 minutes. The hair was kept at 25°C in a constant temperature and humidity chamber under a humidity of 50% for one day, so that the moisture in the hair was kept constant, and then the frictional force was measured in the same temperature and humidity chamber. Here, the percentage (%) divided by the frictional force value (reference value), after washing the hair tress with the anionic surfactant sodium lauryl sulfate, was calculated and is shown in Table 12 below. In this case, the hair was smoother as the frictional force (%) increased.

### Results

**[Table 36]**

| Experimental Category | Experiment #26 (Comparative Preparation Example 1) | Experiment #27 (Comparative Preparation Example 2) | Experiment #28 (Hair-strengthening shampoo) | Experiment #29 (Preparation Example 13) |
|---|---|---|---|---|
| During rinsing | -4% | 55% | 37% | 56% |
| After drying | -6% | 21% | 26% | 29% |

From the results shown in Table 36 above, the shampoo having the polymer composition of the present invention remarkably reduced frictional force, thereby increasing the conditioning effect for imparting smoothness. In particular, in the case of the commercial hair-strengthening shampoo, the smoothing effect was reduced as shown in Experiment #28. In other words, as can be seen from the above embodiments, it was confirmed that the polymer composition prepared by the present invention exhibited the effect of strengthening the bending strength (weakening the flexibility) by preventing the loss of lipids when applied to a conditioning agent, and at the same time, the polymer composition of the present invention was found to be a component for excellent hair conditioning cosmetic compositions for imparting smoothness.

### (4) Tensile strength

Four hair tresses (12 cm in length, 3 g in weight) made of damaged hair by bleaching twice were prepared. The tensile strength (unit: cN, 1 gf = 0.98 cN) of 100 strands of hair was measured in each hair tress, and the average value was calculated and set as a reference value. In this case, a tensile strength measuring device manufactured by DIASTRON was used to measure the tensile strength.

Subsequently, each hair tress was immersed in each of the compositions according to Experiments #26 to #29 of Table 36, except for Experiment #29, for 10 minutes and then washed for 30 seconds with running water. Thereafter, the hair was dried at room temperature (about 25°C) for 24 hours. The tensile strength (cN) of the 100 strands of hair was measured in each hair tress treated as described above, and the average value was calculated. The results are shown in Table 37 below.

**[Table 37]**

| Experimental Category | Experiment #26 (Comparative Preparation Example 1) | Experiment #27 (Comparative Preparation Example 2) | Experiment #28 (Hair-strengthening shampoo) | Experiment #29 (Preparation Example 13) |
|---|---|---|---|---|
| Before treatment | 142 cN | 139 cN | 141 cN | 142 cN |
| After treatment | 141 cN | 112 cN | 123 cN | 165 cN |
| Enhancement | -1 | -27 | -18 | +23 |

As shown in Table 37, it can be seen that the tensile strength was decreased in all other treatment groups, but the tensile strength of the hair was increased only in Preparation Example 13 (Experiment #29) even after washing with shampoo. Therefore, it can be seen that the composition of the present invention also has an effect of increasing the tensile strength of the hair by preventing the loss of internal components of the hair.

### 5) Evaluation of Glossiness

Four hair tresses (12 cm in length, 3 g in weight) made of damaged hair by bleaching twice were prepared, and glossiness was measured using SAMBA (Bossa Nova Tech., USA). The glossiness was evaluated by first measuring the front and back of the tress to adjust the glossiness to a value of 8.5, and then treating the hair with the compositions of Preparation Examples and Comparative Preparation Examples in the same manner and under the same condition as the method of measuring the rate of change of the tensile strength.

**[Table 38]**

| Experimental Category | Experiment #26 (Comparative Preparation Example 1) | Experiment #27 (Comparative Preparation Example 2) | Experiment #28 (Hair-strengthening shampoo) | Experiment #29 (Preparation Example 13) |
|---|---|---|---|---|
| Before treatment | 8.5 | 8.5 | 8.5 | 8.5 |
| After treatment | 8.0 | 7.0 | 9.0 | 19.5 |
| Enhancement | -0.5 | -1.5 | +0.5 | +11 |

As shown in Table 38, it can be seen that all other treatment groups showed a decrease in glossiness, but the glossiness was significantly increased only in Preparation Example 13. Thus, it can be seen therefrom that the composition of the present invention not only protects the lipids during the washing process, and at the same time, exhibits the effect of significantly increasing the glossiness to the hair by providing calmness and trimness to the hair.

## Claims

1. A composition for treating hair, skin, or fiber, comprising the polymer represented by Chemical Formula 1 below: 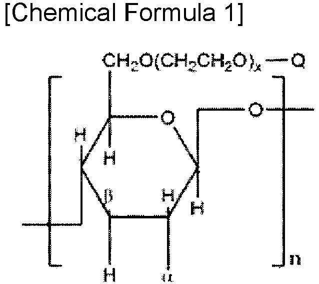
wherein n number of Q is each independently H or 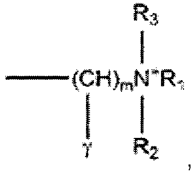 but not all H,
R₁, R₂, and R₃ are each independently C₁₋₆ alkyl, alkenyl, or alkynyl, m is an integer from 1 to 10, γ is H or OH;
x is an integer from 1 to 100;
n is an integer from 10 to 1000;
α and β are each independently O(CH₂CH₂O)ₛH, wherein s is an integer from 0 to 100; and
wherein the molecular weight of the polymer is 300,000 to 2,900,000, and
wherein the polymer represented by Chemical Formula 1 has a nitrogen content of 2.3 wt.% to 3.2 wt.%, based on the total weight of the polymer.

2. The composition of claim 1, wherein in the polymer, the ratio of n: the number of Q, when Q is 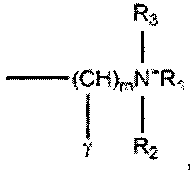 is 1:0.3 to 1:0.7, and the molecular weight of the polymer is 600,000 to 2,500,000.

3. The composition of claim 1, wherein the polymer is comprised in an amount of 0.01 to 10% by weight relative to the total weight of the composition.

4. The composition of claim 1, wherein the polymer provides the use for cumulatively adsorbing an active ingredient onto hair, skin, or fiber by allowing an OH group comprised in α, an OH group comprised in β, or both to be bound to the active ingredient.

5. The composition of claim 4, wherein in the polymer, the ratio of n: the number of Q, when Q is 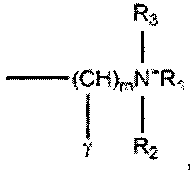 is 1:0.3 to 1:0.7, and the molecular weight of the polymer is 300,000 to 400,000.

6. The composition of claim 4, wherein the active ingredient is a reactive dye.

7. The composition of claim 6, wherein the reactive dye has a reactive group selected from dichlorotriazinyl, difluorochloro pyrimidine, monofluoro triazinyl, dichloroquinoxaline, vinyl sulfone, difluorotriazine, monochloro triazinyl, bromo acrylamide, and trichloropyrimidine,
and wherein the reactive dye comprises a chromophore selected from azo, anthraquinone, phthalocyanine, formazan, and tripendioxazine.

8. The composition of claim 4, wherein the polymer is comprised in an amount of 0.01 to 1 wt% by weight relative to the total weight of the composition.

9. The composition of claim 1, wherein the polymer provides the use for transferring an active ingredient to hair, skin, or fiber by allowing an OH group comprised in α, an OH group comprised in β, or both to be bound to the active ingredient.

10. The composition of claim 9, wherein in the polymer, the ratio of n: the number of Q, when Q is 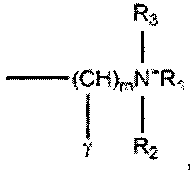 is 1:0.3 to 1:0.7, and the molecular weight of the polymer is 1,300,000 to 2,900,000.

11. The composition of claim 1, providing the use for preventing the loss of hair, skin, or fiber components by further comprising a carbodiimide-based compound and a crosslinking-mediating component having a carboxyl group or an amine group.

12. The composition of claim 11, wherein in the polymer, the ratio of n: the number of Q, when Q is 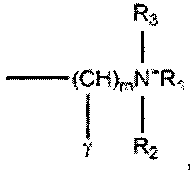 is 1:0.3 to 1:0.7, and the molecular weight of the polymer is 600,000 to 2,500,000.

13. The composition of claim 11, wherein the carbodiimide-based compound is a compound comprising an N=C=N structure.

14. The composition of claim 11, wherein the polymer is comprised in an amount of 0.01% to 10% by weight relative to the total weight of the composition.

15. Use of a composition for treating hair, skin, or fiber, said composition comprising the polymer represented by Chemical Formula 1 below: 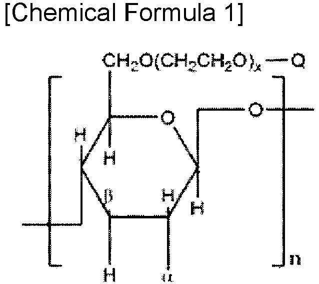
wherein n number of Q is each independently H or 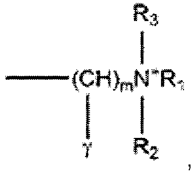 but not all H,
R₁, R₂, and R₃ are each independently C₁₋₆ alkyl, alkenyl, or alkynyl, m is an integer from 1 to 10, γ is H or OH;
x is an integer from 1 to 100;
n is an integer from 10 to 1000;
α and β are each independently O(CH₂CH₂O)ₛH, wherein s is an integer from 0 to 100; and
wherein the molecular weight of the polymer is 300,000 to 2,900,000, and
wherein the polymer represented by Chemical Formula 1 has a nitrogen content of 2.3 wt. % to 3.2 wt. %.

## Patentansprüche

1. Zusammensetzung zur Behandlung von Haar, Haut oder Faser, umfassend das durch die nachstehende chemische Formel 1 dargestellte Polymer: 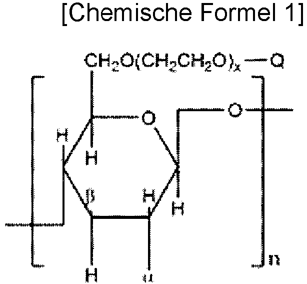
wobei die Anzahl n von Q jeweils unabhängig H oder 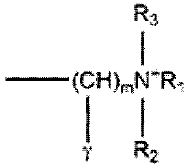 ist, aber nicht alle H sind,
R₁, R₂ und R₃ jeweils unabhängig C₁₋₆ -Alkyl, Alkenyl oder Alkinyl sind, m eine ganze Zahl von 1 bis 10 ist, γ H oder OH ist;
x eine ganze Zahl von 1 bis 100 ist;
n eine ganze Zahl von 10 bis 1000 ist;
α und β jeweils unabhängig O(CH₂CH₂O)ₛH sind, wobei s eine ganze Zahl von 0 bis 100 ist; und
wobei das Molekulargewicht des Polymers 300.000 bis 2.900.000 beträgt, und
wobei das durch die chemische Formel 1 dargestellte Polymer einen Stickstoffgehalt von 2,3 Gew.-% bis 3,2 Gew.-%, bezogen auf das Gesamtgewicht des Polymers, aufweist.

2. Zusammensetzung nach Anspruch 1, wobei in dem Polymer das Verhältnis von n: der Anzahl von Q, wenn Q 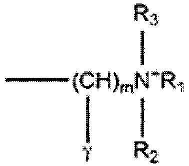 ist, 1:0,3 bis 1:0,7 beträgt und das Molekulargewicht des Polymers 600.000 bis 2.500.000 beträgt.

3. Zusammensetzung nach Anspruch 1, wobei das Polymer in einer Menge von 0,01 bis 10 Gew.-%, bezogen auf das Gesamtgewicht der Zusammensetzung, enthalten ist.

4. Zusammensetzung nach Anspruch 1, wobei das Polymer die Verwendung zum kumulativen Adsorbieren eines Wirkstoffs an Haar, Haut oder Faser bereitstellt, indem es ermöglicht wird, dass eine in α enthaltene OH-Gruppe, eine in β enthaltene OH-Gruppe oder beide an den Wirkstoff gebunden werden.

5. Zusammensetzung nach Anspruch 4, wobei in dem Polymer das Verhältnis von n: der Anzahl von Q, wenn Q 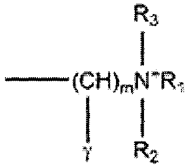 ist, 1:0,3 bis 1:0,7 beträgt und das Molekulargewicht des Polymers 300.000 bis 400.000 beträgt.

6. Zusammensetzung nach Anspruch 4, wobei der Wirkstoff ein reaktiver Farbstoff ist.

7. Zusammensetzung nach Anspruch 6, wobei der reaktive Farbstoff eine reaktive Gruppe aufweist, die aus Dichlortriazinyl, Difluorchlorpyrimidin, Monofluortriazinyl, Dichlorchinoxalin, Vinylsulfon, Difluortriazin, Monochlortriazinyl, Bromacrylamid und Trichlorpyrimidin ausgewählt ist,
und wobei der reaktive Farbstoff ein Chromophor umfasst, das aus Azo, Anthrachinon, Phthalocyanin, Formazan und Tripendioxazin ausgewählt ist.

8. Zusammensetzung nach Anspruch 4, wobei das Polymer in einer Menge von 0,01 bis 1 Gew.-%, bezogen auf das Gesamtgewicht der Zusammensetzung, enthalten ist.

9. Zusammensetzung nach Anspruch 1, wobei das Polymer die Verwendung zum Übertragen eines Wirkstoffs auf Haar, Haut oder Faser bereitstellt, indem es ermöglicht wird, dass eine in α enthaltene OH-Gruppe, eine in β enthaltene OH-Gruppe oder beide an den Wirkstoff gebunden werden.

10. Zusammensetzung nach Anspruch 9, wobei in dem Polymer das Verhältnis von n: der Anzahl von Q, wenn Q 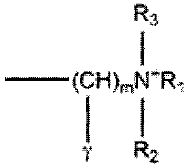 ist, 1:0,3 bis 1:0,7 beträgt und das Molekulargewicht des Polymers 1.300.000 bis 2.900.000 beträgt.

11. Zusammensetzung nach Anspruch 1, die die Verwendung zum Verhindern des Verlusts von Haar-, Haut- oder Faserkomponenten bereitstellt, indem sie ferner eine Verbindung auf Carbodiimidbasis und eine vernetzungsvermittelnde Komponente mit einer Carboxylgruppe oder einer Amingruppe umfasst.

12. Zusammensetzung nach Anspruch 11, wobei in dem Polymer das Verhältnis von n: der Anzahl von Q, wenn Q 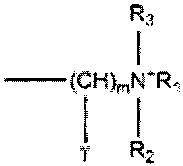 ist, 1:0,3 bis 1:0,7 beträgt und das Molekulargewicht des Polymers 600.000 bis 2.500.000 beträgt.

13. Zusammensetzung nach Anspruch 11, wobei die Verbindung auf Carbodiimidbasis eine Verbindung ist, die eine N=C=N-Struktur umfasst.

14. Zusammensetzung nach Anspruch 11, wobei das Polymer in einer Menge von 0,01 bis 10 Gew.-%, bezogen auf das Gesamtgewicht der Zusammensetzung, enthalten ist.

15. Verwendung einer Zusammensetzung zur Behandlung von Haar, Haut oder Faser, wobei die Zusammensetzung das durch die nachstehende chemische Formel 1 dargestellte Polymer umfasst: 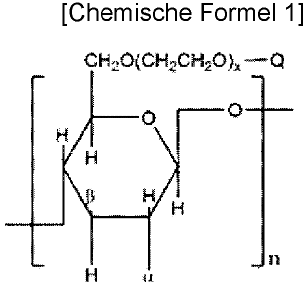
wobei die Anzahl n von Q jeweils unabhängig H oder 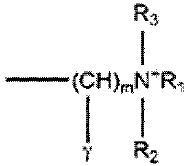 ist, aber nicht alle H sind,
R₁, R₂ und R₃ jeweils unabhängig C₁₋₆ -Alkyl, Alkenyl oder Alkinyl sind, m eine ganze Zahl von 1 bis 10 ist, γ H oder OH ist;
x eine ganze Zahl von 1 bis 100 ist;
n eine ganze Zahl von 10 bis 1000 ist;
α und β jeweils unabhängig O(CH₂CH₂O)ₛH sind, wobei s eine ganze Zahl von 0 bis 100 ist; und
wobei das Molekulargewicht des Polymers 300.000 bis 2.900.000 beträgt, und
wobei das durch die chemische Formel 1 dargestellte Polymer einen Stickstoffgehalt von 2,3 Gew.-% bis 3,2 Gew.-% aufweist.

## Revendications

1. Composition destinée à traiter les cheveux, la peau ou des fibres, comprenant le polymère représenté par la Formule chimique 1 ci-dessous : 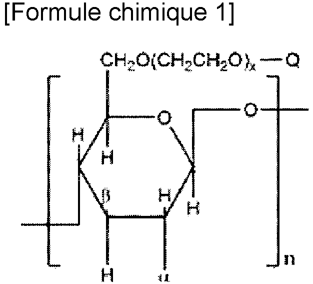
dans laquelle le nombre n de Q est chacun indépendamment H ou 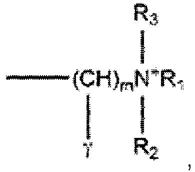 mais pas tous les H,
R₁, R₂ et R₃ sont chacun indépendamment un alkyle, un alkényle ou un alkynyle en C₁₋₆, m est un nombre entier de 1 à 10, γ est H ou OH ;
x est un nombre entier de 1 à 100 ;
n est un nombre entier de 10 à 1000 ;
α et β sont chacun indépendamment O(CH₂CH₂O)ₛH, dans laquelle s est un nombre entier de 0 à 100 ; et
dans laquelle le poids moléculaire du polymère est 300 000 à 2 900 000, et
dans laquelle le polymère représenté par la Formule chimique 1 a une teneur en azote de 2,3 % en poids à 3,2 % en poids, sur la base du poids total du polymère.

2. Composition selon la revendication 1, dans laquelle dans le polymère, le rapport de n : le nombre de Q, quand Q est 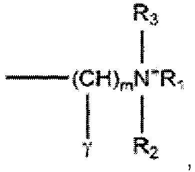 est 1:0,3 à 1:0,7, et le poids moléculaire du polymère est 600 000 à 2 500 000.

3. Composition selon la revendication 1, dans laquelle le polymère est compris dans une quantité de 0,01 à 10 % en poids pour le poids total de la composition.

4. Composition selon la revendication 1, dans laquelle le polymère fournit l'utilisation pour l'adsorption cumulative d'un ingrédient actif sur les cheveux, la peau ou des fibres en permettant à un groupe OH compris dans α, un groupe OH compris dans β, ou les deux, de se lier à l'ingrédient actif.

5. Composition selon la revendication 4, dans laquelle dans le polymère, le rapport de n : le nombre de Q, quand Q est 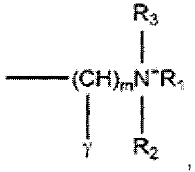 est 1:0,3 à 1:0,7, et le poids moléculaire du polymère est 300 000 à 400 000.

6. Composition selon la revendication 4, dans laquelle l'ingrédient actif est un colorant réactif.

7. Composition selon la revendication 6, dans laquelle le colorant réactif a un groupe réactif sélectionné parmi le dichlorotriazinyle, le difluorochloropyrimidine, le monofluorotriazinyle, la dichloroquinoxaline, la vinylsulfone, la difluorotriazine, le monochlorotriazinyle, le bromoacrylamide et la trichloropyrimidine,
et dans laquelle le colorant réactif comprend un chromophore sélectionné parmi azo, anthraquinone, phtalocyanine, formazane et tripendioxazine.

8. Composition selon la revendication 4, dans laquelle le polymère est compris dans une quantité de 0,01 à 1 % en poids pour le poids total de la composition.

9. Composition selon la revendication 1, dans laquelle le polymère fournit l'utilisation pour le transfert d'un ingrédient actif sur les cheveux, la peau ou des fibres en permettant à un groupe OH compris dans α, un groupe OH compris dans β, ou les deux, de se lier à l'ingrédient actif.

10. Composition selon la revendication 9, dans laquelle dans le polymère, le rapport de n : le nombre de Q, quand Q est 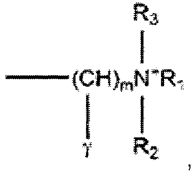 est 1:0,3 à 1:0,7, et le poids moléculaire du polymère est 1 300 000 à 2 900 000.

11. Composition selon la revendication 1, fournissant l'utilisation pour la prévention de la perte des composants des cheveux, de la peau ou des fibres en comprenant en outre un composé à base de carbodiimide et un composant médiateur de réticulation ayant un groupe carboxyle ou un groupe amine.

12. Composition selon la revendication 11, dans laquelle dans le polymère, le rapport de n : le nombre de Q, quand Q est 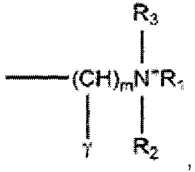 est 1:0,3 à 1:0,7, et le poids moléculaire du polymère est 600 000 à 2 500 000.

13. Composition selon la revendication 11, dans laquelle le composé à base de carbodiimide est un composé comprenant une N=C=N structure.

14. Composition selon la revendication 11, dans laquelle le polymère est compris dans une quantité de 0,01% à 10% en poids pour le poids total de la composition.

15. Utilisation d'une composition destinée à traiter les cheveux, la peau ou des fibres, ladite composition comprenant le polymère représenté par la Formule chimique 1 ci-dessous : 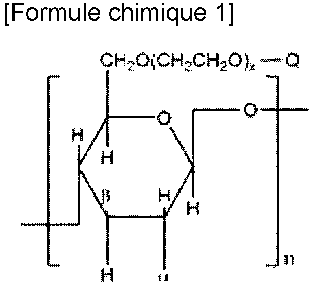
dans laquelle le nombre n de Q est chacun indépendamment H ou 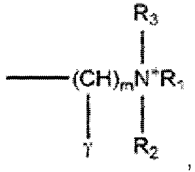 mais pas tous les H,
R₁, R₂ et R₃ sont chacun indépendamment un alkyle, un alkényle ou un alkynyle en C₁₋₆, m est un nombre entier de 1 à 10, γ est H ou OH ;
x est un nombre entier de 1 à 100 ;
n est un nombre entier de 10 à 1000 ;
α et β sont chacun indépendamment O(CH₂CH₂O)ₛH, dans laquelle s est un nombre entier de 0 à 100 ; et
dans laquelle le poids moléculaire du polymère est 300 000 à 2 900 000, et
dans laquelle le polymère représenté par la Formule chimique 1 a une teneur en azote de 2,3 % en poids à 3,2 % en poids.
